(19) Europäisches Patentamt
European Patent Office
Office européen des brevets

(11) EP 1 270 585 B1

(12) # EUROPEAN PATENT SPECIFICATION

(45) Date of publication and mention of the grant of the patent:
**17.05.2006 Bulletin 2006/20**

(21) Application number: **01915695.9**

(22) Date of filing: **22.03.2001**

(51) Int Cl.:
***C07K 7/06*** (2006.01) ***C07K 14/705*** (2006.01)
***A61K 38/10*** (2006.01) ***A61K 38/17*** (2006.01)
***A61P 1/00*** (2006.01) ***A61P 3/00*** (2006.01)
***A61P 9/00*** (2006.01) ***A61P 15/00*** (2006.01)
***A61P 25/00*** (2006.01) ***A61P 31/18*** (2006.01)
***A61P 37/02*** (2006.01)

(86) International application number:
**PCT/JP2001/002278**

(87) International publication number:
**WO 2001/070769 (27.09.2001 Gazette 2001/39)**

(54) **PEPTIDE DERIVATIVE**

PEPTIDDERIVAT

DERIVE PEPTIDIQUE

(84) Designated Contracting States:
**AT BE CH CY DE DK ES FI FR GB GR IE IT LI LU MC NL PT SE TR**

(30) Priority: **23.03.2000 JP 2000087114**
**19.09.2000 JP 2000288891**

(43) Date of publication of application:
**02.01.2003 Bulletin 2003/01**

(73) Proprietor: **Takeda Chemical Industries, Ltd.**
**Osaka-shi,**
**Osaka 541-0045 (JP)**

(72) Inventors:
- **KITADA, Chieko**
**Sakai-shi**
**Osaka 590-0073 (JP)**
- **NISHIZAWA, Naoki**
**Amagasaki-shi**
**Hyogo 661-0981 (JP)**
- **HINUMA, Shuji**
**Tsukuba-shi**
**Ibaraki 305-0821 (JP)**
- **HOSOYA, Masaki**
**Tsuchiura-shi**
**Ibaraki 300-0007 (JP)**

(74) Representative: **Lewin, John Harvey et al**
**Takeda Euro IP Department**
**10 Charles II Street**
**London SW1Y 4AA (GB)**

(56) References cited:
**EP-A- 1 116 727** **WO-A-00/18793**

- **NISHIZAWA, NAOKI ET AL: "High potency analogs of apelin, a ligand of orphan GPCR APJ" PEPTIDE SCIENCE , VOLUME DATE 2000, 37TH, 151-154 CODEN: PSCIFQ; ISSN: 1344-7661, 2001, XP009034527**
- **MARK CAYABYAB ET AL.: 'Apelin, the natural ligand of the orphan seven-transmembrane. Receptor APJ, inhibits human immunodeficiency virus type 1 entry' J. VIROL. vol. 74, no. 24, 2000, pages 11972 - 11976, XP002941748**
- **MIN-XU ZOU ET AL.: 'Apelin peptides block the entry of human immunodeficiency virus (HIV)' FEBS LETTERS vol. 473, 2000, pages 15 - 18, XP002941749**
- **KAZUHIKO TATEMOTO ET AL.: 'Isolation and characterization of a novel endogenous peptide ligand for human APJ receptor' BBRC vol. 251, 1998, pages 471 - 476, XP002941750**
- **BRAIN F. O'DOWD ET AL.: 'A human gene that shows identity with the gene encoding the angiotensin receptor is located on chromosome 11' GENE vol. 136, 1993, pages 355 - 360, XP002941751**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 270 585 B1

## Description

## Technical Field

The present invention relates to a novel peptide having a ligand activity on APJ that is an orphan G protein-coupled receptor protein, use thereof.

## Background Art

Many hormones and neurotransmitters regulate the biological function via specific receptors present on cell membranes. Many of these receptors are coupled with guanine nucleotide-binding protein (hereinafter sometimes simply referred to as G protein) and mediate the intracellular signal transduction via activation of G protein, and these receptor proteins possess the common structure containing seven transmembrane domains and are thus collectively referred to as G protein-coupled receptors or seven-transmembrane receptors (7TMR).

Regulation of functions important for the living body, such as maintenance of homeostasis in the living body, reproduction, development of individuals, metabolism, growth, and regulation in the nerve system, circulatory organ system, immune system, digestive organ system and metabolic system, is carried out through interaction of hormones or neurotransmitters with G protein-coupled receptors. For regulation of biological functions, receptor proteins are known to occur for various hormones and neurotransmitters and play an important role in regulating their functions, but there still remain many unrevealed features as to unknown agonists (hormones, neurotransmitters etc.) and presence of receptors therefor.

Utilizing homology of a partial structure of G protein-coupled receptor proteins to receptor amino acid sequences, a method of searching for DNA encoding a novel receptor protein has been carried out in recent years by use of polymerase chain reaction (abbreviated hereinafter into PCR), and a large number of orphan G protein-coupled receptor proteins whose ligands are not known have been cloned (Libert, F., et al. Science, 244, 569-572, 1989, Welch, S. K., et al., Biochem. Biophys. Res. Commun., 209, 606-613, 1995, Marchese, A., et al., Genomics, 23, 609-618, 1994, Marchese, A., Genomics, 29, 335-344, 1995). Further, novel G protein-coupled receptor proteins have been found one after another by random sequencing of genomic DNA or cDNA (Nomura, N., et al., DNA Research, 1, 27-35, 1994). The general means of determining ligands for these orphan G protein-coupled receptor proteins depended conventionally on mere estimation from homology in the primary structure of the G protein-coupled receptor protein. However, a large number of orphan G protein-coupled receptor proteins have low homology to known receptors, thus making it actually difficult to estimate their corresponding ligands on only the basis of homology in the primary structure except for receptor subtypes of known ligands. On the other hand, a large number of orphan G protein-coupled receptors have been found through gene analysis, and thus it is estimated that a large number of their corresponding unknown ligands are present, but there are few reports on actual identification of ligands for orphan G protein-coupled receptors.

Recently, there are reports wherein a novel opioid peptide was searched for by introducing cDNA encoding an orphan G protein-coupled receptor protein into an animal cell (Reinsheld, R. K. et al., Science, 270, 792-794, 1995, Menular, J.-C., et al., Nature, 377, 532-535, 1995). However, the ligand in this case had been estimated to belong to a family of opioid peptides from homology to known G protein-coupled receptors and distribution in tissues. There is a long history of research and development of substances acting on the living body via opioid receptors, and various antagonists and agonists have been developed. In such development, an agonist to this receptor is found from a group of artificially synthesized compounds and used as a probe to verify expression of the receptor in cells having receptor cDNA introduced therein, and an activating substance similar to the agonist is searched for in the intracellular information transmission system and purified to determine the structure of the ligand.

Further, there is also a report wherein cDNA encoding an orphan G protein-coupled receptor (GRL104) from a snail is introduced into CHO cells, and a novel physiologically active peptide has been identified by using, as an indicator, an increase in specific intracellular calcium levels in the cells expressing the receptor (Cox, K. J. A., et al., J. Neurosci., 17(4), 1197-1205, 1997), but this novel physiologically active peptide had high homology to known leucokinin, and GRL104 was also reactive with known leucokinin. Accordingly, there are few orphan G protein-coupled receptor proteins whose ligands could be roughly estimated, and in particular when homology to the known G protein-coupled receptor protein family is low, there is no or less information on the ligand, and estimation of the ligand was difficult.

As one of reported organ G protein-coupled receptors, there is APJ (O'Dowd, B.F., et al., Gene, 436, 355-359, 1993). APJ has low homology to angiotensin receptor (AT1). A naturally occurring ligand for APJ, and its partial peptides, are described in Biochemical and Biophysical Research Communications, 251, 471-476 (1998), WO 99/33976 (Japanese Patent Application No. 220853/1998), but there is no known ligand derived from a naturally occurring ligand by artificial modifications (for example, a modified ligand wherein one to several constituent amino acids in a naturally occurring ligand are replaced by other amino acids, or side chains of one to several constituent amino acids in a naturally occurring ligand are substituted with suitable substituent groups).

## DISCLOSURE OF INVENTION

A peptide prepared by artificially modifying a naturally occurring ligand for APJ that is an orphan G protein-coupled receptor expressed in the central nervous system, circulatory organ system, generative organ system, immune system, digestive organ system, is considered more useful as a pharmaceutical preparation, than the naturally occurring ligand, but the structure and functions of such a peptide more useful as a pharmaceutical preparation than the naturally occurring ligand have still not been revealed.

Based on this problem, the present inventors synthesized a wide variety of peptides by using, as an indicator, a change in the binding property between peptides which are modified derivatives of the naturally occurring ligand and the receptor protein, and estimated and specified an active site of the naturally occurring ligand, and as a result, the present inventors found modified derivatives of the naturally occurring ligand, which are more useful as a pharmaceutical preparation.

That is, the present invention provides:

(1) A peptide represented by the formula:

P1-Arg-Pro-Arg-Leu-Phe-P2-P3-Gly-Pro-P4-P5 (I)

wherein P1 represents a hydrogen atom, or an amino acid residue or a peptide chain, consisting of 1 to 25 amino acids which are independently the same or different and whose side chain is substituted or unsubstituted, P2 represents a neutral amino acid residue whose side chain is substituted or unsubstituted or a basic amino acid residue whose side chain is substituted or unsubstituted, P3 represents a neutral amino acid residue whose side chain is substituted or unsubstituted, an aromatic amino acid residue whose side chain is substituted or unsubstituted or a basic amino acid residue whose side chain is substituted or unsubstituted, P4 represents a bond or a neutral or aromatic amino acid residue whose side chain is substituted or unsubstituted, P5 represents [1] an amino acid residue whose side chain is substituted or unsubstituted, or its amino acid derivative wherein the C-terminal carboxyl group has been reduced to a hydroxymethyl group or formyl group, [2] a hydroxyl group, or [3] a dipeptide chain formed by binding an amino acid residue whose side chain is substituted or unsubstituted, to an amino acid residue whose side chain is substituted or unsubstituted, or its peptide derivative wherein the C-terminal carboxyl group of the dipeptide has been reduced to a hydroxymethyl group or formyl group, and a side chain of each amino acid residue in the formula -Arg-Pro-Arg-Leu-Phe-or -Gly-Pro- is substituted or unsubstituted;
an ester thereof or an amide thereof, or a salt thereof.

(2) The peptide according to the above-mentioned (1), an ester thereof or an amide thereof, or a salt thereof, wherein P1 is a hydrogen atom, pGlu or Arg-Arg-Gln,

(3) The peptide according to the above-mentioned (1), an ester thereof or an amide thereof, or a salt thereof, wherein P2 is substituted or unsubstituted His or is substituted or unsubstituted Ala.

(4) The peptide according to the above-mentioned (1), an ester thereof or an amide thereof, or a salt thereof, wherein P3 is substituted or unsubstituted Arg or is substituted or unsubstituted Lys.

(5) The peptide according to the above-mentioned (1), an ester thereof or an amide thereof, or a salt thereof, wherein -P4-P5 is -Cha-Pro-Phe (Cl), -Cha-Pro-Phe, -Met-Pro-Phe, -Met-Pro-Phe (Cl), -Cha-Phe or -Met-Phe.

(6) The peptide according to the above-mentioned (1), which is represented by:

[1] Arg-Pro-Arg-Leu-Phe-Ala-Arg-Gly-Pro-Cha-Pro-Phe(Cl),
[2] Arg-Pro-Arg-Leu-Phe-His-Lys-Gly-Pro-Cha-Pro-Phe(Cl),
[3] Arg-Pro-Arg-Leu-Phe-His-Lys-Gly-Pro-Cha-Pro-Phe,
[4] Arg-Pro-Arg-Leu-Phe-His-Lys-Gly-Pro-Met-Pro-Phe(Cl),
[5] Arg-Pro-Arg-Leu-Phe-Ala-Arg-Gly-Pro-Met-Pro-Phe,
[6] Arg-Pro-Arg-Leu-Phe-His-Lys-Gly-Pro-Cha-Phe(Cl),
[7] pGlu-Arg-Pro-Arg-Leu-Phe-Arg-Arg-Gly-Pro-Met-Pro-Phe,
[8] pGlu-Arg-Pro-Arg-Leu-Phe-His-Lys-Gly-Pro-Met-Pro-Phe, or
[9] -Pro-Arg-Leu-Phe-Ala-Arg-Gly-Pro-Met-Phe,

an ester thereof or an amide thereof, or a salt thereof,

(7) A pharmaceutical preparation comprising the peptide described in the above-mentioned (1), an ester thereof or an amide thereof, or a salt thereof.

(8) The pharmaceutical preparation according to the above-mentioned (7), which is a neutral nerve function regulator, a circulatory function regulator, a cardiac function regulator, an immune function regulator, a digestive organ function regulator, a metabolic function regulator or a generative organ regulator.

(9) The pharmaceutical preparation according to the above-mentioned (7), which is a prophylactic and therapeutic agent for HIV infections or AIDS.

(10) Use of the peptide described in the above-mentioned (1), an ester thereof or an amide thereof, or a salt thereof, in the manufacture of a medicament for preventing or treating HIV infections or AIDS.

Further, the present invention provides (11) the pharmaceutical preparation according to the above-mentioned (7), which is a prophylactic and/or therapeutic agent for dementia, melancholia, attention deficit hyperactivity (minimal brain disease) syndrome, mental confusion, anxiety, schizophrenia, psychasthenia, an obstacle in growth hormone secretion, bulimia, overeating, hypercholesterolemia, hyperglyceridemia, hyperlipemia, hyperprolactinemia, diabetes, cancers, pancreatitis, renal diseases, Turner's syndrome, neurosis, rheumatic arthritis, spinal damage, transitory cerebral ischemia paroxysm, amyotrophic lateral sclerosis, acute myocardial infarction, spinal cerebellum degeneration, bone fracture, wounds, atopic dermatitis, osteoporosis, asthma, epilepsy, sterility, arteriosclerosis, pulmonary emphysema, lung edema, imperfect lactation, or the like.

## BEST MODE FOR CARRYING OUT THE INVENTION

The amino acid residue in the present invention refers to a structure excluding its N- or C-terminus, wherein the amino acid has lost a water molecule to form a peptide linkage through which it has been incorporated into a protein or a peptide. For example, the α-amino acid residue refers to the structure $-HNC(R^0)(R^1)CO-$ excluding its N- or C-terminus, wherein the α-amino acid ($H_2NC(R^0)(R^1)COOH$ wherein $R^0$ and $R^1$ are the same or different and represent an arbitrary substituent group) has lost a water molecule to form a peptide linkage through which it has been incorporated into a protein or a peptide. On one hand, the N-terminal amino acid residue is represented by $H_2NC(R^0)(R^1)CO-$ and the C-terminal amino acid residue by $-HNC(R^0)(R^1)COOH$. The β-amino acid residue refers to the structure $-HNC(R^0)(R^1)C(R^2)(R^1)CO-$ excluding its N- or C-terminus, wherein the β-amino acid ($H_2NC(R^0)(R^1)C(R^2)(R^3)COOH$ wherein $R^0$, $R^1$, $R^2$ and $R^3$ are the same or different and represent an arbitrary substituent group) has lost a water molecule to form a peptide linkage through which it has been incorporated into a protein or a peptide. On one hand, the N-terminal amino acid residue is represented by $H_2NC(R^0)(R^1)C(R^2)(R^3)CO-$ and the C-terminal amino acid residue by $-NHC(R^0)(R^1)C(R^2)(R^3)COOH$. The γ-amino acid residue refers to the structure $-NHC(R^0)(R^1)C(R^2)(R^3)C(R^4)(R^5)CO-$ excluding its N- or C-terminus, wherein the γ-amino acid ($H_2NC(R^0)(R^1)C(R^2)(R^3)C(R^4)(R^5)COOH$ wherein $R^0$, $R^1$, $R^2$, $R^3$ $R^4$ and $R^5$ are the same or different and represent an arbitrary substituent group) has lost a water molecule to form a peptide linkage through which it has been incorporated into a protein or a peptide. On one hand, the N-terminal amino acid residue is represented by $H_2NC(R^0)(R^1)C(R^2)(R^3)C(R^4)(R^5)CO-$ and the C-terminal amino acid residue by $-NHC(R^0)(R^1)C(R^2)(R^3)C(R^4)(R^5)COOH$. The ε-amino acid residue refers to the structure $-NHC(R^0)(R^1)C(R^2)(R^3)C(R^4)(R^5)C(R^6)(R^7)C(R^8)(R^9)CO-$ excluding its N- or C-terminus, wherein the ε-amino acid ($H_2NC(R^0)(R^1)C(R^2)(R^3)C(R^4)(R^5)C(R^6)(R^7)C(R^8)(R^9)COOH$ wherein $R^0$, $R^1$, $R^2$, $R^3$ $R^4$, $R^5$, $R^6$, $R^7$, $R^8$ and $R^9$ are the same or different and represent an arbitrary substituent group) has lost a water molecule to form a peptide linkage through which it has been incorporated into a protein or a peptide. On one hand, the N-terminal amino acid residue is represented by $H_2NC(R^0)(R^1)C(R^2)(R^3)C(R^4)(R^5)C(R^6)(R^7)C(R^8)(R^9)CO-$ and the C-terminal amino acid residue by $-HNC(R^0)(R^1)C(R^2)(R^3)C(R^4)(R^5)C(R^6)(R^7)C(R^8)(R^9)COOH$.

In this specification, the amino acid may be any of natural or non-natural, D- or Land α-, β-, γ- or ε-amino acids.

The group forming a side chain of α-amino acid, β-amino acid, γ-amino acid and ε-amino acid includes, for example, (1) a $C_{1-6}$ alkyl group (e.g., methyl, ethyl, n-propyl, isopropyl, n-butyl, isobutyl, sec-butyl, tert-butyl, n-penlyl, preferably $C_{1-3}$ alkyl), (2) a cyano group, (3) a halogen (e.g., fluorine, chlorine, bromine, iodine), (4) a hydroxy-$C_{1-6}$ alkyl group (e.g., hydroxymethyl, hydroxyethyl), (5) a $C_{1-6}$ alkoxy group (e.g., methoxy, ethoxy, n-propoxy, isopropoxy, n-butoxy, tert-butoxy, preferably $C_{1-3}$ alkoxy), (6) a $C_{1-6}$ alkoxy-carbonyl group (e.g., methoxy carbonyl, ethoxy carbonyl, isopropoxy carbonyl, tert-butoxy carbonyl preferably $C_{1-3}$ alkoxy-carbonyl), (7) a $C_{1-4}$ acyl group (e.g., formyl such as formyl, acetyl, propionyl, butyryl and $C_{2-4}$), alkanoyl), (8) a hydroxy group, (9) a group (e.g., methylthio, methanesulfinyl, methanesulfonyl, ethylthio, ethanesulfinyl, ethanesulfonyl) represented by the formula: $-S(O)a-R^{21}$ wherein a is an integer of 0 to 2, and $R^{21}$ represents a group represented by $C_{1-6}$ alkyl (which is specifically the same as described above), (10) benzyloxy carbonyl, (11) a tosyl group, (12) a carbamoyl group, (13) a mercapto group, (14) an amino group, (15) a sulfo group, (16) a phosphono group, (17) a phospho group, (18) a carboxyl group, (19) a tetrazolyl group, (20) an amino-$C_{1-6}$ alkyl group (e.g., aminomethyl, aminoethyl etc.), (21) an aminoallyl group, (22) a thiazolyl group, (23) a thienyl group, (24) an oxazolyl group, (25) a furyl group, (26) a pyranyl group, (27) a pyridyl group, (28) a pyrazyl group, (29) a pyrazinyl group, (30) a pyrimidinyl group, (31) a pyridazinyl group, (32) an indolyl group, (33) an indozinyl group, (34) an isoindolyl group, (35) a pyrrolyl group, (36) an imidazolyl group, (37) an isothiazolyl group, (38) a pyrazolyl group, (39) a chromenyl group, (40) a purinyl group, (41) a quinolizinyl group, (42) a quinolyl group, (43) an isoquinolyl group, (44) a quinazolinyl group, (45) a quinoxalinyl group, (46) a cinnolinyl group, (47) a morpholinyl group, (48) a benzothienyl group, (49) a benzofuranyl group, (50) benzimidazolyl, (51) a benzimidazolyl group, (52) a $C_{3-8}$ cycloalkyl group, (53) a

$C_{1-4}$ alkyl group substituted with a substituent group described in the items (2), (3), (5) to (17), (20) to (52) above, (54) a $C_{1-4}$ acyl group such as fonnyl, $C_{2-4}$ alkanoyl substituted with a substituent group described in the items (2), (3), (5) to (17), (20) to (52) above, (55) a $C_{6-10}$ aryl group (mesityl, tolyl, xylyl, styrenyl) such as phenyl substituted with a substituent group described in the items (1) to (52) above, (56) a $C_{7-15}$ aralkyl group (methylbenzyl, methoxybenzyl) such as benzyl substituted with a substituent group described in the items (1) to (52) above, (57) a $C_{7-15}$ aralkyl group (benzyl, phenethyl, benzhydryl, naphthylmethyl), (58) a $C_{6-10}$ aryl group (phenyl, naphthyl, indenyl), and (59) a hydrogen atom.

The side chain forming an amino acid residue may be bound to a nitrogen atom to form a ring (e.g., proline etc.), or 2 side chains are bound to each other to form a ring (e.g., 3-aminonorbomanecarboxylic acid).

Examples of the α-amino acid include, for example, glycine, alanine, valine, leucine, isoleucine, serine, threonine, cysteine, methionine, aspartic acid, glutamic acid, lysine, arginine, phenylalanine, tyrosine, histidine, tryptophan, asparagine, glutamine, proline, pipecolic acid, norleucine, γ-methylleucine, tert-leucine, norvaline, homoarginine, homoserine, α-aminoisobutyric acid, α-aminobutyric acid, ornithine, α-aminoadipic acid, phenylglycine, thienylglycine, cyclohexylglycine, cyclohexylalanine, thienylalanine, naphthylalanine, biphenylalanine, p-phosphonomethylphenylalanine, octahydroindole-2-carboxylic acid, o-phosphotyrosine, adamantylalanine, benzothienylalanine, pyridylalanine, piperidylalanine, pyrazylalanine, quinolylalanine, thiazolylalanine, homocysteine, homophenylalanine, citrulline, homocitrulline, oxyproline (hydroxyproline), α,β-diaminopropionic acid, α,γ-diaminobutyric acid, aminomalonic acid, 1,2,3,4-tetrahydroisoquinoline-3-carboxylic acid, penicillamine, cycloleucine, 2-amino-4-pentenoic acid.

Examples of the β-amino acid include, for example, β-alanine, β-aminobutyric acid, isoasparagine, 3-aminoadipic acid, 3-aminophenylpropionic acid, 3-amino-2-hydroxy-4-phenylbutyric acid, 3-aminonorbornanecarboxylic acid, 3-aminobicyloheptanecarboxylic acid.

Examples of the γ-amino acid include, for example, γ-aminobutyric acid, isoglutamine, statine, 4-amino-3-hydroxy-5-cyclohexylpentanoic acid, 4-amino-3-hydroxy-5-phenylpentanoic acid, 6-aminopenicillaminic acid, 3-aminoadmantane-1-carboxylic acid.

Examples of the ε-amino acid include, for example, ε-aminocaproic acid, 4-aminomethyl-cyclohexanecarboxylic acid.

The natural amino acid includes glycine, alanine, valine, leucine, isoleucine, serine, threonine, cysteine, cystine, methionine, aspartic acid, glutamic acid, lysine, arginine, phenylalanine, tyrosine, histidine, tryptophan, asparagine, glutamine, proline, ornithine, citrulline.

The non-natural amino acid includes N-methylated amino acids derived from norleucine, γ-methylleucine, tert-leucine, norvaline, homoarginine, homoserine, aminoisobutyric acid, aminoadipic acid (e.g., α-aminoadipic acid), phenylglycine, thienylglycine, cyclohexylglycine, aminobutyric acid, β-alanine, cyclohexylalanine, thienylalanine, naphthylalanine, adamantylalanine, benzothienylalanine, pyridylalanine, piperidylalanine, pyridylalanine, quinolylalanine, thiazolylalanine, isoasparagine, isoglutamine, homocysteine, homophenylalanine, homocitrulline, oxyproline (hydroxyproline), diaminopropionic acid, diaminobutyric acid, aminobenzoic acid, the above-described natural amino acids and non-natural amino acids.

Examples of those substituent groups with which (side chains of) these amino acid residues may be substituted include (1) a $C_{1-6}$ alkyl group (e.g., methyl, ethyl, n-propyl, isopropyl, n-butyl, isobutyl, sec-butyl, tert-butyl, n-pentyl, preferably $C_{1-3}$ alkyl), (2) a cyano group, (3) a halogen (e.g., fluorine, chlorine, bromine, iodine), (4) a hydroxy-$C_{1-6}$ alkyl group (e.g., hydroxymethyl, hydroxy), (5) a $C_{1-6}$ alkoxy group (e.g., methoxy, ethoxy, n-propoxy, isopropoxy, n-butoxy, tert-butoxy, preferably $C_{1-3}$ alkoxy), (6) a $C_{1-6}$ alkoxy-carbonyl group (e.g., methoxy carbonyl, ethoxy carbonyl, isopropoxy carbonyl, tert-butoxy carbonyl, preferably $C_{1-3}$ alkoxy-carbonyl), (7) a $C_{1-4}$ acyl group (e.g., formyl, formyl such as acetyl, propionyl, butyryl and $C_{2-4}$ alkanoyl), (8) a hydroxy group, (9) a group (e.g., methylthio, methanesulfinyl, methanesulfonyl, ethylthio, ethanesulfinyl, ethanesulfonyl) represented by the formula: $-S(O)a-R^{21}$ wherein a is an integer of 0 to 2, and $R^{21}$ represents a group represented by $C_{1-6}$ alkyl (which is specifically the same as described above), (10) benzyloxy carbonyl, (11) a tosyl group, (12) a carbamoyl group, (13) a mercapto group, (14) an amino group, (15) a sulfo group, (16) a phosphono group, (17) a phospho group, (18) a carboxyl group, (19) a tetrazolyl group, (20) an amino-$C_{1-6}$ alkyl group (e.g., aminomethyl, aminoethyl), (21) an aminoallyl group, (22) a thiazolyl group, (23) a thienyl group, (24) an oxazolyl group, (25) a furyl group, (26) a pyranyl group, (27) a pyridyl group, (28) a pyrazyl group, (29) a pyrazinyl group, (30) a pyrinudinyl group, (31) a pyridazinyl group, (32) an indolyl group, (33) an indozinyl group, (34) an isoindolyl group, (35) a pyrrolyl group, (36) an imidazolyl group, (37) an isothiazolyl group, (38) a pyrazolyl group, (39) a chromenyl group, (40) a purinyl group, (41) a quinolizinyl group, (42) a quinolyl group, (43) an isoquinolyl group, (44) a quinazolinyl group, (45) a quinoxalinyl group, (46) a cinnolinyl group, (47) a morpholinyl group, (48) a benzothienyl group, (49) a benzofuranyl group, (50) benzimidazolyl, (51) a benzimidazolyl group, (52) a $C_{3-8}$ cycloalkyl group, (53) an oxo group, (54) a $C_{1-4}$ alkyl group substituted with a substituent group described in the items (2), (3), (5) to (19), (22) to (52) above, (55) a $C_{1-4}$ acyl group such as formyl, $C_{2-4}$ alkanoyl substituted with a substituent group described in the items (2), (3), (5) to (19), (22) to (52) above, (56) a $C_{6-10}$ aryl group (mesityl, tolyl, xylyl, styrenyl) such as phenyl substituted with a substituent group described in the items (1) to (52) above, (57) a $C_{7-15}$ aralkyl group (methylbenzyl, methoxybenzyl) such as benzyl substituted with a substituent group described in the items (1) to (52) above, (58) a $C_{7-15}$ aralkyl group (benzyl, phenethyl, benzhydryl, naphthylmethyl), (59) a $C_{6-10}$ aryl group (phenyl, naphthyl, indenyl), (60) an arginyl group, (61) a histidyl group and (62) an arginyl-arginyl group.

The neutral substituent group includes (1) a $C_{1-6}$ alkyl group (e.g., methyl, ethyl, n-propyl, isopropyl, n-butyl, isobutyl, sec-butyl, tert-butyl, n-pentyl etc., preferably $C_{1-3}$ alkyl), (2) a cyano group, (3) a halogen (e.g., fluorine, chlorine, bromine, iodine), (4) a hydroxy-$C_{1-6}$ alkyl group (e.g., hydroxymethyl, hydroxyethyl), (5) a hydroxy group, (6) a carbamoyl group, (7) a mercapto group, (8) a group represented by the formula: -S(O)a-$R^{21}$ wherein each symbol has the same meaning as defined above, (9) a $C_{6-10}$ aryl group (e.g., phenyl, naphthyl, indenyl, chromenyl), (10) a thienyl group, (11) an oxazolyl group, (12) a furyl group, (13) an indolyl group, (14) an indolizinyl group, (15) an isoindolyl group, (16) a $C_{3-8}$ cycloalkyl group, (17) an oxo group, (18) $C_{1-6}$ alkyl substituted with a substituent group described in the items (2), (3), (5) to (16) above, (19) a $C_{6-10}$ aryl group (mesityl, tolyl, xylyl, styrenyl) such as phenyl, naphthyl etc. substituted with a substituent group described in the items (1) to (16) above, and (20) a $C_{7-15}$ aralkyl group (methylbenzyl, methoxybenzyl) such as benzyl substituted with a substituent group described in the items (1) to (16) above.

The acidic substituent group includes a $C_{1-4}$ alkyl group, a $C_{6-10}$ aryl group such as phenyl and naphthyl, a $C_{7-15}$ aralkyl group such as benzyl, and a carbonyl group, each of which is substituted with a carboxyl group, sulfo group, tetrazolyl group.

The basic substituent group includes (1) an amino-$C_{1-6}$ alkyl group (aminomethyl, aminoethyl), (2) an aminoallyl group, (3) a pyridyl group, (4) a pyradyl group, (5) a pyrazinyl group, (6) a pyridazinyl group, (7) an imidazolyl group, (8) a pyrazolyl group, (9) a pyrazolyl group, (10) a morpholinyl group, (11) an amino group, (12) a $C_{1-4}$ alkyl group substituted with a substituent group described in the items (3) to (10) above, (13) a $C_{7-15}$ aralkyl group such as benzyl substituted with a substituent group described in the items (1) to (11) above, (14) a $C_{6-10}$ aryl group such as phenyl, naphthyl substituted with a substituent group described in the items (1) to (11) above, (15) an arginyl group, (16) a histidyl group and (17) an arginyl-arginyl group.

In this specification, the acidic amino acid (residue) is specifically an amino acid having an acidic group such as carboxyl group, sulfo group or tetrazolyl group in a side chain thereof. Examples thereof include glutamic acid, pyroglutamic acid, aspartic acid, cysteic acid, homocysteic acid, 3-(5-tetrazolyl) alanine, 2-amino-4-(5-tetrazolyl) butyric acid.

In this specification, the basic amino acid (residue) includes, for example, histidine, arginine, ornithine, lysine, diaminopropionic acid, diaminobutyric acid, homoarginine. Examples of the basic amino acid (residue) whose side chain has been substituted include, for example, $N^{\alpha}$-acetylarginine, $N^{\varepsilon}$-tosylarginine, $N^{\varepsilon}$-acetyllysine, $N^{\varepsilon}$-methyllysine, $N^{\varepsilon}$-tosyllysine.

In this specification, the neutral amino acid (residue) is specifically an amino acid such as alanine, valine, norvaline, leucine, isoleucine, alloisoleucine, norleucine, tertiary leucine, γ-methylleucine, proline, phenylglycine, phenylalanine, glutamine, asparagine, serine, threonine, glycine, cysteine, methionine, tryptophan, oxyproline (hydroxyproline), cyclohexylalanine and naphthylalanine.

In this specification, the amino acid (residue) having an aromatic side chain include, for example, tryptophan, phenylalanine, tyrosine, 1-naphthylalanine, 2-naphthylalanine, 2-thienylalanine, histidine, pyridylalanine (2-pyridylalanine), o-methyltyrosine. The amino acid (residue) having an aromatic side chain which has been substituted includes, for example, 3-iodotyrosine, p-phosphonomethylphenylalanine, o-phosphotyrosine.

In this specification, the amino acid (residue) having a hydroxy group in a side chain thereof includes, for example, serine, threonine, tyrosine, oxyproline (hydroxyproline).

The peptide and peptide chain in this specification are represented in accordance with the conventional way of describing peptides, that is, the N-terminus (amino terminus) at the left hand and the C-terminus (carboxyl terminus) at the right hand. In the peptide and peptide chain of the present invention, the C-terminus is usually in the form of a carboxyl group (-COOH) or a carboxylate ($-COO^-$) but may be in the form of an amide ($-CONH_2$) or an ester (-COOR). Examples of the ester group shown by R include a $C_{1-6}$ alkyl group such as methyl, ethyl, n-propyl, isopropyl, n-butyl; a $C_{3-8}$ cycloalkyl group such as cyclopentyl, cyclohexyl; a $C_{6-12}$ aryl group such as phenyl, α-naphthyl; a $C_{7-14}$ aralkyl group such as a phenyl-$C_{1-2}$-alkyl group, e.g., benzyl, phenethyl, benzhydryl, or an α-naphthyl-$C_{1-2}$-alkyl group such as α-naphthylmethyl; and the like. In addition, pivaloyloxymethyl or the like, which is used widely as an ester for oral administration, may also be used.

Where the peptide of the present invention contains a carboxyl group or a carboxylate at a position other than the C-terminus, it may be amidated or esterified, and such an amide or ester is also included within the polypeptide of the present invention. As the ester, for example the C-terminal ester described above or the like is used.

Furthermore, examples of the peptide or peptide chains of the present invention include those peptide or peptide chains, wherein the amino group at the N-terminal amino acid residue of the peptide or peptide chain is protected with a substituent group (for example, [1] a $C_{1-8}$ acyl group such as a $C_{2-6}$ alkanoyl group, e.g., formyl group and acetyl, guanidinoacetyl, thienylacrylyl, pyridylacetyl, [2] a $C_{1-6}$ alkyl group such as methyl, ethyl, propyl, isopropyl, butyl, isobutyl, sec-butyl, tert-butyl, pentyl, hexyl, [3] a $C_{6-10}$ aryl group such as phenyl, naphthyl or a $C_{7-16}$ aralkyl group such as benzyl, phenethyl [4] a tosyl group, [5] a benzyloxycarbonyl group, [6] a group (e.g., methylthio, methanesulfinyl, methanesulfonyl, ethylthio, ethanesulfinyl, ethanesulfonyl) represented by the formula: -S(O)a-$R^{22}$ wherein a is an integer of 0 to 2, and $R^{22}$ is a $C_{1-6}$ alkyl (which is specifically the same as described above), [7] a t-butoxycarbonyl group, [8] a N-9-fluorenyl methoxycarbonyl group); those wherein the N-terminal region is cleaved in vivo and the glutamyl group thus formed is pyroglutaminated; those wherein a substituent (e.g., -OH, -SH, amino group, imidazolyl group, indolyl group, guanidino group) on the side chain of an amino acid in the molecule is protected with a suitable protecting group (e.g., a $C_{1-6}$ acyl group such as a $C_{1-6}$ alkanoyl group, e.g., formyl group, acetyl group), or conjugated proteins such as glycoproteins bound to sugar chains.

In the above formula

Pl-Arg-Pro-Arg-Leu-Phe-P2-P3-Gly-Pro-P4-P5 (I),

(a side chain of) each amino acid residue may be substituted, and the substituent group includes those described above.

In this specification, P1 represents "a hydrogen atom or amino acid residue, or a peptide chain, consisting of 1 to 25 amino acids which may be the same or different and whose side chain may be substituted".

The substituent groups on the "1 to 25 amino acids whose side chain may be substituted" include, for example, those identical with the "substituent groups with which side chains of these amino acid residues may be substituted".

When P1 represents the "amino acids whose side chain may be substituted", preferable examples thereof include, for example, optionally substituted pyroglutamic acid or glutamine whose side chain may be substituted, more preferably pyroglutamic acid or glutamine.

In the "amino acid residues whose side chain may be substituted", "optionally substituted pyroglutamic acid" and "glutamine whose side chain may be substituted", the substituent groups on the amino acid residues include, for example, those identical with the "substituent groups with which side chains of these amino acid residues may be substituted".

As a preferable substituent group on the "optionally substituted pyroglutamic acid", a benzyloxycaronyl group or the like is exemplified.

When P1 represents the "peptide chain consisting of 2 to 25 amino acids which may be the same or different and whose side chain may be substituted", examples thereof include, for example, peptides represented by the formula:

$Y^1$-$Y^2$

wherein $Y^1$ represents amino acid residues, or a peptide chain, consisting of 1 to 17 amino acids which may be the same or different and whose side chain may be substituted, and $Y^2$ represents amino acid residues, or a peptide chain, consisting of 1 to 8 amino acids which may be the same or different and whose side chain may be substituted.

The substituent groups on side chains of amino acid residues, or of amino acid residues in a peptide chain, represented by the above-mentioned $Y^1$ and $Y^2$ include, for example, those identical with the "substituent groups with which side chains of these amino acid residues may be substituted".

Examples of the above-mentioned $Y^1$ include, for example, amino acid residues or peptide chains represented by:

(a) the formula $A^1$-$A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ wherein $A^1$ to $A^{17}$ are the same or different and represent an amino acid residue whose side chain may be substituted,

(b) the formula $A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ wherein each symbol has the same meaning as defined above,

(c) the formula $A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ wherein each symbol has the same meaning as defined above,

(d) the formula $A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ wherein each symbol has the same meaning as defined above,

(e) the formula $A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$ wherein each symbol has the same meaning as defined above,

(f) the formula $A^6$-$A^7$-$A^8$-$A^9$-$A^{10}$-$A^{11}$-$A^{12}$-$A^{13}$-$A^{14}$-$A^{15}$-$A^{16}$-$A^{17}$ wherein each symbol has the same meaning as defined above,

(g) the formula $A^7$-$A^8$-$A^9$-$A^{10}$-$A^{11}$-$A^{12}$-$A^{13}$-$A^{14}$-$A^{15}$-$A^{16}$-$A^{17}$ wherein each symbol has the same meaning as defined above,

(h) the formula $A^8$-$A^9$-$A^{10}$-$A^{11}$-$A^{12}$-$A^{13}$-$A^{14}$-$A^{15}$-$A^{16}$-$A^{17}$ wherein each symbol has the same meaning as defined above,

(i) the formula $A^9$-$A^{10}$-$A^{11}$-$A^{12}$-$A^{13}$-$A^{14}$-$A^{15}$-$A^{16}$-$A^{17}$ wherein each symbol has the same meaning as defined above,

(j) the formula $A^{10}$-$A^{11}$-$A^{12}$-$A^{13}$-$A^{14}$-$A^{15}$-$A^{16}$-$A^{17}$ wherein each symbol has the same meaning as defined above,

(k) the formula $A^{11}$-$A^{12}$-$A^{13}$-$A^{14}$-$A^{15}$-$A^{16}$-$A^{17}$ wherein each symbol has the same meaning as defined above,
(l) the formula $A^{12}$-$A^{13}$-$A^{14}$-$A^{15}$-$A^{16}$-$A^{17}$ wherein each symbol has the same meaning as defined above,
(m) the formula $A^{13}$-$A^{14}$-$A^{15}$-$A^{16}$-$A^{17}$ wherein each symbol has the same meaning as defined above,
(n) the formula $A^{14}$-$A^{15}$-$A^{16}$-$A^{17}$ wherein each symbol has the same meaning as defined above,
(o) the formula $A^{15}$-$A^{16}$-$A^{17}$ wherein each symbol has the same meaning as defined above,
(p) the formula $A^{16}$-$A^{17}$ wherein each symbol has the same meaning as defined above, and
(q) the formula $A^{17}$ wherein $A^{17}$ has the same meaning as defined above.

The above-mentioned $A^1$ represents an amino acid residue whose side chain may be substituted, preferably an amino acid residue having an aromatic side chain, more preferably an L-amino acid residue having an aromatic side chain, still more preferably L-tyrosine.

The above-mentioned $A^2$ and $A^3$ are the same or different, and represent an amino acid residue whose side chain may be substituted, preferably a neutral amino acid residue whose side chain may be substituted, more preferably a neutral L-amino acid residue whose side chain may be substituted, and still more preferably $A^2$ is L-leucine or the like and $A^3$ is L-valine or the like.

The above-mentioned $A^4$ represents an amino acid residue whose side chain may be substituted, preferably a neutral or basic amino acid residue whose side chain may be substituted, more preferably a neutral or basic L-amino acid residue whose side chain may be substituted, still more preferably L-lysine or $N^{\varepsilon}$-acetyllysine.

The above-mentioned $A^5$ represents an amino acid residue whose side chain may be substituted, preferably a neutral amino acid residue whose side chain may be substituted, more preferably optionally substituted L-proline, still more preferably L-proline or the like.

The above-mentioned $A^6$ and $A^9$ are the same or different, and represent an amino acid residue whose side chain may be substituted, preferably a basic amino acid residue whose side chain may be substituted, more preferably a basic L-amino acid residue whose side chain may be substituted, still more preferably L-arginine or the like.

The above-mentioned $A^7$ and $A^{10}$ are the same or different, and represent an amino acid residue whose side chain may be substituted, preferably an amino acid residue having a hydroxy group in a side chain thereof or a neutral amino acid residue whose side chain may be substituted.

As $A^7$, optionally substituted glycine, particularly glycine or the like, is preferably used.

The above-mentioned $A^8$ represents an amino acid residue whose side chain may be substituted, preferably L-proline and an amino acid residue having a hydroxy group in a side chain thereof, more preferably L-serine, L-proline or oxyproline (hydroxyproline).

The above-mentioned $A^{10}$ represents an amino acid residue having a hydroxy group in a side chain thereof or a neutral amino acid residue whose side chain may be substituted, more preferably serine, threonine, asparagine.

The above-mentioned $A^{11}$ and $A^{14}$ are the same or different, and represent an amino acid residue whose side chain may be substituted, preferably a neutral amino acid residue whose side chain may be substituted, and more preferably $A^{11}$ is glycine, $A^{12}$ is L-proline, $A^{13}$ is glycine, $A^{14}$ is L-alanine, L-proline or the like.

The above-mentioned $A^{15}$ represents an amino acid residue whose side chain may be substituted, preferably an amino acid residue having an aromatic side chain, more preferably an L-amino acid residue having an aromatic side chain, still more preferably L-tryptophan.

The above-mentioned $A^{16}$ represents an amino acid residue whose side chain may be substituted, preferably a neutral amino acid residue whose side chain may be substituted, more preferably a neutral L-amino acid residue having a carbamoyl group, still more preferably L-glutanine.

The neutral L-amino acid residue having a carbamoyl group includes, for example, L-glutamine, L-asparagine.

The above-mentioned $A^{17}$ represents an amino acid residue whose side chain may be substituted, preferably a neutral amino acid residue whose side chain may be substituted, more preferably glycine.

Examples of the above-mentioned $Y^2$ include, for example, amino acid residues or peptide chains represented by:

[1] the formula $B^1$-$B^2$-$B^3$-$B^4$-$B^5$-$B^6$-$B^7$-$B^8$ wherein $B^1$ to $B^8$ are the same or different, and represent an amino acid residue whose side chain may be substituted,
[2] the formula $B^2$-$B^3$-$B^4$-$B^5$-$B^6$-$B^7$-$B^8$ wherein each symbol has the same meaning as defined above,
[3] the formula $B^3$-$B^4$-$B^5$-$B^6$-$B^7$-$B^8$ wherein each symbol has the same meaning as defined above,
[4] the formula $B^4$-$B^5$-$B^6$-$B^7$-$B^8$ wherein each symbol has the same meaning as defined above,
[5] the formula $B^5$-$B^6$-$B^7$-$B^8$ wherein each symbol has the same meaning as defined above,
[6] the formula $B^6$-$B^7$-$B^8$ wherein each symbol has the same meaning as defined above,
[7] the formula $B^7$-$B^8$ wherein each symbol has the same meaning as defined above, and
[8] the formula $B^8$ wherein $B^8$ has the same meaning as defined above.

The above-mentioned $B^1$ represents an amino acid residue whose side chain may be substituted, preferably a neutral amino acid residue whose side chain may be substituted, more preferably a neutral L-amino acid residue whose side chain may be substituted, still more preferably optionally substituted glycine, most preferably glycine.

The above-mentioned $B^2$ to $B^4$ are the same or different, and represent an amino acid residue whose side chain may be substituted, preferably a basic amino acid residue whose side chain may be substituted, more preferably a basic L-amino acid residue whose side chain may be substituted, and still more preferably $B^2$ is L-arginine, $B^3$ is L-arginine, and $B^4$ is L-lysine.

The above-mentioned $B^5$ represents an amino acid residue whose side chain may be substituted, preferably an amino acid residue having an aromatic side chain, more preferably an L-amino acid residue having an aromatic side chain, still more preferably L-phenylalanine.

The above-mentioned $B^6$ and $B^7$ are the same or different, and represent an amino acid residue whose side chain may be substituted, preferably a basic amino acid residue whose side chain may be substituted, more preferably a basic L-amino acid residue whose side chain may be substituted, further more preferably L-arginine.

The above-mentioned $B^8$ represents an amino acid residue whose side chain may be substituted, preferably glutamine whose side chain may be substituted, more preferably L-glutamine.

Combinations of $Y^1$ and $Y^2$ include:

the case of the formula represented by the above-mentioned (a) + the formula represented by the above-mentioned [1] (that is, the case where $X^1$ is represented by $A^1$-$A^2$-$A^3$-$A^4$-$A^5$-$A^6$-$A^7$-$A^8$-$A^9$-$A^{10}$-$A^{11}$-$A^{12}$-$A^{13}$-$A^{14}$-$A^{15}$-$A^{16}$-$A^{17}$-$B^1$-$B^2$-$B^3$-$B^4$-$B^5$-$B^6$-$B^7$-$B^8$; this will be omitted in the description of the following combinations),
the case of the formula represented by the above-mentioned (a) + the formula represented by the above-mentioned [2],
the case of the formula represented by the above-mentioned (a) + the formula represented by the above-mentioned [3],
the case of the formula represented by the above-mentioned (a) + the formula represented by the above-mentioned [4],
the case of the formula represented by the above-mentioned (a) + the formula represented by the above-mentioned [5],
the case of the formula represented by the above-mentioned (a) + the formula represented by the above-mentioned [6],
the case of the formula represented by the above-mentioned (a) + the formula represented by the above-mentioned [7],
the case of the formula represented by the above-mentioned (a) + the formula represented by the above-mentioned [8],
the case of the formula represented by the above-mentioned (b) + the formula represented by the above-mentioned [1],
the case of the formula represented by the above-mentioned (b) + the formula represented by the above-mentioned [2],
the case of the formula represented by the above-mentioned (b) + the formula represented by the above-mentioned [3],
the case of the formula represented by the above-mentioned (b) + the formula represented by the above-mentioned [4],
the case of the formula represented by the above-mentioned (b) + the formula represented by the above-mentioned [5],
the case of the formula represented by the above-mentioned (b) + the formula represented by the above-mentioned [6],
the case of the formula represented by the above-mentioned (b) + the formula represented by the above-mentioned [7],
the case of the formula represented by the above-mentioned (b) + the formula represented by the above-mentioned [8],
the case of the formula represented by the above-mentioned (c) + the formula represented by the above-mentioned [1],
the case of the formula represented by the above-mentioned (c) + the formula represented by the above-mentioned [2],
the case of the formula represented by the above-mentioned (c) + the formula represented by the above-mentioned [3],
the case of the formula represented by the above-mentioned (c) + the formula represented by the above-mentioned [4],

the case of the formula represented by the above-mentioned (c) + the formula represented by the above-mentioned [5],
the case of the formula represented by the above-mentioned (c) + the formula represented by the above-mentioned [6],
the case of the formula represented by the above-mentioned (c) + the formula represented by the above-mentioned [7],
the case of the formula represented by the above-mentioned (c) + the formula represented by the above-mentioned [8],
the case of the formula represented by the above-mentioned (d) + the formula represented by the above-mentioned [1],
the case of the formula represented by the above-mentioned (d) + the formula represented by the above-mentioned [2],
the case of the formula represented by the above-mentioned (d) + the formula represented by the above-mentioned [3],
the case of the formula represented by the above-mentioned (d) + the formula represented by the above-mentioned [4],
the case of the formula represented by the above-mentioned (d) + the formula represented by the above-mentioned [5],
the case of the formula represented by the above-mentioned (d) + the formula represented by the above-mentioned [6],
the case of the formula represented by the above-mentioned (d) + the formula represented by the above-mentioned [7],
the case of the formula represented by the above-mentioned (d) + the formula represented by the above-mentioned [8],
the case of the formula represented by the above-mentioned (e) + the formula represented by the above-mentioned [1],
the case of the formula represented by the above-mentioned (e) + the formula represented by the above-mentioned [2],
the case of the formula represented by the above-mentioned (e) + the formula represented by the above-mentioned [3],
the case of the formula represented by the above-mentioned (e) + the formula represented by the above-mentioned [4],
the case of the formula represented by the above-mentioned (e) + the formula represented by the above-mentioned [5],
the case of the formula represented by the above-mentioned (e) + the formula represented by the above-mentioned [6],
the case of the formula represented by the above-mentioned (e) + the formula represented by the above-mentioned [7],
the case of the formula represented by the above-mentioned (e) + the formula represented by the above-mentioned [8],
the case of the formula represented by the above-mentioned (f) + the formula represented by the above-mentioned [1],
the case of the formula represented by the above-mentioned (f) + the formula represented by the above-mentioned [2],
the case of the formula represented by the above-mentioned (f) + the formula represented by the above-mentioned [3],
the case of the formula represented by the above-mentioned (f) + the formula represented by the above-mentioned [4],
the case of the formula represented by the above-mentioned (f) + the formula represented by the above-mentioned [5],
the case of the formula represented by the above-mentioned (f) + the formula represented by the above-mentioned [6],
the case of the formula represented by the above-mentioned (f) + the formula represented by the above-mentioned [7],
the case of the formula represented by the above-mentioned (f) + the formula represented by the above-mentioned [8],
the case of the formula represented by the above-mentioned (g) + the formula represented by the above-mentioned [1],
the case of the formula represented by the above-mentioned (g) + the formula represented by the above-mentioned [2],
the case of the formula represented by the above-mentioned (g) + the formula represented by the above-mentioned [3],
the case of the formula represented by the above-mentioned (g) + the formula represented by the above-mentioned [4],
the case of the formula represented by the above-mentioned (g) + the formula represented by the above-mentioned [5],

the case of the formula represented by the above-mentioned (g) + the formula represented by the above-mentioned [6],
the case of the formula represented by the above-mentioned (g) + the formula represented by the above-mentioned [7],
the case of the formula represented by the above-mentioned (g) + the formula represented by the above-mentioned [8],
the case of the formula represented by the above-mentioned (h) + the formula represented by the above-mentioned [1],
the case of the formula represented by the above-mentioned (h) + the formula represented by the above-mentioned [2],
the case of the formula represented by the above-mentioned (h) + the formula represented by the above-mentioned [3],
the case of the formula represented by the above-mentioned (h) + the formula represented by the above-mentioned [4],
the case of the formula represented by the above-mentioned (h) + the formula represented by the above-mentioned [5],
the case of the formula represented by the above-mentioned (h) + the formula represented by the above-mentioned [6],
the case of the formula represented by the above-mentioned (h) + the formula represented by the above-mentioned [7],
the case of the formula represented by the above-mentioned (h) + the formula represented by the above-mentioned [8],
the case of the formula represented by the above-mentioned (i) + the formula represented by the above-mentioned [1],
the case of the formula represented by the above-mentioned (i) + the formula represented by the above-mentioned [2],
the case of the formula represented by the above-mentioned (i) + the formula represented by the above-mentioned [3],
the case of the formula represented by the above-mentioned (i) + the formula represented by the above-mentioned [4],
the case of the formula represented by the above-mentioned (i) + the formula represented by the above-mentioned [5],
the case of the formula represented by the above-mentioned (i) + the formula represented by the above-mentioned [6],
the case of the formula represented by the above-mentioned (i) + the formula represented by the above-mentioned [7],
the case of the formula represented by the above-mentioned (i) + the formula represented by the above-mentioned [8],
the case of the formula represented by the above-mentioned (j) + the formula represented by the above-mentioned [1],
the case of the formula represented by the above-mentioned (j) + the formula represented by the above-mentioned [2],
the case of the formula represented by the above-mentioned (j) + the formula represented by the above-mentioned [3],
the case of the formula represented by the above-mentioned (j) + the formula represented by the above-mentioned [4],
the case of the formula represented by the above-mentioned (j) + the formula represented by the above-mentioned [5],
the case of the formula represented by the above-mentioned (j) + the formula represented by the above-mentioned [6],
the case of the formula represented by the above-mentioned (j) + the formula represented by the above-mentioned [7],
the case of the formula represented by the above-mentioned (j) + the formula represented by the above-mentioned [8],
the case of the formula represented by the above-mentioned (k) + the formula represented by the above-mentioned [1],
the case of the formula represented by the above-mentioned (k) + the formula represented by the above-mentioned [2],
the case of the formula represented by the above-mentioned (k) + the formula represented by the above-mentioned [3],
the case of the formula represented by the above-mentioned (k) + the formula represented by the above-mentioned [4],
the case of the formula represented by the above-mentioned (k) + the formula represented by the above-mentioned [5],
the case of the formula represented by the above-mentioned (k) + the formula represented by the above-mentioned [6],
the case of the formula represented by the above-mentioned (k) + the formula represented by the above-mentioned [7],
the case of the formula represented by the above-mentioned (k) + the formula represented by the above-mentioned [8],
the case of the formula represented by the above-mentioned (1) + the formula represented by the above-mentioned [1],
the case of the formula represented by the above-mentioned (1) + the formula represented by the above-mentioned [2],

the case of the formula represented by the above-mentioned (l) + the formula represented by the above-mentioned [3],
the case of the formula represented by the above-mentioned (1) + the formula represented by the above-mentioned [4],
the case of the formula represented by the above-mentioned (l) + the formula represented by the above-mentioned [5],
the case of the formula represented by the above-mentioned (l) + the formula represented by the above-mentioned [6],
the case of the formula represented by the above-mentioned (l) + the formula represented by the above-mentioned [7],
the case of the formula represented by the above-mentioned (l) + the formula represented by the above-mentioned [8],
the case of the formula represented by the above-mentioned (m) + the formula represented by the above-mentioned [1],
the case of the formula represented by the above-mentioned (m) + the formula represented by the above-mentioned [2],
the case of the formula represented by the above-mentioned (m) + the formula represented by the above-mentioned [3],
the case of the formula represented by the above-mentioned (m) + the formula represented by the above-mentioned [4],
the case of the formula represented by the above-mentioned (m) + the formula represented by the above-mentioned [5],
the case of the formula represented by the above-mentioned (m) + the formula represented by the above-mentioned [6],
the case of the formula represented by the above-mentioned (m) + the formula represented by the above-mentioned [7],
the case of the formula represented by the above-mentioned (m) + the formula represented by the above-mentioned [8],
the case of the formula represented by the above-mentioned (n) + the formula represented by the above-mentioned [1],
the case of the formula represented by the above-mentioned (n) + the formula represented by the above-mentioned [2],
the case of the formula represented by the above-mentioned (n) + the formula represented by the above-mentioned [3],
the case of the formula represented by the above-mentioned (n) + the formula represented by the above-mentioned [4],
the case of the formula represented by the above-mentioned (n) + the formula represented by the above-mentioned [5],
the case of the formula represented by the above-mentioned (n) + the formula represented by the above-mentioned [6],
the case of the formula represented by the above-mentioned (n) + the formula represented by the above-mentioned [7],
the case of the formula represented by the above-mentioned (n) + the formula represented by the above-mentioned [8],
the case of the formula represented by the above-mentioned (o) + the formula represented by the above-mentioned [1],
the case of the formula represented by the above-mentioned (o) + the formula represented by the above-mentioned [2],
the case of the formula represented by the above-mentioned (o) + the formula represented by the above-mentioned [3],
the case of the formula represented by the above-mentioned (o) + the formula represented by the above-mentioned [4],
the case of the formula represented by the above-mentioned (o) + the formula represented by the above-mentioned [5],
the case of the formula represented by the above-mentioned (o) + the formula represented by the above-mentioned [6],
the case of the formula represented by the above-mentioned (o) + the formula represented by the above-mentioned [7],
the case of the formula represented by the above-mentioned (o) + the formula represented by the above-mentioned [8],
the case of the formula represented by the above-mentioned (p) + the formula represented by the above-mentioned [1],
the case of the formula represented by the above-mentioned (p) + the formula represented by the above-mentioned [2],
the case of the formula represented by the above-mentioned (p) + the formula represented by the above-mentioned [3],
the case of the formula represented by the above-mentioned (p) + the formula represented by the above-mentioned [4],
the case of the formula represented by the above-mentioned (p) + the formula represented by the above-mentioned [5],
the case of the formula represented by the above-mentioned (p) + the formula represented by the above-mentioned [6],
the case of the formula represented by the above-mentioned (p) + the formula represented by the above-mentioned [7],
the case of the formula represented by the above-mentioned (p) + the formula represented by the above-mentioned [8],
the case of the formula represented by the above-mentioned (q) + the formula represented by the above-mentioned [1],
the case of the formula represented by the above-mentioned (q) + the formula represented by the above-mentioned [2],
the case of the formula represented by the above-mentioned (q) + the formula represented by the above-mentioned [3],
the case of the formula represented by the above-mentioned (q) + the formula represented by the above-mentioned [4],
the case of the formula represented by the above-mentioned (q) + the formula represented by the above-mentioned [5],
the case of the formula represented by the above-mentioned (q) + the formula represented by the above-mentioned [6],
the case of the formula represented by the above-mentioned (q) + the formula represented by the above-mentioned [7], and
the case of the formula represented by the above-mentioned (q) + the formula represented by the above-mentioned [8],

among which particularly preferable are:

the case of the formula represented by the above-mentioned (a) + the formula represented by the above-mentioned [1],
the case of the formula represented by the above-mentioned (b) + the formula represented by the above-mentioned [1],
the case of the formula represented by the above-mentioned (c) + the formula represented by the above-mentioned [1],
the case of the formula represented by the above-mentioned (d) + the formula represented by the above-mentioned [1],
the case of the formula represented by the above-mentioned (e) + the formula represented by the above-mentioned [1],
the case of the formula represented by the above-mentioned (f) + the formula represented by the above-mentioned [1],
the case of the formula represented by the above-mentioned (g) + the formula represented by the above-mentioned [1],
the case of the formula represented by the above-mentioned (h) + the formula represented by the above-mentioned [1],
the case of the formula represented by the above-mentioned (i) + the formula represented by the above-mentioned [1],
the case of the formula represented by the above-mentioned (j) + the formula represented by the above-mentioned [1],
the case of the formula represented by the above-mentioned (k) + the formula represented by the above-mentioned [1],
the case of the formula represented by the above-mentioned (l) + the formula represented by the above-mentioned [1],
the case of the formula represented by the above-mentioned (m) + the formula represented by the above-mentioned [1],
the case of the formula represented by the above-mentioned (n) + the formula represented by the above-mentioned [1],
the case of the formula represented by the above-mentioned (o) + the formula represented by the above-mentioned [1],

the case of the formula represented by the above-mentioned (p) + the formula represented by the above-mentioned [1], and

the case of the formula represented by the above-mentioned (q) + the formula represented by the above-mentioned [1].

In particular, the case of the formula represented by the above-mentioned (a) + the formula represented by the above-mentioned [1] and the case of the formula represented by the above-mentioned (b) + the formula represented by the above-mentioned [1] are mentioned as more preferable examples.

Still more preferable examples of the case of the formula represented by the above-mentioned (a) + the formula represented by the above-mentioned [1] and the case of the formula represented by the above-mentioned (b) + the formula represented by the above-mentioned [1] are shown below.

The case of the formula represented by the above-mentioned (b) + the formula represented by the above-mentioned [1] refers to the case where P1 is represented by the formula

$A^2\text{-}A^3\text{-}A^4\text{-}A^5\text{-}A^6\text{-}A^7\ \text{-}A^8\text{-}A^9\text{-}A^{10}\text{-}A^{11}\text{-}A^{12}\text{-}A^{13}\text{-}A^{14}\text{-}A^{15}\text{-}A^{16}\text{-}A^{17}\text{-}B^1\text{-}B^2\text{-}B^3\text{-}B^4\text{-}B^5\text{-}B^6\text{-}B^7\text{-}B^8$ wherein $A^2$ to $A^{17}$ and $B^1$ to $B^8$ have the same meaning as defined above, and in preferable examples in this case,

$A^2$ and $A^3$ are the same or different, and represent a neutral L-amino acid residue whose side chain may be substituted, and preferably $A^2$ is L-leucine or the like and $A^3$ is L-valine or the like,

$A^4$ is a neutral or basic L-amino acid residue whose side chain may be substituted, preferably L-glutamine, L-lysine or $N^{\varepsilon}$-acetyllysine,

$A^5$ is L-proline whose side chain may be substituted, preferably L-proline or the like,

$A^6$ and $A^9$ are the same or different, and represent a basic L-amino acid residue whose side chain may be substituted, preferably L-arginine,

$A^7$ is optionally substituted glycine, preferably glycine or the like,

$A^8$ is L-proline or an amino acid residue having a hydroxy group in a side chain thereof, preferably L-serine, L-proline or oxyproline (hydroxyproline),

$A^{10}$ is an amino acid residue having a hydroxy group in a side chain thereof or a neutral amino acid residue whose side chain may be substituted, preferably L-serine, L-threonine, L-asparagine or the like,

$A^{11}$ is glycine, $A^{12}$ is L-proline, $A^{13}$ is glycine, and $A^{14}$ is L-alanine or L-proline,

$A^{15}$ is an L-amino acid residue having an aromatic side chain, preferably L-tryptophan or the like,

$A^{16}$ is a neutral L-amino acid residue having a carbamoyl group, preferably L-glutamine,

$A^{17}$ is a neutral amino acid residue, preferably glycine,

$B^1$ is a neutral L-amino acid residue whose side chain may be substituted, preferably optionally substituted glycine, more preferably glycine or the like,

$B^2$ and $B^4$ are the same or different, and represent a basic L-amino acid residue whose side chain may be substituted, and preferably $B^2$ is L-arginine, $B^3$ is L-arginine, and $B^4$ is L-lysine,

$B^5$ is an L-amino acid residue having an aromatic side chain, preferably L-phenylalanine or the like,

$B^6$ and $B^7$ are the same or different, and represent a basic L-amino acid residue whose side chain may be substituted, preferably L-arginine or the like, and

$B^8$ is glutamine whose side chain may be substituted, preferably L-glutamine or the like.

The case of the formula represented by the above-mentioned (a) + the formula represented by the above-mentioned [1] refers to the case where $X^1$ is represented by the formula

$A^1\text{-}A^2\text{-}A^3\text{-}A^4\text{-}A^5\text{-}A^6\text{-}A^7\text{-}A^8\text{-}A^9\text{-}A^{10}\text{-}A^{11}A^{12}\text{-}A^{13}\text{-}A^{14}\text{-}A^{17}\text{-}B^1\text{-}B^2\text{-}B^3\text{-}B^3\text{-}B^4\text{-}B^4\text{-}B^6\text{-}B^7\text{-}B^8$ wherein $A^1$ to $A^{17}$ and $B^1$ to $B^8$ have the same meaning as defined above, and in preferable examples in this case,

$A^1$ is an L-amino acid residue having an aromatic side chain, more preferably L-tyrosine or the like,

$A^2$ and $A^3$ are the same or different, and represent a neutral L-amino acid residue whose side chain may be substituted, and preferably $A^2$ is L-leucine or the like, and $A^3$ is L-valine or the like,

$A^4$ is a neutral or basic L-amino acid residue whose side chain may be substituted, preferably L-glutamine, L-$\alpha$-aminoadipic acid, L-lysine or $N^{\varepsilon}$-acetyllysine,

$A^5$ is L-proline whose side chain may be substituted, preferably L-proline or the like,

$A^6$ and $A^9$ are the same or different, and represent a basic L-amino acid residue whose side chain may be substituted, preferably L-arginine or the like,

$A^7$ is optionally substituted glycine, preferably glycine or the like,

$A^8$ is L-proline or an amino acid residue having a hydroxy group in a side chain thereof, preferably L-serine, L-proline or oxyproline (hydroxyproline),

$A^{10}$ is an amino acid residue having a hydroxy group in a side chain thereof or a neutral amino acid residue whose side chain may be substituted, preferably L-serine, L-threonine, L-asparagine or the like,

$R^{11}$ is glycine, $A^{12}$ is L-proline, $A^{13}$ is glycine, and $A^{14}$ is L-alanine or L-proline,

$A^{15}$ is an L-amino acid residue having an aromatic side chain, preferably L-tryptophan or the like,

$A^{16}$ is a neutral L-amino acid residue having a carbamoyl group, preferably L-glutamine or the like,
$A^{17}$ is a neutral amino acid residue, preferably glycine or the like,
$B^1$ is a neutral L-amino acid residue whose side chain may be substituted, preferably optionally substituted glycine, more preferably glycine or the like,
$B^2$ and $B^4$ are the same or different, and represent a basic L-amino acid residue whose side chain may be substituted, and preferably $B^2$ is L-arginine, $B^3$ is L-arginine, and $B^4$ is L-lysine,
$B^5$ is an L-amino acid residue having an aromatic side chain, preferably L-phenylalanine or the like,
$B^6$ and $B^7$ are the same or different, and represent a basic L-amino acid residue whose side chain may be substituted, preferably L-arginine or the like, and
$B^8$ is glutamine whose side chain may be substituted, preferably L-glutamine-L-pyroglutamic acid or the like.

Preferable examples of P1 include:

(1) hydrogen atom,
(2) Arg-Gln,
(3) Arg,
(4) Gln,
(5) pGlu, or
(6) Arg-Arg-Gln or the like.

P1 is more preferably a hydrogen atom, pGlu or Arg-Arg-Gln.

In this specification, P2 represents a neutral amino acid residue whose side chain may be substituted or a basic amino acid residue whose side chain may be substituted, preferably L-alanine which may be substituted (in a side chain thereof), L-histidine which may be substituted (in a side chain thereof).

In this specification, P3 represents a neutral amino acid residue whose side chain may be substituted, an aromatic amino acid residue whose side chain may be substituted, or a basic amino acid residue whose side chain may be substituted. The substituent group on a side chain of the basic amino acid residue includes, for example, a $C_{1-4}$ acyl group, a tosyl group, a $C_{1-6}$ alkyl group.

The $C_{1-4}$ acyl group includes, for example, a $C_{1-4}$ acyl group such as formyl, acetyl, propionyl and butyryl and $C_{2-4}$ alkanoyl group.

The $C_{1-6}$alkyl group includes, for example, methyl, ethyl, propyl, isopropyl, butyl, isobutyl, sec-butyl, tert-butyl, pentyl, hexyl.

P3 is preferably L-lysine whose side chain may be substituted, L-norleucine whose side chain may be substituted or L-arginine whose side chain may be substituted, more preferably L-lysine, L-norleucine or L-arginine whose side chain may be substituted with a $C_{1-4}$ acyl group (e.g., formyl, acetyl, propionyl, butyryl and $C_{2-4}$ alkanoyl), a $C_{1-6}$ alkyl group (e.g., methyl, ethyl, propyl, isopropyl, butyl, isobutyl, sec-butyl, tert-butyl, pentyl, hexyl etc.). or a tosyl group, more preferably L-lysine, L-norleucine, L-arginine, $N^{\varepsilon}$-acetyllysine, $N^{\varepsilon}$-methyllysine, $N^{\varepsilon}$-tosyllysine, $N^{g}$-tosylarginine.

Preferably, P3 is optionally substituted L-arginine or optionally substituted L-lysine.

In this specification, P4 represents a bond, a neutral amino acid whose side chain may be substituted or an aromatic amino acid residue whose side chain may be substituted. P4 is preferably a bond, L-norleucine whose side chain may be substituted, L-methionine whose side chain may be substituted, L-methionine sulfoxide whose side chain may be substituted or L-alanine whose side chain may be substituted, more preferably a bond, L-norleucine, L-methionine, L-methionine sulfoxide or L-cyclohexylalanine.

Further, P4 is particularly preferably L-norleucine, L-methionine or L-cyclohexylalanine.

In this specification, P5 represents [1] an amino acid residue whose side chain may be substituted, or its amino acid derivative wherein the C-terminal carboxyl group has been reduced to a hydroxymethyl group or formyl group, [2] a hydroxyl group, or [3] a dipeptide chain formed by binding an amino acid residue whose side chain may be substituted, to an amino acid residue whose side chain may be substituted, or its peptide derivative wherein the C-terminal carboxyl group has been reduced to a hydroxymethyl group or formyl group.

P5 is preferably [1] an amino acid residue whose side chain may be substituted, or its amino acid derivative wherein the C-terminal carboxyl group has been reduced to a hydroxymethyl group or formyl group, [2] a hydroxyl group or [3] a dipeptide chain formed by binding a neutral amino acid residue whose side chain may be substituted, to an amino acid residue having an aromatic side group which may be substituted, or its peptide derivative wherein the C-terminal carboxyl group has been reduced to a hydroxymethyl group or formyl group.

P5 is more preferably [1] L-proline whose side chain may be substituted, or its amino acid derivative wherein the C-terminal carboxyl group has been reduced to a hydroxymethyl group or formyl group, [2] 4-chlorophenylalanine whose side chain may be substituted, or its amino acid derivative wherein the C-terminal carboxyl group has been reduced to a hydroxymethyl group or formyl group, [3] 2-naphthylalanine whose side chain may be substituted, or its amino acid derivative wherein the C-terminal carboxyl group has been reduced to a hydroxymethyl group or formyl group, [4] cyclohexylalanine whose side chain may be substituted, or its amino acid derivative wherein the C-terminal carboxyl group has been reduced to a hydroxymethyl group or formyl group, [5] a hydroxyl group, or [6] a dipeptide chain formed by binding optionally substituted L-proline to (a) L-phenylalanine whose side chain may be substituted, (b) L-tyrosine whose side chain may be substituted, (c) L-2-thienylalanine whose side chain may be substituted, (d) L-phenylglycine whose side chain may be substituted or (e) L-2-pyridylalanine whose side chain may be substituted, or its peptide derivative wherein the C-terminal carboxyl group has been reduced to a hydroxymethyl group or formyl group.

P5 is still more preferably (1) L-proline or its amino acid derivative wherein the C-terminal carboxyl group has been reduced to a hydroxymethyl group or formyl group, (2) 4-chlorophenylalanine or its amino acid derivative wherein the C-terminal carboxyl group has been reduced to a hydroxymethyl group or formyl group, (3) 2-naphthylalanine or its amino acid derivative wherein the C-terminal carboxyl group has been reduced to a hydroxymethyl group or formyl group, (4) cyclohexylalanine or its amino acid derivative wherein the C-terminal carboxyl group has been reduced to a hydroxymethyl group or formyl group, (5) a hydroxyl group, (6) a dipeptide chain formed by binding L-proline to L-phenylalanine, or its peptide derivative wherein the C-terminal carboxyl group of the dipeptide has been reduced to a hydroxymethyl group or formyl group, (7) a dipeptide chain formed by binding L-proline to L-tyrosine, or its peptide derivative wherein the C-terminal carboxyl group has been reduced to a hydroxymethyl group or formyl group, (8) a dipeptide chain formed by binding L-proline to L-2-thienylalanine, or its peptide derivative wherein the C-terminal carboxyl group of the dipeptide has been reduced to a hydroxymethyl group or formyl group, (9) a dipeptide chain formed by binding L-proline to L-phenylglycine, or its peptide derivative wherein the C-terminal carboxyl group has been reduced to a hydroxymethyl group or formyl group, (10) a dipeptide chain formed by binding L-proline to 4-chlorophenylalanine, or its peptide derivative wherein the C-terminal carboxyl group has been reduced to a hydroxymethyl group or formyl group, (11) a dipeptide chain formed by binding L-proline to 2-naphthylalanine, or its peptide derivative wherein the C-terminal carboxyl group has been reduced to a hydroxymethyl group or formyl group, (12) a dipeptide chain formed by binding L-proline to 3-iodotyrosine, or its peptide derivative wherein the C-terminal carboxyl group has been reduced to a hydroxymethyl group or formyl group, (13) a dipeptide chain formed by binding L-proline to o-methyltyrosine, or its peptide derivative wherein the C-terminal carboxyl group has been reduced to a hydroxymethyl group or formyl group, or (14) a dipeptide chain formed by binding L-proline to L-2-pyridylalanine, or its peptide derivative wherein the C-terminal carboxyl group has been reduced to a hydroxymethyl group or formyl group.

Particularly preferable examples of "-P4-P5" include:

(1) -Nle-Pro-Phe,
(2) -Nle-Pro-Tyr,
(3) -Nle-Pro,
(4) -Nle,
(5) -Met-Pro-Phe,
(6) -Nle-Pro-Thi,
(7) -Nle-Pro-Phg,
(8) -Nle-Pro-Pya(2),
(9) -Met(O),
(10) -Met-Phe(Cl),
(11) -Met-Pro-Phe(Cl),
(12) -Met-Pro-Nal(2),
(13) -Met-Nal(2),
(14)-Met-Cha,
(15)-Cha-Pro-Phe,
(16) -Cha,
(17) -Met-Pro-Tyr(I),
(18) -Cha-Pro-Phe(Cl),
(19) -Cha-Phe(Cl),
(20) -Nle-Pro-Tyr(I),
(21) -Nle-Phe(Cl),
(22) -Cha-Pro-Tyr(I),
(23) -Cha-Tyr(I),
(24) -Cha-Phe,
(25) -Met-Phe,
(26) -Met-Pro-Tyr(Me),
(27) -OH,
(28) -Met,
(29) -Met-Pro-Phe,and

(30) -Ala-Pro-Phe(Cl) or the like.

Still more preferable examples of "-P4-P5" include:

(1) -Cha-Pro-Phe(Cl),
(2) -Cha-Pro-Phe,
(3) -Met-Pro-Phe(Cl),
(4) -Met-Pro-Phe
(5) -Cha-Phe, and
(6) -Met-Phe, more preferably
(1) -Cha-Pro-Phe(Cl),
(2) -Cha-Pro-Phe,
(3) -Met-Pro-Phe(Cl), and
(4) -Met-Pro-Phe.

The peptides of the present invention include, for example:

(1) Arg-Pro-Arg-Leu-Phe-Ala-Arg-Gly-Pro-Cha-Pro-Phe(Cl),
(2) Arg-Pro-Arg-Leu-Phe-His-Lys-Gly-Pro-Cha-Pro-Phe(Cl),
(3) Arg-Pro-Arg-Leu-Phe-His-Lys-Gly-Pro-Che-Pro-Phe,
(4) Arg-Pro-Arg-Leu-Phe-His-Lys-Gly-Pro-Met-Pro-Phe(Cl),
(5) Arg-Pro-Arg-Leu-Phe-Ala-Arg-Gly-Pro-Met-Pro-Phe,
(6) Arg-Pro-Arg-Leu-Phe-His-Lys-Gly-Pro-Cha-Phe(Cl),
(7) pGlu-Arg-Pro-Arg-Leu-Phe-Arg-Arg-Gly-Pro-Met-Pro-Phe,
(8) pGlu-Arg-Pro-Arg-Leu-Phe-His-Lys-Gly-Pro-Met-Pro-Phe,
(9) Arg-Pro-Arg-Leu-Phe-Ala-Arg-Gly-Pro-Met-Phe.

The peptide represented by the above formula
P1-Arg-Pro-Arg-Leu-Phe-P2-P3-Gly-Pro-P4-P5 (1), or esters thereof or salts thereof, include peptides represented by:

[1] Arg-Pro-Arg-Leu-Phe-Ala-Arg-Gly-Pro-Cha-Pro-Phe(Cl),
[2] Arg-Pro-Arg-Leu-Phe-His-Lys-Gly-Pro-Cha-Pro-Phe(Cl),
[3] Arg-Pro-Arg-Leu-Phe-His-Lys-Gly-Pro-Cha-Pro-Phe,
[4] Arg-Pro-Arg-Leu-Phe-His-Lys-Gly-Pro-Met-Pro-Phe(Cl),
[5] Arg-Pro-Arg-Leu-Phe-Ala-Arg-Gly-Pro-Met-Pro-Phe,
[6] Arg-Pro-Arg-Leu-Phe-His-Lys-Gly-Pro-Cha-Phe(Cl),
[7] pGlu-Arg-Pro-Arg-Leu-Phe-Arg-Arg-Gly-Pro-Met-Pro-Phe,
[8] pGlu-Arg-Pro-Arg-Leu-Phe-His-Lys-Gly-Pro-Met-Pro-Phe,
[9] Arg-Pro-Arg-Leu-Phe-Ala-Arg-Gly-Pro-Met- Phe

or esters thereof or salts thereof.

As the salts of the peptide of the present invention, the salts with physiologically acceptable bases (for example alkali metal) and acids (organic acid, inorganic acid) are used. Especially, physiologically acceptable acid addition salts are preferred. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The peptide of the present invention may be manufactured by obtaining the naturally occurring ligand by a method of purifying the peptide from tissues or cells of humans or warm-blooded animals and then modifying the ligand by methods for synthesizing peptides which will be described later. Alternatively, the peptide of the present invention can be produced by the methods for synthesizing proteins described later without using the naturally occurring ligand as the starting material.

Where the naturally occurring ligand is manufactured from tissues or cells of humans or warm-blooded animals, tissues or cells of humans or warm-blooded animals are homogenized, then extracted with an acid or the like, and the extract is isolated and purified by a combination of salting-out, dialysis and chromatographic techniques such as gel filtration, reverse phase chromatography, ion exchange chromatography, affinity chromatography and the like.

The naturally occurring ligand can be obtained according to a method described in e.g. WO 99/33976 (Japanese Patent Application No. 220853/1998).

The peptide of the present invention can be manufactured by publicly known methods for peptide synthesis. For the methods for peptide synthesis, for example, either solid phase synthesis or liquid phase synthesis may be used. That is, the partial peptide or amino acids that can construct the peptide of the present invention are condensed with the remaining part, and where the product contains protecting groups, these protecting groups can be eliminated to give the desired peptide. Publicly known methods for condensation and elimination of the protecting groups are described in [1]-[5] below.

[1] M. Bodanszky & M.A. Ondetti: Peptide Synthesis, Interscience Publishers, New York (1966)
[2] Schroeder & Luebke: The Peptide, Academic Press, New York (1965)
[3] Nobuo Izumiya, et al.: Peptide Gosei-no-Kiso to Jikken (Basics and experiments of peptide synthesis), published by Maruzen Co. (1975)
[4] Haruaki Yajima & Shunpei Sakakibara: Seikagaku Jikken Koza (Biochemical Experiment) 1, Tanpakushitsu no Kagaku (Chemistry of Proteins) IV, 205 (1977)
[5] Haruaki Yajima, ed.: Zoku Iyakuhin no Kaihatsu (A sequel to Development of Pharmaceuticals), Vol. 14, Peptide Synthesis, published by Hirokawa Shoten

After completion of the reaction, the peptide of the present invention may be purified and isolated by a combination of conventional purification methods such as solvent extraction, distillation, column chromatography, liquid chromatography and recrystallization. When the peptide obtained by the above methods is in a free form, the peptide can be converted into an appropriate salt by a publicly known method; when the protein is obtained in a salt form, it can be converted into a free form by a publicly known method.
For condensation of the protected amino acids or the peptide, a variety of activation reagents for protein synthesis may be used, and trisphosphonium, tetramethyluronium and carbodiimides are particularly preferable. The trisphosphonium includes benzotriazol-1-yloxytrispirolidinophosphonium hexafluorophosphate (PyBOP), bromotrispirolidinophosphonium hexafluorophosphate (PyBroP) etc., the tetramethyluronium includes 2-(1H-benzotriazol-1-yl)-1,1,3,3,-tetramethyluronium tetrafluoroborate, 2-(5-norbornane-2,3-dicarboxyimide)-1,1,3,3-tetramethyluronium tetrafluoroborate, O-(N-succinudyl)-1,1,3,3-tetramethyluronium tetrafluroborate, and the carbodiimides include DCC, N, N'-diisopropylcarbodiimide, N-ethyl-N'-(3-dimethylaminopropyl) carbodiimide. For condensation by these reagents, a racemization inhibitor (e.g., HONB, HOBt, HOOBt) is preferably added. Solvents used in condensation may be chosen from solvents known to be usable for peptide condensation reactions. Examples of such solvents are acid amides such as water-free or water-containing N,N-dimethylformamide, N,N-dimethylacetamide, N-methylpyrrolidone; halogenated hydrocarbons such as methylene chloride, chloroform; alcohols such as trifluoroethanol; sulfoxides such as dimethylsulfoxide; tertiary amines such as pyridine; ethers such as dioxane, tetrahydrofuran, nitriles such as acetonitrile, propionitrile; esters such as methyl acetate, ethyl acetate; and appropriate mixtures of these solvents. The reaction temperature is appropriately chosen from the range known to be applicable to peptide binding reactions and is usually selected in the range of approximately -20 °C to 50 °C. The activated amino acid derivatives are used generally in an excess of 1.5 to 4 times. In the case of solid phase synthesis, the condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole, whereby the subsequent reactions can not be adversely affected.
Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc. Protective groups used for a carboxyl group include, for example, a $C_{1-6}$ alkyl, $C_{3-8}$ cycloalkyl group and $C_{7-14}$ aralkyl group which are described above as R, allyl, 2-adamantyl, 4-nitrobenzyl, 4-methoxybenzyl, 4-chlorobenzyl, phenacyl group and benzyloxycarbonyl hydrazide, t-butoxycarbonyl hydrazide, trityl hydrazide.
The hydroxyl group of serine and threonine can be protected through, for example, its esterification or etherification. Examples of groups appropriately used for the esterification include groups derived from organic acids, for example, a lower ($C_{2-4}$) alkanoyl group such as acetyl group and an aroyl group such as benzoyl group. Examples of groups appropriately used for the etherification include a benzyl group, tetrahydropyranyl group, t-butyl group, trityl (Trt).
Examples of groups for protecting the phenolic hydroxyl group of tyrosine include Bzl, 2,6-dichlorobenzyl, 2-nitrobenzyl, Br-Z, t-butyl.
Examples of groups used to protect the imidazole moiety of histidine include Tos, 4-methoxy-2,3,6-trimethylbenzenesulfonyl (Mtr), DNP, Bom, Bum, Boc, Trt, Fmoc.
Examples of groups for protecting the guanidino group of arginine include Tos, Z, 4-methoxy-2,3,6-trimethylbenzenesulfonyl (Mtr), p-methoxybenzenesulfonyl (MBS), 2,2,5,7,8-pentamethyl chromane-6-sulfonyl (Pmc), mesitylene-2-sulfonyl (Mts), 2,2,4,6,7-pentamethyldihydrobenzofurane-5-sulfonyl (Pbf), Boc, Z, $NO_2$.

Examples of groups used to protect the side-chain amino acid of lysine include Z, Cl-Z, trifluoroacetyl, Boc, Fmoc, Trt, Mtr, 4,4-dimethyl-2,6-dioxocyclohexylideneale (Dde).

Examples of groups used to protect the indolyl moiety of tryptophan include formyl (For), Z, Boc, Mts, Mtr.

Examples of groups used to protect asparagine and glutamine include Trt, xanthyl (Xan), 4,4'-dimethoxybenzhydryl (Mbh), 2,4,6-trimethoxybenzyl (Tmob).

Examples of the activated carboxyl groups in the starting compounds include the corresponding acid anhydrides, azides, activated esters [esters with alcohols, e.g., pentachlorophenol, 2,4,5-trichlorophenol, 2,4-dinitrophenol, cyanomethyl alcohol, p-nitrophenol, HONB, N-hydroxysuccimide, 1-hydroxybenzotriazole, (HOBt))]. As the activated amino acids, in which the amino groups are activated in the starting material, the corresponding phosphorous amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid, trifluoroacetic acid, trimethylsilane bromide (TMSBr), trimethylsilyltrifluoromethane sulfonate, tetrafluoroboric acid, tris(trifluoro)boron, boron tribromide or a mixed solution thereof; a treatment with a base such as diisopropylethylamine, triethylamine, piperidine or piperazine; and reduction with sodium in liquid ammonia. The elimination reaction by the acid treatment described above is carried out generally at a temperature of -20°C to 40°C, and in the acid treatment, it is efficient to add a cation scavenger such as anisole, phenol, thioanisole, m-cresol or p-cresol, as well as dimethylsulfide, 1,4-butanedithiol or 1,2-ethanedithiol. Furthermore, a 2,4-dinitrophenyl group used as a protecting group for the imidazole of histidine is removed by a treatment with thiophenol, while a formyl group used as a protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction can be appropriately selected from publicly known groups and publicly known means.

In a method for obtaining the amides of the peptide, for example, solid phase synthesis is carried out using a resin for synthesis of amides, or the α-carboxyl group of the carboxy terminal amino acid is amidated, then the peptide chain is extended from the amino group side to a desired length, a peptide in which only the protecting group of the N-terminal α-amino group in the peptide chain has been eliminated from the peptide and a peptide (or amino acids) in which only the protecting group of the C-terminal carboxyl group has been eliminated are prepared, and the two peptides are condensed in a mixture of the solvents described above. The details of the condensation reaction are the same as described above. After the protected peptide obtained by the condensation is purified, all the protecting groups are eliminated by the method described above to give the desired crude peptide. This crude peptide is purified by various known purification means, and lyophilization of the major fraction gives the amide of the desired peptide.

To prepare the esterified peptide, for example, the α-carboxyl group of the carboxy terminal amino acid is condensed with a desired alcohol to prepare the amino acid ester, which is followed by procedure similar to the preparation of the amidated peptide above to give the ester form of the desired peptide.

The peptide of the present invention may also be a fusion protein with a protein whose functions or properties are well known.

The peptide of the present invention can be used in [1] searching for the physiological action of the peptide of the present invention, [2] development of a receptor binding assay system using the expression system of the recombinant receptor protein and screening of candidates for pharmaceutical compounds, and [3] development of pharmaceutical preparations such as a neutral nerve function regulator, a circulatory function regulator, a cardiac function regulator, an immune function regulator, a digestive organ function regulator, a metabolic function regulator or a generative organ regulator.

In particular, by the use of the receptor binding assay system using the expression system of the recombinant G protein-coupled receptor protein which will be described later, G protein-coupled receptor protein agonists or antagonists specific to warm-blooded animals such as humans can be screened, and the agonists or antagonists can be used as prophylactic and/or therapeutic agents for various diseases.

With respect to [3] above, the peptide of the present invention is recognized as a ligand by the G protein-coupled receptor protein (e.g., APJ) expressed in the central nervous system, circulatory organ system, heart, immune system, digestive organ system, metabolic system or generative organ system and is thus useful as a safe and low-toxic pharmaceutical agent. The peptide of the present invention is involved in a neutral nerve function-regulating action, a circulatory function-regulating action, a cardiac function-regulating action, an immune function-regulating action, a digestive organ function-regulating action, a metabolic function-regulating action or a generative organ function-regulating action, so the peptide can be used as a prophylactic and/or therapeutic agent for diseases such as senile dementia, cerebrovascular dementia, dementia attributable to degenerative diseases of system degenerative type (for example, Alzheimer's disease, Parkinson's disease, Pick' disease, Huntington's disease), dementia attributable to infectious diseases (for example, delayed viral infections such as Creutzfeldt-Jakob disease), dementia attributable to endocrine, metabolic and poisoning diseases (for example, hypothyroidism, vitamin B12 deficiency, alcoholism, poisoning due to various chemicals, metallic or organic compounds), dementia attributable to tumor diseases (for example, brain tumor), dementia attributable to traumatic diseases (for example, chronic hematocele beneath the dura mater), melancholia, attention deficit hyperactivity (minimal brain disease) syndrome, mental confusion, anxiety, schizophrenia, psychasthenia, an obstacle in growth hormone secretion (for example, gigantism, acromegaly), bulimia, overeating, hypercholesterolemia, hyperglyceridemia, hyperlipemia, hyperprolactinemia, diabetes (for example, diabetic complications, diabetic renal trouble, diabetic nervous disturbance, diabetic retinopathy), cancers (for example, breast cancer, lymphotic leukocyte, lung cancer, bladder cancer, ovary cancer, prostate cancer), pancreatitis, renal diseases (for example, chronic renal trouble, nephritis), Turner's syndrome, neurosis, rheumatic arthritis, spinal damage, transitory cerebral ischemia paroxysm, amyotrophic lateral sclerosis, acute myocardial infarction, spinal cerebellum degeneration, bone fracture, wounds, atopic dermatitis, osteoporosis, asthma, epilepsy, sterility, arteriosclerosis, pulmonary emphysema, lung edema, or imperfect lactation. Further, the peptide can be used as a hypnotic sedative, an improver for nutritional conditions after operation, a hypertensive agent or the like.

In addition, the peptide can be used as a prophylactic and/or therapeutic agent for HIV infections and AIDS (acquired immune deficiency syndrome).

When the peptide of the present invention is used as the above-described pharmaceutical preparation, the peptide can be prepared into a pharmaceutical composition in a conventional manner. For example, the peptide can be used orally, for example, in the form of tablets which may be coated with sugar or an enteric coating if necessary and desired, capsules, elixirs, microcapsules, or parenterally in the form of injections such as a sterile solution and a suspension in water or with other pharmaceutically acceptable liquid. For example, these preparations can be manufactured by mixing the compound or a salt thereof with a physiologically acceptable carrier, a flavoring agent, an excipient, a vehicle, an antiseptic agent, a stabilizer, a binder, in a unit dosage form required in a generally accepted manner that is applied to making pharmaceutical preparations. The effective component in the preparation is controlled in such a dose that an appropriate dose is obtained within the specified range given.

Additives miscible with tablets, capsules, include a binder such as gelatin, corn starch, tragacanth gum, and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, to prepare the pharmaceutical composition.

Examples of an aqueous medium for injection include physiological saline and an isotonic solution containing glucose and other auxiliary agents (e.g., D-sorbitol, D-mannitol, sodium chloride) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50). Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer), a soothing agent (e.g., benzalkonium chloride, procaine hydrochloride), a stabilizer (e.g., human serum albumin, polyethylene glycol), a preservative (e.g., benzyl alcohol, phenol), an antioxidant. The thus prepared liquid for injection is normally filled in an appropriate ampoule. Since the thus obtained pharmaceutical preparation is safe and low toxic, the preparation can be administered to mammalians (e.g., humans, mice, rats, guinea pigs, rabbits, sheep, swine, bovine, cats, dogs, monkeys, chimpanzees).

The dose of the peptide of the present invention varies depending on conditions; in oral administration, e.g., for the patient with HIV infections, the dose is normally about 0.1 mg to about 100 mg, preferably about 1.0 to about 50 mg, and more preferably about 1.0 to about 20 mg per day (as 60 kg body weight). In parenteral administration, the single dose varies depending on subject to be administered, target organ, conditions, routes for administration, but it is advantageous, e.g., for the patient with HIV infections, to administer the active ingredient intravenously in a daily dose of about 0.01 to about 30 mg, preferably about 0.1 to about 20 mg, and more preferably about 0.1 to about 10 mg (as 60 kg body weight). For other animal species, the corresponding dose as converted per 60 kg body weight can be administered.

The G protein-coupled receptor protein to the peptide of the present invention described above is a G protein-coupled receptor protein which is derived from any cells or tissues (e.g., thymus, spleen, brain, kidney, lever, gonad, thyroid, gall-bladder, bone marrow, adrenal gland, skin, muscle, lung, digestive tract, blood vessel, heart) from human and warm-blooded animals (for example, warm-blooded mammalians (e.g., rabbits, sheep, goats, rats, mice, guinea pigs, bovine, horses, swine), birds (e.g., chickens, pigeons, ducks, geese, quails), and which has an amino acid sequence that has the same or substantially the same amino acid sequence as that represented by SEQ ID NO:19. That is, the G protein-coupled receptor protein includes not only a protein containing the amino acid sequence represented by SEQ ID NO:19 in this specification, but also a protein containing an amino acid sequence having at least about 90 to 99.9 % homology to the amino acid sequence represented by SEQ ID NO:19 in this specification and having the activity substantially equivalent to that of the protein containing the amino acid sequence represented by SEQ ID NO: 19 in this specification.

The substantially equivalent activity of these proteins includes, for example, a ligand binding activity, a signal transduction activity. The term "substantially equivalent" is used to mean that the nature of the ligand binding activity is the same. Therefore, quantitative factors such as level of the ligand binding activity, the molecular weight of the receptor protein may differ.

The G protein-coupled receptor protein includes those wherein the N-terminal Met is protected with a protecting group (for example, a $C_{1-6}$ acyl group such as a $C_{2-6}$ alkanoyl group, e.g., formyl, acetyl), those wherein the N-terminal region of Gln is cleaved in vivo and the Gln is pyroglutaminated, and those wherein a side chain of the amino acid residue in the molecule is protected with a suitable protecting group (e.g., a $C_{1-6}$ acyl group such as formyl group, acetyl group), or conjugated proteins such as glycoproteins bound to sugar chains.

The salt of the G protein-coupled receptor protein includes the same salt as that of the peptide described above.

The G protein-coupled receptor protein, salts thereof or partial peptides thereof may be manufactured by a publicly known method used to purify a protein from human or other mammalian cells or tissues, or by the above-described method for synthesizing peptides.

As the partial peptides of the G protein-coupled receptor protein, for example those domains in the G protein-coupled receptor protein molecule which are exposed to the outside of a cell membrane are used. That is, the partial peptide is a peptide containing the parts analyzed to be extracellular domains (hydrophilic domains) in the hydrophobic plotting analysis of the G protein-coupled receptor protein. Further, a peptide containing a hydrophobic domain in part can be used as well. In addition, the peptide may contain each domain separately or plural domains together.

As a salt of the partial peptide of the G protein-coupled receptor protein, the same salt as that of the peptide described above is used.

The DNA encoding the G protein-coupled receptor protein may be any DNA containing a nucleotide sequence encoding the G protein-coupled receptor protein containing the same or substantially the same amino acid sequence as that represented by SEQ ID NO:19 in this specification. Further, the DNA may be genomic DNA, genomic DNA library, cDNA derived from the cells and tissues described above, cDNA library derived from the cells and tissues described above and synthetic DNA. The vector to be used for the library may be bacteriophage, plasmid, cosmid and phagemid. The DNA may also be directly amplified by RT-PCR using the RNA or mRNA fraction prepared from the cells and tissues described above.

Specifically, as the DNA encoding the G protein-coupled receptor protein containing the amino acid sequence represented by SEQ ID NO:19 in this specification, e.g. DNA having the nucleotide sequence shown by SEQ ID NO:20 in this specification is used.

Hereinafter, the peptide of the present invention or salts thereof (hereinafter sometimes referred to as the peptide of the present invention) are specifically described for the use or applications.

(1) A prophylactic/therapeutic agent for a deficiency of the ligand peptide

Depending on the action of the peptide of the present invention on the G protein-coupled receptor protein (APJ), the peptide of the present invention can also be used as a prophylactic/therapeutic agent for a deficiency of the ligand peptide or G protein-coupled receptor protein.

For example, when there is a patient in the case where because of a reduction in the G protein-coupled receptor protein (APJ) in the body, the physiological actions of the ligand (a neutral nerve function-regulating action, a circulatory function-regulating action, a cardiac function-regulating action, an immune function-regulating action, a digestive organ function-regulating action, a metabolic function-regulating action or a generative organ function-regulating action) cannot be expected, the amount of the ligand peptide in the body of the patient can be increased and the actions of the ligand peptide can be sufficiently exhibited by administering the peptide of the present invention into the patient. Accordingly, the peptide of the present invention can be used as a safe and low-toxic prophylactic/therapeutic agent for a deficiency of the ligand peptide.

(2) Method of quantifying the G protein-coupled receptor protein (APJ) to the ligand peptide

Since the peptide of the present invention have a property of binding to the G protein-coupled receptor protein (APJ) or a salt thereof or a partial peptide of the receptor protein or a salt thereof, the concentration of the G protein-coupled receptor protein (APJ) or a salt thereof, or a partial peptide of the receptor protein, an amide thereof, an ester thereof or a salt thereof in the body can be quantified with good sensitivity.

As the partial peptide of the G protein-coupled receptor protein (APJ), for example, those domains in the G protein-coupled receptor protein (APJ) molecule which are exposed to the outside of a cell membrane are used. That is, the partial peptide is a peptide containing the parts analyzed to be extracellular domains (hydrophilic domains) in the hydrophobic plotting analysis of the G protein-coupled receptor protein (APJ). Further, a peptide containing a hydrophobic domain in part can be used as well. In addition, the peptide may contain each domain separately or plural domains together.

The amide and ester of the partial peptide of the G protein-coupled receptor protein (APJ) can be obtained in the same manner as for the amide or ester of the peptide of the present invention described above. As the salt of the partial peptide of the G protein-coupled receptor protein (APJ), the same salt as that of the peptide of the present invention described above can be used.

The quantification methods of the present invention can be used in combination with, for example, a competitive method.

That is, the concentration of the G protein-coupled receptor protein (APJ) or a salt thereof, or a partial peptide of the G protein-coupled receptor protein (APJ), an amide thereof, an ester thereof or a salt thereof in a test sample can be measured by contacting the test sample to the peptide of the present invention. Specifically, the methods can be used by following, for example, the methods described in (1) and (2) below or its modified methods.

[1] Hiroshi Irie, ed. "Radioimmunoassay," Kodansha, published in 1974
[2] Hiroshi Irie, ed. "Sequel to the Radioimmunoassay," Kodansha, published in 1979.

(3) Methods of screening compounds that alter the binding property between the G protein-coupled receptor protein (APJ) and the peptide of the present invention.

Using the G protein-coupled receptor protein (APJ), a salt thereof, a partial peptide thereof, or an amide, ester or salt of the partial peptide, or using the receptor binding assay system of the expression system constructed using the recombinant receptor protein (APJ), compounds (e.g., peptides, proteins, non-peptide compounds, synthetic compounds, fermentation products) or salt forms thereof that alter the binding property between the peptide etc, of the present invention and the G protein-coupled receptor protein (APJ) can be efficiently screened. Such compounds include compounds that have the G protein-coupled receptor (APJ)-mediated cell stimulating activities (e.g., activities that promote or suppress arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction) (so-called agonists to the G protein-coupled receptor) and compounds that do not have the cell stimulating activity (so-called antagonists to the G protein-coupled receptor). The terms "alter the binding property" encompass both inhibition of binding to the peptide of the present invention and promotion of binding to the peptide of the present invention.

That is, the present invention provides methods of screening compounds or their salt forms that alter the binding property between the peptide of the present invention and the G protein-coupled receptor protein (APJ), characterized by comparing (i) the case wherein the peptide of the present invention are brought into contact with the G protein-coupled receptor protein (APJ), a salt thereof, a partial peptide thereof, or a salt of the partial peptide, with (ii) the case wherein the peptide of the present invention and a test compound are brought into contact with the G protein-coupled receptor protein (APJ), a salt thereof, a partial peptide thereof, or an amide, ester or salt of the partial peptide.

The screening methods of the present invention are characterized by assaying, for example, the amount of the peptide of the present invention bound to the G protein-coupled receptor protein (APJ) or a partial peptide of the receptor protein, the cell stimulating activity, and comparing the property between (i) the case wherein the peptide of the present invention are brought into contact with the G protein-coupled receptor protein (APJ) or a partial peptide of the receptor protein and (ii) the case wherein the peptide of the present invention and a test compound are brought into contact with the G protein-coupled receptor protein (APJ) or a partial peptide of the receptor protein.

More specifically, the present invention provides the following screening methods:

[1] a method of screening a compound or its salt that alters the binding property between the peptide of the present invention and the G protein-coupled receptor protein (APJ), which comprises:

measuring and comparing the amount of the labeled peptide of the present invention bound to the G protein-coupled receptor protein (APJ) or a salt thereof, or a partial peptide thereof, or an amide, ester or salt of the partial peptide between the case wherein the labeled peptide of the present invention are brought into contact with the G protein-coupled receptor protein (APJ), a salt thereof, a partial peptide of the G protein-coupled receptor protein (APJ), or an amide, ester or salt of the partial peptide and the case wherein the labeled peptide of the present invention and a test compound are brought into contact with the G protein-coupled receptor protein (APJ), a salt thereof, a partial peptide of the G protein-coupled receptor protein (APJ), or an amide, ester or salt of the partial peptide;

[2] a method of screening a compound or its salt that alters the binding property between the peptide of the present invention and the G protein-coupled receptor protein (APJ), which comprises:

measuring and comparing the amount of the labeled peptide of the present invention bound to cells or the membrane fraction of the cells between the case where the labeled peptide of the present invention are brought into contact with the cells or cell membrane fraction containing the G protein-coupled receptor protein (APJ) and the case where the labeled peptide of the present invention and a test compound are brought into contact with the cells or cell membrane fraction containing the G protein-coupled receptor protein (APJ);

[3] a method of screening a compound or its salt that alters the binding property between the peptide of the present invention and the G protein-coupled receptor protein (APJ), which comprises:

measuring and comparing the amount of the labeled peptide of the present invention bound to the G protein-coupled receptor protein (APJ) between the case where the labeled peptide of the present invention is brought into contact with the G protein-coupled receptor protein (APJ) expressed on the cell membrane induced by culturing a transformant containing the DNA encoding the G protein-coupled receptor protein (APJ) and the case where the labeled peptide etc. of the present invention and a test compound are brought into contact with the G protein-coupled receptor protein (APJ) expressed on the cell membrane induced by culturing a transformant containing the DNA encoding the G protein-coupled receptor protein (APJ);

[4] a method of screening a compound or its salt that alters the binding property between the peptide of the present invention and the G protein-coupled receptor protein (APJ), which comprises:

measuring and comparing the receptor-mediated cell stimulating activity (e.g., the activity that promotes or suppresses arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction between the case where a compound (e.g., the peptide of the present invention) that activates the G protein-coupled receptor protein (APJ) is brought into contact with cells containing the G protein-coupled receptor protein (APJ) and the case where the compound that activates the G protein-coupled receptor protein (APJ) and a test compound are brought into contact with cells containing the G protein-coupled receptor protein (APJ); and

[5] a method of screening a compound or its salt that alters the binding property between the peptide of the present invention and the G protein-coupled receptor protein (APJ), which comprises:

measuring and comparing the receptor-mediated cell stimulating activity (e.g., the activity that promotes or suppresses arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction) between the case where a compound (e.g., the peptide of the present invention) that activates the G protein-coupled receptor protein (APJ) is brought into contact with the G protein-coupled receptor protein (APJ) expressed on the cell membrane induced by culturing a transformant containing the DNA encoding the G protein-coupled receptor protein (APJ) and the case where the compound that activates the G protein-coupled receptor protein (APJ) and a test compound are brought into contact with the G protein-coupled receptor protein (APJ) expressed on the cell membrane induced by culturing a transformant containing the DNA encoding the G protein-coupled receptor protein (APJ).

Hereinafter, the screening methods of the present invention are described more specifically.

First, for the G protein-coupled receptor protein (APJ) used for the screening methods of the present invention, any substance may be used so long as it contains the G protein-coupled receptor protein described above or a partial peptide of the G protein-coupled receptor protein, and the cell membrane fraction from human or mammalian organs is preferred. However, it is preferable to use the G protein-coupled receptor protein (APJ) produced in a large amount using a recombinant for the screening because human-derived organs are hardly obtainable.

In the screening methods of the present invention, when cells containing the G protein-coupled receptor protein or the membrane fraction of the cells is used, the preparation method described later may be followed.

Where cells containing the G protein-coupled receptor protein are used, the cells may be fixed using glutaraldehyde, formalin. The fixation can be made by a publicly known method.

The cells containing the G protein-coupled receptor protein are host cells that express the G protein-coupled receptor protein, and for the host cells, Escherichia coli, Bacillus subtilis, yeast, insects, insect cells, animal cells and the like are used.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)).

Examples of the bacteria belonging to the genus Bacillus include Bacillus subtilis MI114 (Gene, 24, 255 (1983)), 207-21 (Journal of Biochemistry, 95, 87 (1984)).

Examples of yeast include Saccharomyces cereviseae AH22, AH22R$^-$, NA87-11A, DKD-5D, 20B-12.

As the insect, for example, a larva of Bombyx mori can be used (Maeda, et al., Nature, 315, 592 (1985)).

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea; and for the virus BmNPV, Bombyx mori N cells (BmN cells), are used. Examples of the Sf cell which can be used are Sf9 cells (ATCC CRL1711) and Sf21 cells (both cells are described in Vaughn, J. L. et al., In Vivo, 13, 213-217 (1977).

Examples of animal cells include monkey cells COS-7, Vero cells, Chinese hamster cells CHO, DHFR gene deficient Chinese hamster cells CHO (CHO (dhfr$^-$) cell), mouse L cells, mouse 3T3 cells, mouse myeloma cells, human HEK293 cells, human FL cells, 293 cells, C127 cells, BALB3T3 cells, SP-2/O cells.

The cell membrane fraction refers to a fraction abundant in cell membrane obtained by cell disruption and subsequent fractionation by a publicly known method. Cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the G protein-coupled receptor protein expressed and membrane components such as cell-derived phospholipids and membrane proteins.

The amount of the G protein-coupled receptor protein in the cells containing the G protein-coupled receptor protein and in the membrane fraction is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantities of samples can be assayed with the same lot.

To perform the methods [1] to [3] supra for screening compounds that alter the binding property between the peptide etc. of the present invention and the G protein-coupled receptor, an appropriate G protein-coupled receptor fraction and the labeled peptide of the present invention are required. The G protein-coupled receptor fraction is preferably a fraction of naturally occurring G protein-coupled receptor or a recombinant G protein-coupled receptor fraction having an activity equivalent to that of the natural protein. Herein, the term "equivalent activity" is intended to mean a ligand binding activity or the like that is equivalent to that possessed by the naturally occurring receptor protein. For the labeled ligand, a labeled ligand and a labeled ligand analogue are used. For example, ligands labeled with [$^3$H], [$^{125}$I], [$^{14}$C], [$^{35}$S] are used.

Specifically, to screen the compounds that alter the binding property between the peptide of the present invention and the G protein-coupled receptor protein, first, the receptor protein standard is prepared by suspending cells or cell membrane fraction containing the G protein-coupled receptor protein in a buffer appropriate for the screening. For the buffer, any buffer that does not interfere with the binding of the ligand to the receptor is usable and examples of such a buffer are phosphate buffer, Tris-hydrochloride buffer, having pH of 4 to 10 (preferably pH of 6 to 8). To minimize a non-specific binding, a surfactant such as CHAPS, Tween-80™ (Kao-Atlas Co.), digitonin, deoxycholate may be added to the buffer. To inhibit degradation of the receptor and the peptide of the present invention by proteases, protease inhibitors such as PMSF, leupeptin, E-64 (manufactured by Peptide Research Laboratory, Co.), and pepstatin may be added. To 0.01 to 10 ml of the receptor solution, a given amount (5,000 to 500,000 cpm) of the labeled peptide of the present invention is added, and $10^{-4}$ to $10^{-10}$ $\mu$M test compound is allowed to be coexistent therewith. To examine non-specific binding (NSB), a reaction tube containing an unlabeled test compound in a large excess is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, and washed with an appropriate volume of the same buffer, and the residual radioactivity on the glass fiber filter paper is then measured by means of a liquid scintillation counter or $\gamma$-counter. Regarding the count ($B_0$ - NSB) obtained by subtracting the amount of non-specific binding (NSB) from the count obtained in the absence of any competitive substance ($B_0$) as 100%, when the amount of specific binding (B - NSB) is, for example, 50% or less, the test compound can be selected as a candidate substance having a potential of competitive inhibition.

To carry out the method of screening compounds that alter the binding property between the peptide of the present invention and the G protein-coupled receptor protein (APJ), the G protein-coupled receptor protein-mediated cell stimulating activities (e.g., the activities that promote or suppress arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction) may be determined by a publicly known method, or using an assay kit commercially available. Specifically, cells containing the G protein-coupled receptor protein are first cultured on a multi-well plate. Prior to screening, the medium is replaced with fresh medium or with an appropriate non-cytotoxic buffer, followed by incubation for a given period of time in the presence of a test compound, then the cells are extracted or the supernatant is recovered, and the resulting product is quantified by appropriate procedures. Where it is difficult to detect the production of the index substance (e.g., arachidonic acid) for the cell stimulating activity due to a degrading enzyme contained in the cells, an inhibitor against such a degrading enzyme may be added prior to the assay. For detecting activities such as the cAMP production suppression activity, the baseline production in the cells is increased by forskolin or the like and the suppressing effect on the increased baseline production may then be detected.

Screening by assaying the cell stimulating activity requires suitable cells that have expressed the G protein-coupled receptor protein. For the cells that have expressed the G protein-coupled receptor protein in the present invention, the cell line expressing the recombinant G protein-coupled receptor protein (APJ) described above and the like are desirable.

For the test compound, for example, peptides, proteins, non-peptide compounds, synthetic compounds, fermentation products, cell extracts, plant extracts, and animal tissue extracts are used, and these compounds may be novel or known compounds.

The kits for screening the compounds or their salts that alter the binding property between the peptide the present invention and the G protein-coupled receptor protein (APJ) comprise the G protein-coupled receptor protein or a salt thereof, a partial peptide of the G protein-coupled receptor protein or an amide, ester or salt thereof, cells containing the G protein-coupled receptor protein or the membrane fraction of cells containing the G protein-coupled receptor protein, and the peptide of the present invention.

Examples of the screening kits of the present invention are as follow.

1. Reagents for screening

[1] Buffer for measurement and washing

Hanks' balanced salt solution (Gibco Co.) supplemented with 0.05 % bovine serum albumin (Sigma Co.).

The solution is sterilized by filtration through a 0.45 μm filter and stored at 4°C or may be prepared just before use.

[2] Standard G protein-coupled receptor (APJ)

CHO cells expressing the G protein-coupled receptor protein (APJ) are passaged in a 12-well plate at a density of $5 \times 10^5$ cells/well followed by culturing at 37°C under 5% $CO_2$ and 95% air for 2 days.

[3] Labeled peptide of the present invention

The peptide of the present invention labeled with [$^3$H], [$^{125}$I], [$^{14}$C], [$^{35}$S], which were dissolved in a suitable solvent or buffer, are stored at 4°C or -20°C, and diluted to 1 μM with the measurement buffer just before use.

[4] Standard solution of the peptide etc. of the present invention

The peptide of the present invention is dissolved in and adjusted to 1 mM with PBS containing 0.1 % bovine serum albumin (manufactured by Sigma Co.) and stored at -20°C.

2. Measurement method

[1] CHO cells expressing the G protein-coupled receptor protein (APJ) are cultured in a 12-well culture plate and washed twice with 1 ml of the measurement buffer, and 490 μl of the measurement buffer is added to each well.
[2] After adding 5 μl of $10^{-3}$ to $10^{-10}$ M test compound solution, 5 μl of the labeled peptide of the present invention is added to the mixture, and the cells are incubated at room temperature for 1 hour. To determine the amount of the non-specific binding, 5 μl of $10^{-3}$ M ligand is added in place of the test compound.

[3] The reaction solution is removed, and the wells are washed 3 times with the washing buffer. The labeled peptide of the present invention bound to the cells is dissolved in 0.2 N NaOH-1% SDS, and mixed with 4 ml of liquid scintillator A (Wako Pure Chemical Industries, Ltd.)

[4] The radioactivity is measured using a liquid scintillation counter (Beckman Co.), and the percent maximum binding (PMB) is calculated by the equation below.

$$PMB = [(B - NSB)/(B_0 - NSB)] \times 100$$

PMB: Percent maximum binding
B: Value obtained in the presence of a test compound
NSB: Non-specific binding
Bo: Maximum binding

The compounds or salts thereof, which are obtainable using the screening methods or the screening kits of the present invention, are the compounds that alter (suppress or promote) the binding property between the peptide of the present invention and the G protein-coupled receptor (APJ), and specifically these compounds are compounds that have the G protein-coupled receptor-mediated cell stimulating activity (so-called agonists to the G protein-coupled receptor) or compounds having no cell stimulating activity (so-called antagonists to the G protein-coupled receptor). The compounds may be peptides, proteins, non-peptide compounds, synthetic compounds, fermentation products, and may be novel or known compounds.

The method of specifically evaluating whether the resultant compound is an agonist or antagonist to the G protein-coupled receptor may follow the following (i) or (ii):

(i) A compound that alters (particularly suppresses) the binding property between the peptide of the present invention and the G protein-coupled receptor (APJ) is obtained by the binding assay shown in the screening methods in [1] to [3] above, followed by determining whether the compound has the G protein coupled receptor (APJ)-mediated cell stimulating activities described above. A compound or a salt thereof having the cell stimulating activities is an agonist to the G protein-coupled receptor, while a compound not having the cell stimulating activities is an antagonist to the G protein-coupled receptor.

(ii)

(a) A test compound is brought into contact with cells containing the G protein-coupled receptor protein (APJ) and measured for the G protein coupled receptor (APJ)-mediated cell stimulating activities described above. A compound or a salt thereof having the cell stimulating activities is an agonist to the G protein-coupled receptor.

(b) The G protein coupled receptor (APJ)-mediated cell stimulating activities are measured and compared between the case where a compound (for example, the peptide of the present invention or an agonist to the G protein-coupled receptor) activating the G protein-coupled receptor is brought into contact with cells containing the G protein-coupled receptor protein (APJ) and the case where the compound activating the G protein-coupled receptor and a test compound are brought into contact with cells containing the G protein-coupled receptor protein (APJ). A compound or a salt thereof that can reduce the cell stimulating activities due to the compound activating the G protein-coupled receptor (APJ) is an antagonist to the G protein-coupled receptor.

Since agonists to the G protein-coupled receptor have the same physiological activities as those of the peptide of the present invention for the G protein-coupled receptor (APJ), the agonists similar to the peptide etc. of the present invention are useful as safe and low-toxic pharmaceuticals.

Since antagonists to the G protein-coupled receptor can suppress the physiological activities of the peptide of the present invention for the G protein-coupled receptor (APJ), the antagonists are useful as safe and low-toxic pharmaceuticals that inhibit the receptor activities.

The peptide of the present invention are involved in a neutral nerve function-regulating action, a circulatory function-regulating action, a cardiac function-regulating action, an immune function-regulating action, a digestive organ function-regulating action, a metabolic function-regulating action or a generative organ function-regulating action, and thus the agonist or antagonist described above can be used as a prophylactic and therapeutic agent for diseases such as senile dementia, cerebrovascular dementia, dementia attributable to degenerative diseases of system degenerative type (for example, Alzheimer's disease, Parkinson's disease, Pick' disease, Huntington's disease), dementia attributable to infectious diseases (for example, delayed viral infections such as Creutzfeldt-Jakob disease), dementia attributable to endocrine, metabolic and poisoning diseases (for example, hypothyroidism, vitamin B12 deficiency, alcoholism, poisoning due to various chemicals, metallic or organic compounds), dementia attributable to tumor diseases (for example, brain tumor), dementia attributable to traumatic diseases (for example, chronic hematocele beneath the dura mater), melancholia, attention deficit hyperactivity (minimal brain disease) syndrome, mental confusion, anxiety, schizophrenia, psychasthenia, an obstacle in growth hormone secretion (for example, gigantism, acromegaly), bulimia, overeating, hypercholesterolemia, hyperglyceridemia, hyperlipemia, hyperprolactinemia, hypoglycemia, hypopituitarism, pituitary dwarfism, diabetes (for example, diabetic complications, diabetic renal trouble, diabetic nervous disturbance, diabetic retinopathy), cancers (for example, breast cancer, lymphotic leukocyte, lung cancer, bladder cancer, ovary cancer, prostate cancer), pancreatitis, renal diseases (for example, chronic renal trouble, nephritis), Turner's syndrome, neurosis, rheumatic arthritis, spinal damage, transitory cerebral ischemia paroxysm, amyotrophic lateral sclerosis, acute myocardial infarction, spinal cerebellum degeneration, bone fracture, wounds, atopic dermatitis, osteoporosis, asthma, epilepsy, sterility, arteriosclerosis, pulmonary emphysema, lung edema, imperfect lactation or the like. Further, it can be used as a hypnotic sedative, an improver for nutritional conditions after operation, a hypertensive agent or a hypotensive agent.

In addition, it can be used as a prophylactic and/or therapeutic agent for HIV infections and AIDS (acquired immune deficiency syndrome).

As the salts of the compounds obtained by the screening method or screening kit described above, for example, pharmaceutically acceptable salts are used. For example, the salts with inorganic bases, the salts with organic bases, the salts with inorganic acids, the salts with organic acids, or the salts with basic or acidic amino acids are exemplified.

Preferable examples of the salts with inorganic bases include, for example, alkali metal salts such as sodium salt, potassium salt and the like, alkaline earth metal salts such as calcium salt, magnesium salt and the like, and an aluminum salt, ammonium salt and the like.

Preferable examples of the salts with organic bases include, for example, salts with trimethylamine, triethylamine, pyridine, picoline, 2,6-lutidine, ethanolamine, diethanolamine, triethanolamine, cyclohexylamine, dicylohexylamine, N,N'-dibenzylethylenediamine and the like.

Preferable examples of the salts with inorganic acids include, for examples, salts with hydrochloric acid, hydrobromic acid, sulfuric acid, phosphoric acid and the like.

Preferable examples of the salts with organic acids include, for examples, salts with formic acid, acetic acid, propionic acid, fumaric acid, oxalic acid, tartaric acid, maleic acid, citric acid, succinic acid, malic acid, methanesulfonic acid, benzenesulfonic acid, benzoic acid and the like.

Preferable examples of the salts with basic amino acids include, for examples, salts with arginine, lysine, ornithine and the like, and preferable examples of the salts with acidic amino acids include, for examples, salts with aspartic acid, glutamic acid and the like.

When the compounds or salts thereof obtained by the screening method or the screening kit of the present invention are used as the above-described pharmaceutical preparations, they can be used in the same manner as in use of the peptide of the present invention in the pharmaceutical preparations described above.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA: | deoxyribonucleic acid |
| cDNA: | complementary deoxyribonucleic acid |
| A: | adenine |
| T: | thymine |
| G: | guanine |
| C: | cytosine |
| Y: | thymine or cytosine |
| N: | thymine, cytosine, adenine or guanine |
| R: | adenine or guanine |
| M: | cytosine or adenine |
| W: | thymine or adenine |
| S: | cytosine or guanine |
| RNA: | ribonucleic acid |
| mRNA: | messenger ribonucleic acid |
| dATP: | deoxyadenosine triphosphate |
| dTTP: | deoxythymidine triphosphate |
| dGTP: | deoxyguanosine triphosphate |
| dCTP: | deoxycytidine triphosphate |
| ATP: | adenosine triphosphate |
| EDTA: | ethylenediaminetetraacetic acid |

| | |
|---|---|
| SDS: | sodium dodecyl sulfate |
| EIA: | enzyme immunoassay |
| Gly or G: | glycine |
| Ala or A: | alanine |
| Val or V: | valine |
| Leu or L: | leucine |
| Ile or I: | isoleucine |
| Ser or S: | serine |
| Thr or T: | threonine |
| Cys or C: | cysteine |
| Met or M: | methionine |
| Glu or E: | glutamic acid |
| Asp or D: | aspartic acid |
| Lys or K: | lysine |
| Arg or R: | arginine |
| His or H: | histidine |
| Phe or F: | phenylalanine |
| Tyr or Y: | :tyrosine |
| Trp or W: | tryptophan |
| Pro or P: | proline |
| Asn or N: | asparagine |
| Gln or Q: | glutamine |
| PGlu: | pyroglutamic acid |
| Me: | methyl group |
| Et: | ethyl group |
| Bu: | butyl group |
| Ph: | phenyl group |
| Nle: | norleucine |
| Thi: | 2-thienylalanine |
| Phg: | phenylglycine |
| Pya (2): | 2-pyridylalanine |
| Adi ($NH_2$): | 2-aminoadipic acid-6 amide |
| Hyp: | oxyproline (hydroxyproline) |
| Ac-Arg: | $N^{\alpha}$-acetylarginine |
| Lys (Ac): | $N^{\varepsilon}$-acetyllysine |
| Lys (Me): | $N^{\varepsilon}$-methyllysine |
| Lys (Tos): | $N^{\varepsilon}$-tosyllysine |
| Arg (Tos): | $N^{\varepsilon}$-tosylarginine |
| Phe (Cl): | 4-chlorophenylalanine |
| Nal (2): | 2-naphthylalanine |
| Cha: | cyclohexylalanine |
| Met (O): | methionine sulfoxide |
| Tyr (Me): | O-methyltyrosine |
| Tyr (I): | 3-iodotyrosine |

The substituent groups, protecting groups and reagents, which are frequently used throughout the specification, are shown by the following abbreviations.

| | |
|---|---|
| Tos: | p-toluenesulfonyl |
| HONB: | N-hydroxy-5-norbornane-2,3-dicarboxyimide |
| Bzl: | benzyl |
| Z: | benzyloxycarbonyl |
| Br-Z: | 2-bromobenzyloxycarbonyl |
| Cl-Z: | 2-chlorobenzyloxycarbonyl |
| Boc: | t-butoxycarbonyl |
| HOBt: | 1-hydroxybenztriazole |
| DCC: | N,N'-dicylohexylcarbodiimide |
| TFA: | trifluoroacetic acid |

| | |
|---|---|
| Fmoc: | N-9-fluorenylmethoxycarbonyl |
| DNP: | dinitrophenyl |
| Bum: | tertiary butoxymethyl |
| Trt: | trityl |
| Pbf: | 2,2,4,6,7-pentamethyl dihydrobenzofurane-5-sulfonyl |
| HOOBt: | 3-hydroxy-4-oxo-3,4-dihydro-1,2,3-benzotriazine |
| TFE: | trifluoroethanol |
| HOAt: | 1-hydroxy-7-azabenzotriazole |
| PyBrop: | bromotrispirolidinophosphonium hexafluorophosphate |
| TMS-Br: | trimethylsilyl bromide |
| TC: | thiazolidine-4(R)-carboxamide group |
| Bom: | benzyloxymethyl |
| NMP: | N-methylpyrrolidone |
| PAM: | phenylacetoamide methyl |
| DCM: | dichloromethane |
| DMF: | N,N-dimethylformamide |
| DIEA: | N,N-diisopropylethylamine |
| Clt: | 2-chlorotrityl |
| For: | formyl |
| Lys (Arg-Arg): | $N^{\varepsilon}$-arginyl arginyl lysine |
| AM: | aminomethyl |
| Pyn: | 1-pyrenylalanine |
| Mor: | morpholine |
| N-MeAla: | N-methylalanine |
| Lys $(Me)_2$: | $N^{\varepsilon}$-diniethyllysine |
| Dap: | diaminopropionic acid |
| Mmt: | 4-methoxytrityl |
| Adi: | adipoyl |
| Dap $(C_6)$: | $N^{\beta}$-hexanoyldiaminopropionic acid |
| DIPCDI: | N,N'-diisopropylcarbodiimide |
| NME2: | dimethylamine |
| Dap (Ac): | $N^{\beta}$-acetyldiaminopropionic acid |
| Dap (Adi): | $N^{\beta}$-adipoyldiaminopropionic acid |

The sequence identification numbers in the sequence listing of the specification indicates the following sequence, respectively.

[SEQ ID NO:1]

This shows the amino acid sequence of the peptide obtained in Example 1 described later.

[SEQ ID NO:2]

This shows the amino acid sequence of the peptide obtained in Example 2 described later.

[SEQ ID NO:3]

This shows the amino acid sequence of the peptide obtained in Example 3 described later.

[SEQ ID NO:4]

This shows the amino acid sequence of the peptide obtained in Example 4 described later.

[SEQ ID NO:5]

This shows the amino acid sequence of the peptide obtained in Example 5 described later.

[SEQ ID NO:6]

This shows the amino acid sequence of the peptide obtained in Example 6 described later.

[SEQ ID NO:7]

This shows the amino acid sequence of the peptide obtained in Example 7 described later.

[SEQ ID NO:8]

This shows the amino acid sequence of the peptide obtained in Example 8 described later.

[SEQ ID NO:9]

This shows the amino acid sequence of the peptide obtained in Example 9 described later.

[SEQ ID NO:10]

This shows the amino acid sequence of the peptide obtained in Example 10 described later.

[SEQ ID NO:11]

This shows the amino acid sequence of the peptide obtained in Example 11 described later.

[SEQ ID NO:12]

This shows the amino acid sequence of the peptide obtained in Example 12 described later.

[SEQ ID NO:13]

This shows the amino acid sequence of the peptide obtained in Example 13 described later.

[SEQ ID NO:14]

This shows the amino acid sequence of the peptide obtained in Example 14 described later.

[SEQ ID NO:15]

This shows the amino acid sequence of the peptide obtained in Example 15 described later.

[SEQ ID NO:16]

This shows the amino acid sequence of the peptide obtained in Example 16 described later.

[SEQ ID NO:17]

This shows the amino acid sequence of the peptide obtained in Reference Example 1 described later.

[SEQ ID NO:18]

This shows the amino acid sequence of the peptide obtained in Reference Example 2 described later.

[SEQ ID NO:19]

This shows the amino acid sequence of APJ.

[SEQ ID NO:20]

This shows a nucleotide sequence coding for the amino acid sequence shown by SEQ ID NO:19.

[SEQ ID NO:21]

This shows the amino acid sequence of the peptide obtained in Example 17 described later.

[SEQ ID NO:22]

This shows the amino acid sequence of the peptide obtained in Example 18 described later.

[SEQ ID NO:23]

This shows the amino acid sequence of the peptide obtained in Example 19 described later.

[SEQ ID NO:24]

This shows the amino acid sequence of the peptide obtained in Example 20 described later.

[SEQ ID NO:25]

This shows the amino acid sequence of the peptide obtained in Example 21 described later.

[SEQ ID NO:26]

This shows the amino acid sequence of the peptide obtained in Example 22 described later.

[SEQ ID NO:27]

This shows the amino acid sequence of the peptide obtained in Example 23 described later.

[SEQ ID NO:28]

This shows the amino acid sequence of the peptide obtained in Example 24 described later.

[SEQ ID NO:29]

This shows the amino acid sequence of the peptide obtained in Example 25 described later.

[SEQ ID NO:30]

This shows the amino acid sequence of the peptide obtained in Example 26 described later.

[SEQ ID NO:31]

This shows the amino acid sequence of the peptide obtained in Example 27 described later.

[SEQ ID NO:32]

This shows the amino acid sequence of the peptide obtained in Example 28 described later.

[SEQ ID NO:33]

This shows the amino acid sequence of the peptide obtained in Example 29 described later.

[SEQ ID NO:34]

This shows the amino acid sequence of the peptide obtained in Example 30 described later.

[SEQ ID NO:35]

This shows the amino acid sequence of the peptide obtained in Example 31 described later.

[SEQ ID NO:36]

This shows the amino acid sequence of the peptide obtained in Example 32 described later.

[SEQ ID NO:37]

This shows the amino acid sequence of the peptide obtained in Example 33 described later.

[SEQ ID NO:38]

This shows the amino acid sequence of the peptide obtained in Example 34 described later.

[SEQ ID NO:39]

This shows the amino acid sequence of the peptide obtained in Example 35 described later.

[SEQ ID NO:40]

This shows the amino acid sequence of the peptide obtained in Example 36 described later.

[SEQ ID NO:41]

This shows the amino acid sequence of the peptide obtained in Example 37 described later.

[SEQ ID NO:42]

This shows the amino acid sequence of the peptide obtained in Example 38 described later.

[SEQ ID NO:43]

This shows the amino acid sequence of the peptide obtained in Example 39 described later.

[SEQ ID NO:44]

This shows the amino acid sequence of the peptide obtained in Example 40 described later.

[SEQ ID NO:45]

This shows the amino acid sequence of the peptide obtained in Example 41 described later.

[SEQ ID NO:46]

This shows the amino acid sequence of the peptide obtained in Example 42 described later.

[SEQ ID NO:47]

This shows the amino acid sequence of the peptide obtained in Example 43 described later.

[SEQ ID NO:48]

This shows the amino acid sequence of the peptide obtained in Example 44 described later.

[SEQ ID NO:49]

This shows the amino acid sequence of the peptide obtained in Example 45 described later.

[SEQ ID NO:50]

This shows the amino acid sequence of the peptide obtained in Example 46 described later.

[SEQ ID NO:51]

This shows the amino acid sequence of the peptide obtained in Example 47 described later.

[SEQ ID NO:52]

This shows the amino acid sequence of the peptide obtained in Example 48 described later.

[SEQ ID NO:53]

This shows the amino acid sequence of the peptide obtained in Example 49 described later.

[SEQ ID NO:54]

This shows the amino acid sequence of the peptide obtained in Example 50 described later.

[SEQ ID NO:55]

This shows the amino acid sequence of the peptide obtained in Example 51 described later.

[SEQ ID NO:56]

This shows the amino acid sequence of the peptide obtained in Example 52 described later.

[SEQ ID NO:57]

This shows the amino acid sequence of the peptide obtained in Example 53 described later.

[SEQ ID NO:58]

This shows the amino acid sequence of the peptide obtained in Example 54 described later.

[SEQ ID NO:59]

This shows the amino acid sequence of the peptide obtained in Example 55 described later.

[SEQ ID NO:60]

This shows the amino acid sequence of the peptide obtained in Example 56 described later.

[SEQ ID NO:61]

This shows the amino acid sequence of the peptide obtained in Example 57 described later.

[SEQ ID NO:62]

This shows the amino acid sequence of the peptide obtained in Example 58 described later.

[SEQ ID NO:63]

This shows the amino acid sequence of the peptide obtained in Example 59 described later.

[SEQ ID NO:64]

This shows the amino acid sequence of the peptide obtained in Example 60 described later.

[SEQ ID NO:65]

This shows the amino acid sequence of the peptide obtained in Example 61 described later.

[SEQ ID NO:66]

This shows the amino acid sequence of the peptide obtained in Example 62 described later.

[SEQ ID NO:67]

This shows the amino acid sequence of the peptide obtained in Example 63 described later.

[SEQ ID NO:68]

This shows the amino acid sequence of the peptide obtained in Example 64 described later.

[SEQ ID NO:69]

This shows the amino acid sequence of the peptide obtained in Example 65 described later.

[SEQ ID NO:70]

This shows the amino acid sequence of the peptide obtained in Example 66 described later.

[SEQ ID NO:71]

This shows the amino acid sequence of the peptide obtained in Example 67 described later.

[SEQ ID NO:72]

This shows the amino acid sequence of the peptide obtained in Example 68 described later.

## EXAMPLES

The present invention is described in detail below with reference to REFERENCE EXAMPLES, EXAMPLES and EXPERIMENTAL EXAMPLES, but is not deemed to limit the scope of the present invention thereto.

Example 1

Production of Arg-Arg-Gln-Arg-Pro-Arg-Leu-Ser-Ala-Arg-Gly-Pro-Met-Pro-Pbe(Cl) (SEQ ID NO: 1)

0.25 mmol of Fmoc-Phe(Cl)-O-Clt resin (0.495 mmol/g) having Fmoc-Phe(Cl)-OH introduced into commercial 2-chlorotrityl resin (Clt resin, 1.22 mmol/g) was introduced into a reaction bath in a peptide synthesizer ABI 433A and subjected to solid phase synthesis by a Fmoc/DCC/HOBt method. As the side chain-protecting group for Fmoc amino acid, a Pbf group was used for Arg, a tBu group for Ser, and a Trt group for Gln. As other amino acids, those whose side chain had not been protected were used, and a peptide chain was introduced from Pro in the sequence shown above towards the N-terminus, whereby the objective protected peptide resin was obtained.

This resin, 200 mg (39.8 mmol), was stirred in 3 ml mixed solution of TFA, thioanisole, m-cresol, $H_2O$, ethanedithiol and triisopropylsilane (80 : 5 : 5 : 5 : 2.5 : 2.5) at room temperature for 2 hours, and ether was added to the reaction solution to precipitate white powder which was separated by centrifugation, and the supernatant was removed; these procedures were repeated 3 times. The residue was extracted with water and lyophilized to give 91.7 mg white powder. The resultant crude peptide, 52.2 mg, was subjected to preparative HPLC on a TSK GEL ODS 120T column (20x300 mm) and eluted with a linear gradient of solution A (0.1 % TFA-water)/solution B (acetonitrile containing 0.1 % TFA) (60 minutes) from 81/19 to 71/29, and the desired fractions were collected lyophilized to give 15.5 mg white powder.
$(M+H)^+$ by mass spectrometric analysis: 1858.7 (theoretical 1859.0)
HPLC elution time: 19.7 minutes
Elution conditions
Column: YMC ODS AM-301, S-5 μm, 120 A (4.6x 100 mm)
Eluent: elution with a linear gradient (25 minutes) of solution A (0.1 % TFA-water)/solution B (acetonitrile containing 0.1 % TFA) from 100/0 to 50/50.
Flow rate: 1.0 ml/min.

Hereinafter, the compounds in Examples 2 to 16 and Reference Example 1 were obtained by the same solid phase synthesis, de-protection and purification as in Example 1. The $(M+H)^+$ value (found value and theoretical value) of each compound by mass spectrometric analysis and the HPLC retention time (min.) are shown in Table 1.

Compound in Example 2:

Arg-Arg-Gln-Arg-Pro-Arg-Leu-Ser-His-Lys-Gly-Pro-Cha-Pro-Phe(Cl (SEQ ID NO: 2)

Compound in Example 3:

Arg-Pro-Arg-Leu-Ser-Ala-Arg-Gly-Pro-Met-Pro-Phe(Cl) (SEQ ID NO: 3)

Compound in Example 4:

Arg-Pro-Arg-Leu-Ser-Ala-Arg-Gly-Pro-Cha-Phe(Cl) (SEQ ID NO: 4)

Compound in Example 5:

Arg-Pro-Arg-Leu-Ser-Ala-Arg-Gly-Pro-Cha-Pro-Phe(Cl) (SEQ ID NO: 5)

Compound in Example 6:

Arg-Arg-Gln-Arg-Pro-Arg-Leu-Ser-Ala-Arg-Gly-Pro-Cha (SEQ ID NO: 6)

Compound in Example 7:

Arg-Pro-Arg-Leu-Ser-His-Lys-Gly-Pro-Cha-Pro-Phe(Cl) (SEQ ID NO: 7)

Compound in Example 8:

Arg-Pro-Arg-Leu-Ser-His-Lys-Gly-Pro-Cha-Pro-Phe(Cl) (SEQ ID NO: 8)

Compound in Example 9:

pGlu-Arg-Pro-Arg-Leu-Ser-His-Lys-Gly-Pro-Cha-Pro-Phe (SEQ ID NO: 9)

Compound in Example 10:

pGlu-Arg-Pro-Arg-Leu-Ser-His-Lys-Gly-Pro-Met-Pro-Phe(Cl) (SEQ ID NO: 10)

Compound in Example 11:

Arg-Pro-Arg-Leu-Ser-Ala-Arg-Gly-Pro-Cha-Pro-Phe (SEQ ID NO: 11)

Compound in Example 12:

Arg-Pro-Arg-Leu-Phe-Ala-Arg-Gly-Pro-Cha-Pro-Phe(Cl) (SEQ ID NO: 12)

Compound in Example 13:

Arg-Pro-Arg-Leu-Phe-His-Lys-Gly-Pro-Cha-Pro-Phe(Cl) (SEQ ID NO: 13)

Compound in Example 14:

Arg-Arg-Gln-Arg-Pro-Arg-Leu-Ser-His-Lys-Gly-Pro-Nle-Pro-Tyr (SEQ ID NO: 14)

Compound in Example 15:

Arg-Pro-Arg-Leu-Phe-His-Lys-Gly-Pro-Cha-Pro-Phe (SEQ ID NO: 15)

Compound in Example 16:

Arg-Pro-Arg-Leu-Phe-His-Lys-Gly-Pro-Met-Pro-Phe(Cl) (SEQ ID NO: 16)

Compound in Reference Example 1:

Leu-Val-Gln-Pro-Arg-Gly-Ser-Arg-Asn-Gly-Pro-Gly-Pro-Trp-Gln-Gly-Gly-Arg-Arg-Lys-Phe-Arg-Arg-Gln-Arg-Pro-Arg-Leu-Ser-His-Lys-Gly-Pro-Nle-Pro-Tyr (SEQ ID NO: 17)

Table 1

| | $M+H^+$ (Measured) | $M+H^+$ (Calculated) | HPLC Retention Time (min.) under the same conditions as Ex. 1 |
|---|---|---|---|
| Compound in Ex. 2 | 1919.7 | 1919.1 | 21.5 |
| Compound in Ex. 3 | 1418.4 | 1418.7 | 20.2 |
| Compound in Ex. 4 | 1343.7 | 1343.7 | 22.2 |
| Compound in Ex. 5 | 1440.7 | 1440.8 | 22.8 |
| Compound in Ex. 6 | 1602.6 | 1603.0 | 18.4 |
| Compound in Ex. 7 | 1478.5 | 1478.8 | 22.3 |
| Compound in Ex. 8 | 1456.5 | 1456.7 | 19.7 |
| Compound in Ex. 9 | 1555.8 | 1555.9 | 21.4 |
| Compound in Ex. 10 | 1567.7 | 1567.8 | 19.8 |
| Compound in Ex. 11 | 1406.5 | 1406.8 | 21.6 |
| Compound in Ex. 12 | 1500.5 | 1500.8 | 24.2 |
| Compound in Ex. 13 | 1538.7 | 1538.9 | 22.8 |
| Compound in Ex. 14 | 1860.9 | 1861.1 | 16.8 |
| Compound in Ex. 15 | 1504.7 | 1504.9 | 22.1 |
| Compound in Ex. 16 | 1516.6 | 1516.8 | 20.6 |
| Compound in Ref. Ex. 1 | 4193.6 | 4194.6 | 17.5 |

The compounds in Examples 17 to 19, Examples 21 to 46, and Examples 48 to 50 were obtained by the same solid phase synthesis, de-protection and purification as in Example 1. The $(M+H)^+$ value (found value and theoretical value) of each compound by mass spectrometric analysis and the HPLC retention time (min.) are shown in Table 2.

Compound in Example 17:

Arg-Arg-Gln-Arg-Pro-Arg-Leu-Ser-Ala-Arg-Gly-Pro-Met-Pro-Phe (SEQ ID NO: 21)

Compound in Example 18:

Arg-Arg-Gln-Arg-Pro-Arg-Leu-Ser-Ala-Arg-Gly-Pro-Met (SEQ ID NO: 22)

Compound in Example 19:

Arg-Arg-Gln-Arg-Pro-Arg-Leu-Ser-Ala-Arg-Gly-Pro (SEQ ID NO: 23)

Compound in Example 21:

Arg-Arg-Lys(Arg-Arg)-Arg-Pro-Arg-Leu-Ser-His-Lys-Gly-Pro-Met-Pro-Phe (SEQ ID NO: 25)

Compound in Example 22:

Arg-Arg-Arg-Arg-Pro-Arg-Leu-Ser-His-Lys-Gly-Pro-Met-Pro-Phe (SEQ ID NO: 26)

Compound in Example 23:

Arg-Arg-Lys-Arg-Pro-Arg-Leu-Ser-His-Lys-Gly-Pro-Met-Pro-Phe (SEQ ID NO: 27)

Compound in Example 24:

Arg-Pro-Arg-Leu-Ser-Ala-Arg-Gly-Pro-Met-Pro-Phe (SEQ ID NO: 28)

Compound in Example 25:

Arg-Arg-Ala-Arg-Pro-Arg-Leu-Ser-His-Lys-Gly-Pro-Met-Pro-Phe (SEQ ID NO: 29)

Compound in Example 26:

pGlu-Arg-Pro-Arg-Leu-Ser-Ala-Arg-Gly-Pro-Met-Pro-Phe (SEQ ID NO: 30)

Compound in Example 27:

Arg-Pro-Arg-Leu-Ser-Ala-Arg-Gly-Pro-Met (SEQ ID NO: 31)

Compound in Example 28:

Arg-Pro-Arg-Leu-Ser-His-Lys-Gly-Pro-Met-Phe(Cl) (SEQ ID NO: 32)

Compound in Example 29:

pGlu-Arg-Pro-Arg-Leu-Ser-Ala-Lys-Gly-Pro-Met-Pro-Phe (SEQ ID NO: 33)

Compound in Example 30:

pGlu-Arg-Pro-Arg-Leu-Ser-Arg-Lys-Gly-Pro-Met-Pro-Phe (SEQ ID NO: 34)

Compound in Example 31:

Arg-Pro-Arg-Leu-Phe-Ala-Arg-Gly-Pro-Met-Pro-Phe (SEQ ID NO: 35)

Compound in Example 32:

Arg-Pro-Arg-Leu-Ser-His-Lys-Gly-Pro-Met-Pro-Nal(2) (SEQ ID NO: 36)

Compound in Example 33:

Arg-Arg-Phe-Arg-Pro-Arg-Leu-Ser-His-Lys-Gly-Pro-Met-Pro-Phe (SEQ ID NO: 37)

Compound in Example 34:

pGlu-Arg-Pro-Arg-Leu-Ser-His-Arg-Gly-Pro-Met-Pro-Phe (SEQ ID NO: 38)

Compound in Example 35:

pGlu-Arg-Pro-Arg-Leu-Ser-His-Lys-Gly-Pro-Met-Phe(Cl) (SEQ ID NO: 39)

Compound in Example 36:

Arg-Pro-Arg-Leu-Ser-His-Lys-Gly-Pro-Met-Cha (SEQ ID NO: 40)

Compound in Example 37:

Arg-Pro-Arg-Leu-Phe-His-Lys-Gly-Pro-Cha-Phe(Cl) (SEQ ID NO: 41)

Compound in Example 38:

pGlu-Arg-Pro-Arg-Leu-Ser-Leu-Lys-Gly-Pro-Met-Pro-Phe (SEQ ID NO: 42)

Compound in Example 39:

pGlu-Arg-Pro-Arg-Leu-Ser-His-Lys-Gly-Pro-Met-Pro-Nal(2) (SEQ ID NO: 43)

Compound in Example 40:

Arg-Pro-Arg-Leu-Ser-His-Lys-Gly-Pro-Met-Nal(2) (SEQ ID NO: 44)

Compound in Example 41:

pGlu-Arg-Pro-Arg-Leu-Ser-Arg-Arg-Gly-Pro-Met-Pro-Phe (SEQ ID NO: 45)

Compound in Example 42:

pGlu-Arg-Pro-Arg-Leu-Phe-Arg-Arg-Gly-Pro-Met-Pro-Phe (SEQ ID NO: 46)

Compound in Example 43:

pGlu-Arg-Pro-Arg-Leu-Ser-Phe-Lys-Gly-Pro-Met-Pro-Phe (SEQ ID NO: 47)

Compound in Example 44:

pGlu-Arg-Pro-Arg-Leu-Phe-His-Lys-Gly-Pro-Met-Pro-Phe (SEQ ID NO: 48)

Compound in Example 45:

pGlu-Arg-Pro-Arg-Leu-Ser-His-Lys-Gly-Pro-Met-Cha (SEQ ID NO: 49)

Compound in Example 46:

pGlu-Arg-Pro-Arg-Leu-Ser-His-Lys-Gly-Pro-Met-Nal(2) (SEQ ID NO: 50)

Compound in Example 48:

pGlu-Arg-Pro-Arg-Leu-Ser-His-Phe-Gly-Pro-Met-Pro-Phe (SEQ ID NO: 52)

Compound in Example 49:

Arg-Pro-Arg-Leu-Ser-His-Lys-Gly-Pro-Met-Pro-Cha (SEQ ID NO: 53)

Compound in Example 50:

pGlu-Arg-Pro-Arg-Leu-Ser-His-Leu-Gly-Pro-Met-Pro-Phe (SEQ ID NO: 54)

Table 2

| | $M + H^+$ (Measured) | $M + H^+$ (Calculated) | H P L C Retention Time (min.) under the same conditions as Ex. I |
|---|---|---|---|
| Compound in Ex. 17 | 1825.1 | 1825.0 | 18.43 |
| Compound in Ex. 18 | 1581.0 | 1580.9 | 15.22 |
| Compound in Ex. 19 | 1449.6 | 1449.9 | 14.09 |
| Compound in Ex. 21 | 2175.9 | 2175.3 | 17.46 |
| Compound in Ex. 22 | 1890.9 | 1891.1 | 17.55 |
| Compound in Ex. 23 | 1863.1 | 1863.1 | 17.32 |
| Compound in Ex. 24 | 1384.8 | 1384.8 | 18.74 |
| Compound in Ex. 25 | 1805.9 | 1806.0 | 17.73 |
| Compound in Ex. 26 | 1495.7 | 1495.8 | 19.10 |
| Compound in Ex. 27 | 1140.8 | 1140.6 | 15.16 |
| Compound in Ex. 28 | 1359.6 | 1359.7 | 19.32 |
| Compound in Ex. 29 | 1467.7 | 1467.8 | 18.86 |
| Compound in Ex. 30 | 1522.7 | 1522.9 | 18.34 |
| Compound in Ex. 31 | 1444.6 | 1444.8 | 20.92 |
| Compound in Ex. 32 | 1472.6 | 1472.8 | 20.48 |
| Compound in Ex. 33 | 1883.4 | 1883.0 | 18.05 |
| Compound in Ex. 34 | 1561.8 | 1561.8 | 18.40 |

Table continued

| | M + H+ (Measured) | M + H+ (Calculated) | H P L C Retention Time (min.) under the same conditions as Ex. I |
|---|---|---|---|
| Compound in Ex. 35 | 1471.0 | 1470.7 | 19.39 |
| Compound in Ex. 36 | 1331.5 | 1331.8 | 19.22 |
| Compound in Ex. 37 | 1441.8 | 1441.8 | 22.38 |
| Compound in Ex. 38 | 1509.7 | 1509.8 | 20.46 |
| Compound in Ex. 39 | 1583.6 | 1583.8 | 20.83 |
| Compound in Ex. 40 | 1375.5 | 1375.7 | 20.35 |
| Compound in Ex. 41 | 1580.7 | 1580.9 | 18.51 |
| Compound in Ex. 42 | 1641.5 | 1641.9 | 20.15 |
| Compound in Ex. 43 | 1543.6 | 1543.8 | 20.87 |
| Compound in Ex. 44 | 1593.7 | 1593.8 | 19.70 |
| Compound in Ex. 45 | 1442.5 | 1442.8 | 19.88 |
| Compound in Ex. 46 | 1486.6 | 1486.8 | 20.26 |
| Compound in Ex. 48 | 1522.7 | 1522.8 | 21.14 |
| Compound in Ex. 49 | 1428.7 | 1428.8 | 19.41 |
| Compound in Ex. 50 | 1515.9 | 1515.8 | 20.71 |

Example 20:

Production of Arg-Arg-Gln-Arg-Pro-Arg-Leu-Ser-His-Lys-Gly-Pro-Met(O) (SEQ ID NO: 24)

20 mg crude peptide obtained in the same method as in Example 1 above was applied onto a reverse phase chromatography column (2.6×60 cm) charged with LiChroprep™ RP-18, then washed with 200 ml of 0.1 % aqueous TFA and eluted with a linear gradient using 300 ml of 0.1 % aqueous TFA and 300 ml of 33 % aqueous acetonitrile containing 0.1 % TFA, and the desired fractions were collected and lyophilized to give 30 mg white powder.
$(M+H)^+$ by mass spectrometric analysis: 1634.9 (theoretical 1634.9)
HPLC elution time: 14.03 minutes
Elution conditions
Column: Wakosil5C18T (4.6 × 100 mm)
Eluent: elution with a linear gradient (25 minutes) of solution A (0.1 %TFA-water)/solution B (acetonitrile containing 0.1 % TFA) from 55/95 to 55/45. Flow rate: 1.0 ml/min.

Example 47:

Production of Arg-Pro-Arg-Leu-Phe-Ala-Arg-Gly-Pro-Met-Phe (SEQ ID NO: 51)

15 mg crude peptide obtained in the same method as in Example 1 above was subjected to preparative HPLC on a TSK GEL ODS 120T column (20x300 mm) and eluted with a linear gradient using solution A (0.1 %TFA-water)/solution B (acetonitrile containing 0.1 % TFA) (60 minutes) from 81/19 to 71/29, and the desired fractions were collected and lyophilized to give 10.5 mg white powder.
$(M+H)^+$ by mass spectrometric analysis: 1347.5 (theoretical 1347.7)
HPLC elution time: 15.81 minutes
Elution conditions
Column: YMC ODS AM-301, S-5 μm, 120 A (4.6x 100 mm)
Eluent: elution with a linear gradient (25 minutes) of solution A (0.1 % TFA-water)/solution B (acetonitrile containing 0.1 % TFA) from 10/90 to 60/40.
Flow rate: 1.0 ml/min.

Reference Example 2:

Production of pGlu-Arg-Pro-Arg-Leu-Ser-His-Lys-Gly-Pro-Nle- Pro-Tyr (SEQ ID NO: 18)

0.25 mmol of Fmoc-Gly-O-Clt resin (0.392 mmol/g) having Fmoc-Gly-OH introduced into commercial 2-chlorotrityl resin (Clt resin, 1.3 mmol/g) was introduced into a reaction bath in a peptide synthesizer ABI 433A, and Fmoc-Lys (Boc), Fmoc-His (Trt), Fmoc-Ser (tBu), Fmoc-Leu, Fmoc-Arg (Pbf), Fmoc-Pro, Fmoc-Arg (Pbf) and Boc-Gln were introduced in this order by the Fmoc/DCC/HOBt method to give the objective protected peptide resin.

1 g of this resin was stirred in 20 ml mixture of AcOH : TFE : DCM (1 : 2 : 7) at room temperature for 2 hours, and the resin was removed by filtration, the solvent was distilled away, whereby the product was crystallized to give 362 mg of (Boc-Gln-Arg(Pbf)-Pro-Arg(Pbf)-Leu-Ser(iBu)-His(Trt)-Lys(Boc)-Gly-OH) was obtained.

Boc-Pro, Boc-Nle and Boc-Pro were condensed in this order with H-Tyr(Bzl)-OBzl HCl to give 217.8 mg Boc-Pro-Nic-Pro-Tyr(Bzl)-Bzl.

100 mg of Boc-Gln-Arg(Pbf)-Pro-Arg(Pbf)-Leu-Ser(tBu)-His(Trt)-Lys(Boc)-Gly-OH and 8.04 mg HOAt were dissolved in 1 ml mixture of DCM : DMF (4 : 1), and 38.5 μl DIEA, 34.20 mg PyBrop and 62.2 mg of H-Pro-Nle-Pro-Tyr (Bzl)-(Bzl)·HCl (Boc-Pro-Nle-Pro-Tyr(Bzl)-OBzl (prepared by treating with 4 N HCl/dioxane) were added thereto under cooling on ice, and after the ice bath was removed, the mixture was stirred at room temperature for 1 hour. After the solution was neutralized by adding citric acid crystals, the solvent was distilled away, water was added thereto, and the precipitated solids were extracted with chloroform. The product was washed with 1 N hydrochloric acid, saturated aqueous sodium bicarbonate, and saturated saline and dried over sodium sulfate anhydride, and the solvent was distilled away, ether was added thereto, and the resultant powder was collected by filtration and purified by re-precipitation with ethyl acetate and ether to give 122.27 mg of Boc-Gln-Arg(Pbf)-Pro-Arg(Pbf)-Leu-Ser(tBu)-His(Trt)-Lys(Boc)-Gly-Pro-Nle-Pro-Tyr(Bzl)-OBzl.

63.6 mg of this protected peptide was stirred in a mixture of 1089 μl thioanisole, 122 μl m-cresol, 238.5 μl triisopropylsilane and 4.7 ml TFA at room temperature for 90 minutes, and then 1.23 ml TMS-Br was added thereto, the reaction mixture was stirred for 1 hour under cooling on ice, and after the ice bath was removed, the reaction solution was further stirred for 1 hour on a water bath at 20 °C. After the reaction, the reaction solution was distilled away, then ether was added to the residues to precipitate white powder which was then separated by filtration, and the supernatant was removed; these procedures were repeated 3 times. The residue was extracted with water, lyophilized, and passed through a Sephadex G-25 gel filtration column with 50 % aqueous acetic acid, and major fractions were heated at 70 °C for 2 hours in 80 % AcOH, and the solution was diluted with water and lyophilized. The resultant crude peptide was subjected to preparative HPLC on a TSK GEL ODS 120T column (20x 300 mm) and eluted with a linear gradient of solution A (0.1 %TFA-water)/solution B (acetonitrile containing 0.1 % TFA) (60 minutes) from 85/15 to 75/25, and the fractions containing the desired product were collected and lyophilized to give 29.18 mg white powder.
$(M+H)^+$ by mass spectrometric analysis: 1532.0 (theoretical 1531.9)
HPLC elution time: 14.6 minutes
Elution conditions
Column: Wakosil 5C18T (4.6×100 mm)
Eluent: elution with a linear gradient (25 minutes) of solution A (0.1 %TFA-water)/solution B (acetonitrile containing 0.1 % TFA) from 95/5 to 45/55.
Flow rate: 1.0 ml/min.

Example 51:

Production of Arg-Arg-Gln-Arg-Pro-Arg-Leu-Ser-Ala-Arg-Gly-NMe2 (SEQ ID NO: 55)

Commercial 4-sulfamylbutyryl AM resin, 1 g (1.09 mmol/g), was reacted at room temperature for 30 minutes with 356 mg (1.199 mmol) Fmoc-Gly-OH, 567 mg (1.09 mmol) PyBOP and 0.417 ml (2.398 mmol) DIEA in DMF. After filtration, the resin was washed with DMF and reacted at room temperature for 2 hours with 1.03 ml (10.9 mmol) Ac2O and 0.882 ml (10.9 mmol) pyridine in DMF. The resin was washed with DMF and MeOH, dried and treated with piperidine, and the absorbance of free Fmoc group was measured, and 1.0496 g (Fmoc-Gly content: 0.295 mmol/g) of Fmoc-Gly-4-sulfamylbutyryl AM resin was obtained.

Using the Fmoc-Gly-4-sulfamylbutyryl AM resin, the same solid phase synthesis as in Example 1 was carried out, and after the final Arg was introduced, the Fmoc group was replaced by a Boc group. This resin was activated with $ICH_2CN$ and DIEA in NMP and then treated with 50 % aqueous $NHMe_2$ to cut off the protected peptide. The peptide was treated with a mixture of TFA, thioanisole, m-cresol, $H_2O$, ethanedithiol, triisopropylsilane (80 : 5 : 5 : 5 : 2.5 : 2.5) in the same manner as in Example 1 and purified by HPLC in the same manner to give the objective product.

Example 52:

Production of Arg-Arg-Gln-Arg-Pro-Arg-Leu-Ser-Ala-Arg-Gly-Mor (SEQ ID NO: 56)

The objective product was obtained by the same treatment and purification as in Example 51: except that morpholine was used in place of 50 % aqueous $NHMe_2$.

The compounds in Examples 53 to 64 were obtained by the same solid phase synthesis, de-protection and purification as in Example 1.

Example 53:

Arg-Pro-Arg-Leu-Ser-Ala-Arg-Gly-Pro-Ala-Pro-Phe(Cl) (SEQ ID NO: 57)

Example 54:

Arg-Pro-Arg-Leu-Ser-Ala-Arg-Gly-Gly-Met-Pro-Phe(Cl) (SEQ ID NO: 58)

Example 55:

Arg-Pro-Arg-Leu-Ser-Ala-Arg-Gly-N-MeAla-Met-Pro-Phe(Cl) (SEQ ID NO: 59)

Example 56:

Arg-Pro-Arg-Leu-Ser-His-Ala-Gly-Pro-Cha-Pro-Phe(Cl) (SEQ ID NO: 60)

Example 57:

Arg-Pro-Arg-Ala-Ser-His-Lys-Gly-Pro-Cha-Pro-Phe(Cl) (SEQ ID NO: 61)

Example 58:

Arg-Pro-Ala-Leu-Ser-His-Lys-Gly-Pro-Cha-Pro-Phe(Cl) (SEQ ID NO: 62)

Example 59:

The C-terminus of the compound in Example 1 was amidated in a usual manner to give Arg-Arg-Gln-Arg-Pro-Arg-Leu-Ser-Ala-Arg-Gly-Pro-Met-Pro-Phe-(Cl)-$NH_2$.

Example 60:

Arg-Pro-Arg-Leu-Ser-Ala-Arg-Gly-Pro-Cha-Pyn (SEQ ID NO: 63)

Example 61:

Arg-Pro-Arg-Leu-Ser-Ala-Arg-Gly-Pro-Cha-Pro-Pyn (SEQ ID NO: 64)

Example 62:

Arg-Arg-Gln-Arg-Pro-Arg-Leu-Ser-Ala-Arg-Gly-Gly-Cha (SEQ ID NO: 65)

Example 63:

Arg-Pro-Lys$(Me)_2$-Leu-Ser-Ala-Arg-Gly-Pro-Met-Pro-Phe (SEQ ID NO: 66)

Example 64:

Arg-Pro-Arg-Leu-Ser-Ala-Lys$(Me)_2$-Gly-Pro-Met-Pro-Phe (SEQ ID NO: 67)

Example 65: Production of Arg-Pro-Arg-Leu-Ser-Dap-Arg-Gly-Pro-Cha-Pro-Phe(Cl) (SEQ ID NO: 68)

Using commercial Wang resin and Fmoc-Dap (Mmt) whose side chain amino group was protected with Mmt, solid phase synthesis was carried out in the same manner as in Example 1. The resulting resin was treated and purified in the same manner as in Example 1 to give the objective product.

Example 66:

Production of Arg-Pro-Arg-Leu-Ser-Dap(Ac)-Arg-Gly-Pro-Cha-Pro-Phe(Cl) (SEQ ID NO: 69)

The resin obtained in Example 65 was treated with 1 % TFA/5 % triisopropylsilane/94 % DCM, whereby only the protecting group Mmt for the side chain of Dap was removed, and the resin was acetylated with acetic anhydride, then treated and purified in the same manner as in Example 65, to give the objective product.

Example 67:

Production of Arg-Pro-Arg-Leu-Ser-Dap($C_6$)-Arg-Gly-Pro-Cha-Pro-Phe(Cl) (SEQ ID NO: 70)
The objective product was obtained in the same manner as in Example 66 except that the amino group was modified with hexanoic acid and DIPCDI in place of acetic anhydride.

Example 68:

Production of Arg-Pro-Arg-Leu-Ser-Dap(Adi)-Arg-Gly-Pro-Cha-Pro-Phe(Cl) (SEQ ID NO: 71)

The objective product was obtained by using adipic acid in place of hexanoic acid in Example 67.
The $(M+H)^+$ value (found value and theoretical value) of each of the compounds in Examples 51 to 68 by mass spectrometric analysis and the HPLC retention time (min.) are shown in Table 3.

Table 3

| | $M+H^+$ (Measured) | $M+H^+$ (Calculated) | H P L C Retention Time (min.) under the same conditions as Ex. 1 |
|---|---|---|---|
| Compound in Ex. 51 | 1379.9 | 1379.9 | 13.44 |
| Compound in Ex. 52 | 1421.8 | 1421.9 | 13.38 |
| Compound in Ex. 53 | 1358.6 | 1358.7 | 18.78 |
| Compound in Ex. 54 | 1378.6 | 1378.7 | 19.44 |
| Compound in Ex. 55 | 1406.6 | 1406.7 | 20.21 |
| Compound in Ex. 56 | 1421.8 | 1421.8 | 22.87 |
| Compound in Ex. 57 | 1436.4 | 1436.8 | 21.61 |
| Compound in Ex. 58 | 1393.4 | 1393.8 | 22.42 |
| Compound in Ex. 59 | 1859.0 | 1859.0 | 19.00 |
| Compound in Ex. 60 | 1433.6 | 1433.8 | 25.20 |
| Compound in Ex. 61 | 1530.9 | 1530.9 | 25.96 |
| Compound in Ex. 62 | 1563.1 | 1562.8 | 17.39 |
| Compound in Ex. 63 | 1384.5 | 1384.8 | 18.40 |
| Compound in Ex. 64 | 1384.5 | 1384.8 | 18.43 |
| Compound in Ex. 65 | 1455.6 | 1455.8 | 22.12 |
| Compound in Ex. 66 | 1497.6 | 1497.8 | 22.68 |
| Compound in Ex. 67 | 1553.8 | 1553.9 | 20.13 |
| Compound in Ex. 68 | 1583.6 | 1583.8 | 22.76 |
| (HPLC conditions in Examples 60 and 61:C, 0 to 90 %; AcCN/45 min.; Flow, 1 ml/min.; YMC ODS AM-301, S-5 μm, 120A (4.6 × 100 mm).) | | | |

Experimental Example 1: Measurement of an inhibitory activity on forskolin-stimulated cAMP production

CHO-A10 clone 6 cells described in Example 7 in WO 99/33976 (Japanese Patent Application No. 220853/1998) were inoculated at a density of $3\times10^5$ cells/well on a 24-well tissue culture plate and cultured overnight. Hanks' balanced salt solution (HBSS) containing 0.2 mM 3-isobutyl-1-methylxanthine (IBMX) and 0.05 % bovine serum albumin was prepared as an assay buffer, and each well was washed twice with 500 μl assay buffer and pre-incubated at 37 °C for 30 minutes. Further, each well was washed once with 500 μl assay buffer, and a sample dissolved in the assay buffer containing 1 μM forskolin was added at a volume of 500 μl/well and incubated at 37 °C for 30 minutes. Wells incubated with the assay buffer not containing forskolin were prepared in the same manner in order to determine the basal level of cellular cAMP production, while wells incubated with the assay buffer containing forskolin were also prepared in order to determine the maximum level of cAMP production stimulated with forskolin. After incubation was finished, each well was washed once with 500 μl assay buffer, and 500 μl lysis buffer 1B attached to a cAMP EIA system from Amersham was added to each well, to extract cAMP. According to the protocol of the kit, 100 μl of each extract was used to determine the level of cAMP. For the inhibitory activity of the sample on cAMP production, the difference (inhibited cAMP production) between the maximum level and the level of cAMP in the sample-containing wells was determined, and the inhibitory activity was expressed as percentage relative to the increased level of cAMP production stimulated with forskolin (that is, the difference between the maximum level and basal level), and from this dose-reaction curve, the $EC_{50}$ was determined.

Table 4 shows the activities of the compounds in Examples 1 to 16 as determined in the method in Experimental Example 1, Table 5 shows the activities of the compounds in Examples 17 to 50 as determined in the method in Experimental Example 1, and Table 6 shows the activities of the compounds in Examples 51 to 68 as determined in the method in Experimental Example 1.

Table 4

| Compounds | $EC_{50}$(nM) |
|---|---|
| Compound in Ex. 1 | 0.39 |
| Compound in Ex. 2 | 0.53 |
| Compound in Ex. 3 | 0.32 |
| Compound in Ex. 4 | 0.65 |
| Compound in Ex. 5 | 0.51 |
| Compound in Ex. 6 | 0.55 |
| Compound in Ex. 7 | 0.41 |
| Compound in Ex. 8 | 0.34 |
| Compound in Ex. 9 | 0.35 |
| Compound in Ex. 10 | 0.61 |
| Compound in Ex. 11 | 0.59 |
| Compound in Ex. 12 | 1.2 |
| Compound in Ex. 13 | 1 |
| Compound in Ex. 14 | 0.28 |
| Compound in Ex. 15 | 0.46 |
| Compound in Ex. 16 | 0.38 |

Table 5

| Compounds | $EC_{50}$ (nM) |
|---|---|
| Compound in Ex. 17 | 0.39 |
| Compound in Ex. 18 | 0.8 |
| Compound in Ex. 19 | 0.26 |
| Compound in Ex. 20 | 0.48 |
| Compound in Ex. 21 | 0.57 |
| Compound in Ex. 22 | 0.32 |
| Compound in Ex. 23 | 0.31 |
| Compound in Ex. 24 | 0.25 |
| Compound in Ex. 25 | 0.25 |
| Compound in Ex. 26 | 0.17 |

Table continued

| Compounds | $EC_{50}$ (nM) |
|---|---|
| Compound in Ex. 27 | 0.093 |
| Compound in Ex. 28 | 0.16 |
| Compound in Ex. 29 | 0.22 |
| Compound in Ex. 30 | 0.081 |
| Compound in Ex. 31 | 0.14 |
| Compound in Ex. 32 | 0.1 |
| Compound in Ex. 33 | 1.1 |
| Compound in Ex. 34 | 0.2 |
| Compound in Ex. 35 | 0.43 |
| Compound in Ex. 36 | 0.22 |
| Compound in Ex. 37 | 0.43 |
| Compound in Ex. 38 | 0.16 |
| Compound in Ex. 39 | 0.4 |
| Compound in Ex. 40 | 0.37 |
| Compound in Ex. 41 | 0.069 |
| Compound in Ex. 42 | 0.15 |
| Compound in Ex. 43 | 0.24 |
| Compound in Ex. 44 | 0.22 |
| Compound in Ex. 45 | 0.3 |
| Compound in Ex. 46 | 0.52 |
| Compound in Ex. 47 | 0.36 |
| Compound in Ex. 48 | 0.3 |
| Compound in Ex. 49 | 0.71 |
| Compound in Ex. 50 | 0.16 |

Table 6

| | $EC_{50}$ (nM) |
|---|---|
| Compound in Ex. 51 | 0.49 |
| Compound in Ex. 52 | 1.2 |
| Compound in Ex. 53 | 0.37 |
| Compound in Ex. 54 | 0.19 |
| Compound in Ex. 55 | 0.27 |
| Compound in Ex. 56 | 0.58 |
| Compound in Ex. 57 | 0.52 |
| Compound in Ex. 58 | 0.41 |
| Compound in Ex. 59 | 0.32 |
| Compound in Ex. 60 | 1.1 |
| Compound in Ex. 61 | 2.2 |

Table continued

| | $EC_{50}$ (nM) |
|---|---|
| Compound in Ex. 62 | 0.23 |
| Compound in Ex. 63 | 0.33 |
| Compound in Ex. 64 | 0.38 |
| Compound in Ex. 65 | 0.52 |
| Compound in Ex. 66 | 0.69 |
| Compound in Ex. 67 | 1.1 |
| Compound in Ex. 68 | 0.42 |

Experimental Example 2: Receptor binding assay-1:

The compound in Reference Example 1 was labeled with $^{125}$I by the lactoperoxidase method in the following manner. 20 μl lactoperoxidase (Sigma Co.) dissolved at a concentration of 10 μg/ml in 0.1 M HEPES-NaOH/pH 7.0, 20 μl iodine-125 (IMS-30, 74 MBq, Amersham) and 30 % aqueous hydrogen peroxide (Wako Pure Chemical Industries, Ltd.) were diluted 6000-fold, and 20 μl of the diluted aqueous solution was added to 20 μl of 0.1 mM aqueous peptide solution, mixed with a vortex mixer and incubated at room temperature for 10 minutes. The reaction was terminated by adding 600 μl distilled water containing 0.1 % TFA, and the reaction solution was separated by reverse phase HPLC using a TSKgel ODS-80TM CTR column (4.6× 100 mm), and the peak fractions of the labeled product formed by the reaction were collected. This product was mixed with an equal volume of an assay buffer (50 mM Tris-HCl/pH 7.5, 5 mM EDTA, 0.5 mM PMSF, 20 μg/ml leupeptin, 0.1 μg/ml pepstatin A, 4 μg/ml E-64 (Peptide Research Laboratory, Co.), 0.1 % bovine serum albumin) and stored at -30 °C before use.

CHO-A10 cells were cultured, recovered with PBS containing 5 mM EDTA from a culture vessel, and suspended in the above-mentioned assay buffer not containing bovine serum albumin. The cells were homogenized 3 times at 12,000 rpm for 15 seconds by a Polytron homogenizer (manufactured by Kinematica Inc.) and then centrifuged (1000xg, 4 °C, 10 minutes), and the supernatant was recovered. The precipitates were subjected again to the same procedure as above, and the respective supernatants were combined and centrifuged at 100,000×g, 4 °C for 1 hour, to recover precipitates (membrane fraction). The membrane fraction was suspended again in a small amount of the buffer, homogenized with a Teflon homogenizer and stored at -80 °C before use.

Binding assay was carried out under the following conditions. 100 μm solution containing 0.25 μg of the membrane fraction, the $^{125}$I-labeled compound at a final concentration of 100 pM, a test sample and an assay buffer were put onto each well of a 96-well polypropylene plate, and incubated at room temperature for 1.5 hours. Measurement of non-specific binding was carried out using a well containing 1 μM unlabeled compound in place of the test sample. After the incubation was finished, a cell harvester (Packard) adapted to 96 wells and a filter unit (GF/C, Packard) were used for separation thereby capturing the membrane fraction on the filter. After the filter was sufficiently dried, Microcinti 0 (Packard) was added thereto, and using Top Count (Packard), the amount of the labeled material captured together with the membrane fraction on the filter was measured. After the amount of non-specific binding was subtracted from each measurement, the degree (degree of inhibition) of the binding reduced by adding the test sample, relative to that (total binding) of the well not containing the sample, was calculated, and the $IC_{50}$ was calculated from a dose-inhibition curve of each sample (Tables 7 to 9).

Table 7

| Compounds | $IC_{50}$(nM) |
|---|---|
| Compound in Ex. 1 | 0.2 |
| Compound in Ex. 2 | 0.47 |
| Compound in Ex. 3 | 0.91 |
| Compound in Ex. 4 | 1 |
| Compound in Ex. 5 | 1.2 |
| Compound in Ex. 6 | 1.4 |
| Compound in Ex. 7 | 1.4 |

Table continued

| Compounds | $IC_{50}$(nM) |
|---|---|
| Compound in Ex. 8 | 13 |
| Compound in Ex. 9 | 13 |
| Compound in Ex. 10 | 15 |
| Compound in Ex. 11 | 2.2 |
| Compound in Ex. 12 | 2.3 |
| Compound in Ex. 13 | 2.7 |
| Compound in Ex. 14 | 2.7 |
| Compound in Ex. 15 | 8 |
| Compound in Ex. 16 | 8 |

Table 8

| Compounds | $IC_{50}$ (nM) |
|---|---|
| Compound in Ex. 17 | 0.75 |
| Compound in Ex. 18 | 2.5 |
| Compound in Ex. 21 | 2.9 |
| Compound in Ex. 22 | 6.7 |
| Compound in Ex. 23 | 9.2 |
| Compound in Ex. 24 | 27 |
| Compound in Ex. 25 | 34 |
| Compound in Ex. 26 | 37 |

Table 9

| | $I C_{50}$ (nM) |
|---|---|
| Compound in Ex. 53 | 0.37 |
| Compound in Ex. 55 | 0.27 |
| Compound in Ex. 56 | 0.58 |
| Compound in Ex. 57 | 0.52 |
| Compound in Ex. 58 | 0.41 |
| Compound in Ex. 59 | 0.32 |
| Compound in Ex. 60 | 1.1 |
| Compound in Ex. 61 | 2.2 |
| Compound in Ex. 62 | 0.23 |
| Compound in Ex. 63 | 0.33 |
| Compound in Ex. 64 | 0.38 |
| Compound in Ex. 65 | 0.52 |
| Compound in Ex. 66 | 0.69 |
| Compound in Ex. 67 | 1.1 |

Table continued

| | I $C_{50}$ (nM) |
|---|---|
| Compound in Ex. 68 | 0.42 |

Experimental Example 3: Receptor binding assay-2:

The compound in Reference Example 2 was used, and the $IC_{50}$ was calculated from a dose-inhibition curve of each sample (Tables 10 to 12).

Table 10

| Compounds | $IC_{50}$(nM) |
|---|---|
| Compound in Ex. 1 | 0.1 |
| Compound in Ex. 2 | 0.33 |
| Compound in Ex. 3 | 0.036 |
| Compound in Ex. 4 | 0.1 |
| Compound in Ex. 5 | 0.13 |
| Compound in Ex. 6 | 0.046 |
| Compound in Ex. 7 | 0.12 |
| Compound in Ex. 8 | 0.039 |
| Compound in Ex. 9 | 0.16 |
| Compound in Ex. 10 | 0.071 |
| Compound in Ex. 11 | 0.084 |
| Compound in Ex. 12 | 0.38 |
| Compound in Ex. 13 | 0.29 |
| Compound in Ex. 14 | 0.027 |
| Compound in Ex. 15 | 0.13 |
| Compound in Ex. 16 | 0.066 |

Table 11

| Compounds | $IC_{50}$ (nM) |
|---|---|
| Compound in Ex. 17 | 0.036 |
| Compound in Ex. 18 | 0.023 |
| Compound in Ex. 19 | 0.071 |
| Compound in Ex. 20 | 0.62 |
| Compound in Ex. 21 | 0.84 |
| Compound in Ex. 22 | 0.12 |
| Compound in Ex. 23 | 0.09 |
| Compound in Ex. 24 | 0.057 |
| Compound in Ex. 25 | 0.067 |
| Compound in Ex. 26 | 0.12 |
| Compound in Ex. 27 | 0.11 |

Table continued

| Compounds | $IC_{50}$ (nM) |
|---|---|
| Compound in Ex. 28 | 0.17 |
| Compound in Ex. 29 | 0.21 |
| Compound in Ex. 30 | 0.23 |
| Compound in Ex. 31 | 0.27 |
| Compound in Ex. 32 | 0.35 |
| Compound in Ex. 33 | 0.37 |
| Compound in Ex. 34 | 0.4 |
| Compound in Ex. 35 | 0.48 |
| Compound in Ex. 36 | 0.51 |
| Compound in Ex. 37 | 0.54 |
| Compound in Ex. 38 | 0.59 |
| Compound in Ex. 39 | 0.6 |
| Compound in Ex. 40 | 0.61 |
| Compound in Ex. 41 | 0.73 |
| Compound in Ex. 42 | 0.81 |
| Compound in Ex. 43 | 0.95 |
| Compound in Ex. 44 | 0.99 |
| Compound in Ex. 45 | 1.3 |
| Compound in Ex. 46 | 1.4 |
| Compound in Ex. 47 | 1.8 |
| Compound in Ex. 48 | 2.6 |
| Compound in Ex. 49 | 3.5 |
| Compound in Ex. 50 | 3.6 |

Table 12

| | $IIC_{50}$ (nM) |
|---|---|
| Compound in Ex. 51 | 0.16 |
| Compound in Ex. 52 | 0.26 |
| Compound in Ex. 53 | 0.064 |
| Compound in Ex. 54 | 0.29 |
| Compound in Ex. 55 | 0.09 |
| Compound in Ex. 56 | 0.22 |
| Compound in Ex. 57 | 0.17 |
| Compound in Ex. 58 | 0.15 |
| Compound in Ex. 59 | 0.18 |
| Compound in Ex. 60 | 0.8 |
| Compound in Ex. 61 | 0.56 |
| Compound in Ex. 62 | 0.14 |

Table continued

| | $IIC_{50}$ (nM) |
|---|---|
| Compound in Ex. 63 | 0.074 |
| Compound in Ex. 64 | 0.096 |
| Compound in Ex. 65 | 0.26 |
| Compound in Ex. 66 | 0.11 |
| Compound in Ex. 67 | 0.45 |
| Compound in Ex. 68 | 0.13 |

## INDUSTRIAL APPLICABILITY

The peptide derivative of the present invention is involved in a neutral nerve function-regulating action, a circulatory function-regulating action, a cardiac function-regulating action, an immune function-regulating action, a digestive organ function-regulating action, a metabolic function-regulating action or a generative organ function-regulating action, and thus the agonist or antagonist described above can be used as a prophylactic and/or therapeutic agent for diseases such as senile dementia, cerebrovascular dementia, dementia attributable to degenerative diseases of system degenerative type (for example, Alzheimer's disease, Parkinson's disease, Pick' disease, Huntington's disease), dementia attributable to infectious diseases (for example, delayed viral infections such as Creutzfeldt-Jakob disease), dementia attributable to endocrine, metabolic and poisoning diseases (for example, hypothyroidism, vitamin 12 deficiency, alcoholism, poisoning due to various chemicals, metallic or organic compounds), dementia attributable to tumor diseases (for example, brain tumor), dementia attributable to traumatic diseases (for example, chronic hematocele beneath the dura mater), melancholia, attention deficit hyperactivity (minimal brain disease) syndrome, mental confusion, anxiety, schizophrenia, psychasthenia, an obstacle in growth hormone secretion (for example, gigantisms, acromegaly), bulimia, overeating, hypercholesterolemia, hyperglyceridemia, hyperlipemia, hyperprolactinemia, hypoglycemia, hypopituitarism, pituitary dwarfism, diabetes (for example, diabetic complications, diabetic renal trouble, diabetic nervous disturbance, diabetic retinopathy), cancers (for example, breast cancer, lymphotic leukocyte, lung cancer, bladder cancer, ovary cancer, prostate cancer), pancreatitis, renal diseases (for example, chronic renal trouble, nephritis), Turner's syndrome, neurosis, rheumatic arthritis, spinal damage, transitory cerebral ischemia paroxysm, amyotrophic lateral sclerosis, acute myocardial infarction, spinal cerebellum degeneration, bone fracture, wounds, atopic dermatitis, osteoporosis, asthma, epilepsy, sterility, arteriosclerosis, pulmonary emphysema, lung edema, imperfect lactation or the like. Further, it can be used as a hypnotic sedative, an improver for nutritional conditions after operation, a hypertensive agent or a hypotensive agent.

In addition, it can be used as a prophylactic and/or therapeutic agent for HIV infections and AIDS (acquired immune deficiency syndrome).

**Claims**

**1.** A peptide represented by the formula:

P1-Arg-Pro-Arg-Leu-Phe-P2-P3-Gly-Pro-P4-P5 (I)

wherein P1 represents a hydrogen atom, or an amino acid residue or a peptide chain, consisting of 1 to 25 amino acids which are independently the same or different and whose side chain is substituted or unsubstituted, P2 represents a neutral amino acid residue whose side chain is substituted or unsubstituted or a basic amino acid residue whose side chain is substituted or unsubstituted, P3 represents a neutral amino acid residue whose side chain is substituted or unsubstituted, an aromatic amino acid residue whose side chain is substituted or unsubstituted or a basic amino acid residue whose side chain is substituted or unsubstituted, P4 represents a bond or a neutral or aromatic amino acid residue whose side chain is substituted or unsubstituted, P5 represents [1] an amino acid residue whose side chain is substituted or unsubstituted, or its amino acid derivative wherein the C-terminal carboxyl group has been reduced to a hydroxymethyl group or formyl group, [2] a hydroxyl group, or [3] a dipeptide chain formed by binding an amino acid residue whose side chain is substituted or unsubstituted, to an amino acid residue whose side chain is substituted or unsubstituted, or its peptide derivative wherein the C-terminal carboxyl group of the dipeptide has been reduced to a hydroxymethyl group or formyl group, and a side chain of each amino acid residue in the formula -Arg-Pro-Arg-Leu-Phe- or -Gly-Pro- is substituted or unsubstituted;
an ester thereof or an amide thereof, or a salt thereof.

**2.** The peptide according to claim 1, an ester thereof or an amide thereof, or a salt thereof, wherein P1 is a hydrogen atom, pGlu or Arg-Arg-Gln.

3. The peptide according to claim 1, an ester thereof or an amide thereof, or a salt thereof, wherein P2 is substituted or unsubstituted His or is substituted or unsubstituted Ala.

4. The peptide according to claim 1, an ester thereof or an amide thereof, or a salt thereof, wherein P3 is substituted or unsubstituted Arg or is substituted or unsubstituted Lys.

5. The peptide according to claim 1, an ester thereof or an amide thereof, or a salt thereof, wherein -P4-P5 is -Cha-Pro-Phe (Cl), -Cha-Pro-Phe, -Met-Pro-Phe, -Met-Pro-Phe (Cl), -Cha-Phe or -Met-Phe.

6. The peptide according to claim 1, which is represented by:

[1] Arg-Pro-Arg-Leu-Phe-Ala-Arg-Gly-Pro-Cha-Pro-Phe(Cl),
[2] Arg-Pro-Arg-Leu-Phe-His-Lys-Gly-Pro-Cha-Pro-Phe(Cl),
[3] Arg-Pro-Arg-Leu-Phe-His-Lys-Gly-Pro-Cha-Pro-Phe,
[4] Arg-Pro-Arg-Leu-Phe-His-Lys-Gly-Pro-Met-Pro-Phe(Cl),
[5] Arg-Pro-Arg-Leu-Phe-Ala-Arg-Gly-Pro-Met-Pro-Phe,
[6] Arg-Pro-Arg-Leu-Phe-His-Lys-Gly-Pro-Cha-Phe(Cl),
[7] pGlu-Arg-Pro-Arg-Leu-Phe-Arg-A.rg-Gly-Pro-Met-Pro-Phe,
[8] pGlu-Arg-Pro-Arg-Leu-Phe-His-Lys-Gly-Pro-Met-Pro-Phe, or
[9] Arg-Pro-Arg-Leu-Phe-Ala-Arg-Gly-Pro-Met-Phe,

an ester thereof or an amide thereof, or a salt thereof.

7. A pharmaceutical preparation comprising the peptide described in claim 1, an ester thereof or an amide thereof, or a salt thereof.

8. The pharmaceutical preparation according to claim 7, which is a neutral nerve function regulator, a circulatory function regulator, a cardiac function regulator, an immune function regulator, a digestive organ function regulator, a metabolic function regulator or a generative organ regulator.

9. The pharmaceutical preparation according to claim 7, which is a prophylactic and therapeutic agent for HIV infections or AIDS.

10. Use of the peptide described in claim 1, an ester thereof or an amide thereof, or a salt thereof, in the manufacture of a medicament for preventing or treating HIV infections or AIDS.

## Revendications

1. Peptide représenté par la formule :

P1-Arg-Pro-Arg-Leu-Phe-P2-P3-Gly-Pro-P4-P5 (I)

dans laquelle

- P1 représente un atome d'hydrogène, ou un résidu d'acide aminé ou une chaîne peptidique, comportant 1 à 25 résidus d'acide aminé qui peuvent être indépendamment identiques ou différents et dont la chaîne latérale porte ou non un substituant ;
- P2 représente un résidu d'acide aminé neutre dont la chaîne latérale porte ou non un substituant, ou un résidu d'acide aminé basique dont la chaîne latérale porte ou non un substituant ;
- P3 représente un résidu d'acide aminé neutre dont la chaîne latérale porte ou non un substituant, un résidu d'acide aminé aromatique dont la chaîne latérale porte ou non un substituant, ou un résidu d'acide aminé basique dont la chaîne latérale porte ou non un substituant ;
- P4 représente une liaison ou un résidu d'acide aminé neutre ou aromatique dont la chaîne latérale porte ou non un substituant,
- et P5 représente :

1) un résidu d'acide aminé dont la chaîne latérale porte ou non un substituant, ou un dérivé d'acide aminé où le groupe carboxyle C-terminal a été réduit en un groupe formyle ou hydroxyméthyle,
2) un groupe hydroxyle,
3) ou une chaîne dipeptidique constituée par liaison d'un résidu d'acide aminé, dont la chaîne latérale porte ou non un substituant, à un résidu d'acide aminé dont la chaîne latérale porte ou non un substituant, ou un dérivé de peptide où le groupe carboxyle C-terminal d'un tel dipeptide a été réduit en un groupe formyle ou hydroxyméthyle,
et la chaîne latérale de chacun des résidus d'acide aminé indiqués dans la formule -Arg-Pro-Arg-Leu-Phe- ou -Gly-Pro- porte ou non un substituant,

ou ester ou amide dérivé d'un tel peptide, ou sel d'un tel peptide.

**2.** Peptide conforme à la revendication 1, ou ester ou amide dérivé d'un tel peptide, ou sel d'un tel peptide, dans lequel P1 représente un atome d'hydrogène, pGlu ou Arg-Arg-Gln.

**3.** Peptide conforme à la revendication 1, ou ester ou amide dérivé d'un tel peptide, ou sel d'un tel peptide, dans lequel P2 représente un résidu His portant ou non un sustituant ou Ala portant ou non un sustituant.

**4.** Peptide conforme à la revendication 1, ou ester ou amide dérivé d'un tel peptide, ou sel d'un tel peptide, dans lequel P3 représente un résidu Arg portant ou non un sustituant ou Lys portant ou non un sustituant

**5.** Peptide conforme à la revendication 1, ou ester ou amide dérivé d'un tel peptide, ou sel d'un tel peptide, dans lequel -P4-P5 représente - Cha-Pro-Phe(Cl), -Cha-Pro-Phe, -Met-Pro-Phe, -Met-Pro-Phe(Cl), - Cha-Phe ou -Met-Phe.

**6.** Peptide conforme à la revendication 1, représenté par l'une des formules suivantes :

[1] Arg-Pro-Arg-Leu-Phe-Ala-Arg-Gly-Pro-Cha-Pro-Phe(Cl)
[2] Arg-Pro-Arg-Leu-Phe-His-Lys-Gly-Pro-Cha-Pro-Phe(Cl)
[3] Arg-Pro-Arg-Leu-Phe-His-Lys-Gly-Pro-Cha-Pro-Phe
[4] Arg-Pro-Arg-Leu-Phe-His-Lys-Gly-Pro-Met-Pro-Phe(Cl)
[5] Arg-Pro-Arg-Leu-Phe-Ala-Arg-Gly-Pro-Met-Pro-Phe
[6] Arg-Pro-Arg-Leu-Phe-His-Lys-Gly-Pro-Cha-Phe(Cl)
[7] pGlu-Arg-Pro-Arg-Leu-Phe-Arg-Arg-Gly-Pro-Met-Pro-Phe
[8] pGlu-Arg-Pro-Arg-Leu-Phe-His-Lys-Gly-Pro-Met-Pro-Phe
[9] Arg-Pro-Arg-Leu-Phe-Ala-Arg-Gly-Pro-Met-Phe

ou ester ou amide dérivé d'un tel peptide, ou sel d'un tel peptide.

**7.** Préparation pharmaceutique comprenant un peptide conforme à la revendication 1, ou un ester ou amide dérivé d'un tel peptide, ou un sel d'un tel peptide.

**8.** Préparation pharmaceutique conforme à la revendication 7, qui est un régulateur neutre de la fonction nerveuse, un régulateur de la fonction circulatoire, un régulateur de la fonction cardiaque, un régulateur de la fonction immunitaire, un régulateur de la fonction des organes digestifs, un régulateur de la fonction métabolique ou un régulateur des organes de la reproduction.

**9.** Préparation pharmaceutique conforme à la revendication 7, qui est un agent prophylactique et thérapeutique contre les infections par VIH ou le sida.

**10.** Emploi d'un peptide conforme à la revendication 1, ou d'un ester ou amide dérivé d'un tel peptide, ou d'un sel d'un tel peptide, dans la fabrication d'un médicament destiné à la prévention ou au traitement des infections par VIH ou du sida.

## Patentansprüche

**1.** Peptid, dargestellt durch die Formel

P1-Arg-Pro-Arg-Leu-Phe-P2-P3-Gly-Pro-P4-P5 (I)

worin P1 ein Wasserstoffatom oder einen Aminosäurerest oder eine Peptidkette darstellt, bestehend aus 1 bis 25 Aminosäuren, die unabhängig voneinander dieselben oder verschieden sind, und deren Seitenkette substituiert oder unsubstituiert ist, P2 einen neutralen Aminosäurerest, dessen Seitenkette substituiert oder unsubstituiert ist, oder einen basischen Aminosäurerest, dessen Seitenkette substituiert oder unsubstituiert ist, darstellt, P3 einen neutralen Aminosäurerest, dessen Seitenkette substituiert oder unsubstituiert ist, einen aromatischen Aminosäurerest, dessen Seitenkette substituiert oder unsubstituiert ist, oder einen basischen Aminosäurerest, dessen Seitenkette substituiert oder unsubstituiert ist, darstellt, P4 eine Bindung oder einen neutralen oder aromatischen Aminosäurerest, dessen Seitenkette substituiert oder unsubstituiert ist, darstellt, P5 [1] einen Aminosäurerest, dessen Seitenkene substituiert oder unsubstituiert ist, oder sein Aminosäurederivat, worin die C-terminale Carboxylgruppe zu einer Hydroxymethylgruppe oder Formylgruppe reduziert worden ist, [2] eine Hydroxylgruppe oder [3] eine Dipeptidkette, gebildet durch Binden eines Aminosäurerests, dessen Seitenkette substituiert oder unsubstituiert ist, an einen Aminosäurerest, dessen Seitenkette substituiert oder unsubstituiert ist, oder sein Peptidderivat darstellt, worin die C-terminale Carboxylgruppe des Dipeptids zu einer Hydroxymethylgruppe oder Formylgruppe reduziert worden ist, und eine Seitenkette jedes Aminosäurerests in der Formel -Arg-Pro-Arg-Leu-Phe- oder -Gly-Pro- substituiert oder unsubstituiert ist; ein Ester hiervon oder ein Amid hiervon oder ein Salz von diesen.

**2.** Peptid nach Anspruch 1, ein Ester hiervon oder ein Amid hiervon oder ein Salz von diesen, worin P1 ein Wasserstoffatom, pGlu oder Arg-Arg-Gln ist.

**3.** Peptid nach Anspruch 1, ein Ester hiervon oder ein Amid hiervon oder ein Salz von diesen, worin P2 substituiertes oder unsubstituiertes His oder substituiertes oder unsubstituiertes Ala ist.

**4.** Peptid nach Anspruch 1, ein Ester hiervon oder ein Amid hiervon oder ein Salz von diesen, worin P3 substituiertes oder unsubstituiertes Arg oder substituiertes oder unsubstituiertes Lys ist.

**5.** Peptid nach Anspruch 1, ein Ester hiervon oder ein Amid hiervon oder ein Salz von diesen, worin -P4-P5 -Cha-Pro-Phe (Cl), -Cha-Pro-Phe, -Met-Pro-Phe, -Met-Pro-Phe (Cl), -Cha-Phe oder -Met-Phe ist.

**6.** Peptid nach Anspruch 1, das dargestellt ist durch:

[1] Arg-Pro-Arg-Leu-Phe-Ala-Arg-Gly-Pro-Cha-Pro-Phe (Cl),
[2] Arg-Pro-Arg-Leu-Phe-His-Lys-Gly-Pro-Cha-Pro-Phe (Cl),
[3] Arg-Pro-Arg-Leu-Phe-His-Lys-Gly-Pro-Cha-Pro-Phe,
[4] Arg-Pro-Arg-Leu-Phe-His-Lys-Gly-Pro-Met-Pro-Phe(Cl),
[5] Arg-Pro-Arg-Leu-Phe-Ala-Arg-Gly-Pro-Met-Pro-Phe,
[6] Arg-Pro-Arg-Leu-Phe-His-Lys-Gly-Pro-Cha-Phe(Cl),
[7] pGlu-Arg-Pro-Arg-Leu-Phe-Arg-Arg-Gly-Pro-Met-Pro-Phe,
[8] pGlu-Arg-Pro-Arg-Leu-Phe-His-Lys-Gly-Pro-Met-Pro-Phe oder
[9] Arg-Pro-Arg-Leu-Phe-Ala-Arg-Gly-Pro-Met-Phe,

ein Ester hiervon oder ein Amid hiervon oder ein Salz von diesen.

**7.** Pharmazeutisches Präparat, umfassend das Peptid, das in Anspruch 1 beschrieben wird, ein Ester hiervon oder ein Amid hiervon oder ein Salz von diesen.

**8.** Pharmazeutisches Präparat nach Anspruch 7, das ein neutraler Nervenfunktionsregulator, ein Zirkulationsfunktionsregulator, ein Herzfunktionsregulator, ein Immunfunktionsregulator, ein Verdauungsorganfunktionsregulator, ein Stoffwechselfunktionsregulator oder ein Fortpflanzungsorganregulator ist.

**9.** Pharmazeutisches Präparat nach Anspruch 7, das ein prophylaktisches und therapeutisches Mittel für HIV-Infektionen oder AIDS ist.

**10.** Verwendung des Peptids nach Anspruch 1, eines Esters hiervon oder eines Amids hiervon oder eines Salzes hiervon bei der Herstellung eines Medikaments zur Vorbeugung oder Behandlung von HIV-Infektionen oder AIDS.

[Sequence Listing]

<110> Takeda Chemical Industries, Ltd.
<120> Peptide derivative
<130> 2702WOOP
<150> JP 12-87114
<151> 2000-03-23
<150> JP 12-288891
<151> 2000-09-19
<160> 72
<210> 1
<211> 15
<212> PRT
<213> Artificial Sequence
<223> Xaa means Phe(Cl)
<400> 1

Arg Arg Gln Arg Pro Arg Leu Ser Ala Arg Gly Pro Met Pro Xaa
1 5 10 15

<210> 2
<211> 15
<212> PRT
<213> Artificial Sequence
<223> Xaa on the 13th position means Cha, Xaa on the 15th position means Phe(Cl)
<400> 2

Arg Arg Gln Arg Pro Arg Leu Ser His Lys Gly Pro Cha Pro Xaa
1 5 10 15

<210> 3
<211> 12
<212> PRT
<213> Artificial Sequence
<223> Xaa means Phe (Cl)
<400> 3

Arg Pro Arg Leu Ser Ala Arg Gly Pro Met Pro Xaa
1 5 10

<210> 4
<211> 11
<212> PRT
<213> Artificial Sequence
<223> Xaa on the 10th position means Cha, Xaa on the 11th position means Phe (Cl)
<400> 4

Arg Pro Arg Leu Ser Ala Arg Gly Pro Xaa Xaa
1 5 10

<210> 5
<211> 12
<212> PRT
<213> Artificial Sequence
<223> Xaa on the 10th position means Cha, Xaa on the 12th position means Phe(Cl)
<400> 5

Arg Pro Arg Leu Ser Ala Arg Gly Pro Xaa Pro Xaa
1 5 10

<210> 6
<211> 13

<212> PRT
<213> Artificial Sequence
<223> Xaa means Cha.
<400> 6

Arg Arg Gln Arg Pro Arg Leu Ser Ala Arg Gly Pro Cha
1 5 10

<210> 7
<211> 12
<212> PRT
<213> Artificial Sequence
<223> Xaa on the 10th position means Cha, Xaa on the 12th position means Phe (Cl)
<400> 7

Arg Pro Arg Leu Ser His Lys Gly Pro Xaa Pro Xaa
1 5 10

<210> 8
<211> 12
<212> PRT
<213> Artificial Sequence
<223> Xaa means Phe(Cl)
<400> 8

Arg Pro Arg Leu Ser His Lys Gly Pro Met Pro Xaa
1 5 10

<210> 9
<211> 13
<212> PRT
<213> Artificial Sequence
<223> Xaa on the 1st position means pGlu, Xaa on the 11th position means Cha.
<400> 9

Xaa Arg Pro Arg Leu Ser His Lys Gly Pro Xaa Pro Phe
1 5 10

<210> 10
<211> 13
<212> PRT
<213> Artificial Sequence
<223> Xaa on the 1st position means pGlu, Xaa on the 13th position means Phe (Cl)
<400> 10

Xaa Arg Pro Arg Leu Ser His Lys Gly Pro Met Pro Xaa
1 5 10

<210> 11
<211> 12
<212> PRT
<213> Artificial Sequence
<223> Xaa means Cha.
<400> 11

Arg Pro Arg Leu Ser Ala Arg Gly Pro Xaa Pro Phe
1 5 10

<210> 12
<211> 12
<212> PRT
<213> Artificial Sequence
<223> Xaa on the 10th position means Cha, Xaa on the 12th position means Phe (Cl)
<400> 12

Arg Pro Arg Leu Phe Ala Arg Gly Pro Xaa Pro Xaa
1 5 10

<210> 13
<211> 12
<212> PRT
<213> Artificial Sequence
<223> Xaa on the 10th position means Cha, Xaa on the 12th position means Phe(Cl).
<400> 13

Arg Pro Arg Leu Phe His Lys Gly Pro Xaa Pro Xaa
1 5 10

<210> 14
<211> 15
<212> PRT
<213> Artificial Sequence
<223> Xaa means Nle.
<400> 14

Arg Arg Gln Arg Pro Arg Leu Ser His Lys Gly Pro Xaa Pro Tyr
1 5 10 15

<210> 15
<211> 12
<212> PRT
<213> Artificial Sequence
<223> Xaa means Cha.
<400> 15

Arg Pro Arg Leu Phe His Lys Gly Pro Xaa Pro Phe
1 5 10

<210> 16
<211> 12
<212> PRT
<213> Artificial Sequence
<223> Xaa means Phe(Cl)
<400> 16

Arg Pro Arg Leu Phe His Lys Gly Pro Met Pro Xaa
1 5 10

<210> 17
<211> 36
<212> PRT
<213> Artificial Sequence
<223> Xaa means Nle.
<400> 17

Leu Val Gln Pro Arg Gly Ser Arg Asn Gly Pro Gly Pro Trp Gln Gly
1 5 10 15
Gly Arg Arg Lys Phe Arg Arg Gln Arg Pro Arg Leu Ser His Lys Gly
20 25 30
Pro Xaa Pro Tyr
35

<210> 18
<211> 13
<212> PRT
<213> Artificial Sequence
<223> Xaa on the 1st position means pGlu, Xaa on the 11th position means Nle.
<400> 18

Xaa Arg Pro Arg Leu Ser His Lys Gly Pro Xaa Pro Tyr
1 5 10

<210> 19
<211> 380
<212> PRT
<213> Unknown
<220>
<223>
<400> 19

Met Glu Glu Gly Gly Asp Phe Asp Asn Tyr Tyr Gly Ala Asp Asn Gln
1 5 10 15

Ser Glu Cys Glu Tyr Thr Asp Trp Lys Ser Ser Gly Ala Leu Ile Pro
20 25 30

Ala Ile Tyr Met Leu Val Phe Leu Leu Gly Thr Thr Gly Asn Gly Leu
35 40 45

Val Leu Trp Thr Val Phe Arg Ser Ser Arg Glu Lys Arg Arg Ser Ala
50 55 60

Asp Ile Phe Ile Ala Ser Leu Ala Val Ala Asp Leu Thr Phe Val Val
65 70 75 80

Thr Leu Pro Leu Trp Ala Thr Tyr Thr Tyr Arg Asp Tyr Asp Trp Pro
85 90 95

Phe Gly Thr Phe Phe Cys Lys Leu Ser Ser Tyr Leu Ile Phe Val Asn
100 105 110

Met Tyr Ala Ser Val Phe Cys Leu Thr Gly Leu Ser Phe Asp Arg Tyr
115 120 125

Leu Ala Ile Val Arg Pro Val Ala Asn Ala Arg Leu Arg Leu Arg Val
130 135 140

Ser Gly Ala Val Ala Thr Ala Val Leu Trp Val Leu Ala Ala Leu Leu
145 150 155 160

Ala Met Pro Val Met Val Leu Arg Thr Thr Gly Asp Leu Glu Asn Thr
165 170 175

Thr Lys Val Gln Cys Tyr Met Asp Tyr Ser Met Val Ala Thr Val Ser
180 185 190

Ser Glu Trp Ala Trp Glu Val Gly Leu Gly Val Ser Ser Thr Thr Val
195 200 205

Gly Phe Val Val Pro Phe Thr Ile Met Leu Thr Cys Tyr Phe Phe Ile
210 215 220

Ala Gln Thr Ile Ala Gly His Phe Arg Lys Glu Arg Ile Glu Gly Leu
225 230 235 240

Arg Lys Arg Arg Arg Leu Leu Ser Ile Ile Val Val Leu Val Val Thr
245 250 255

Phe Ala Leu Cys Trp Met Pro Tyr His Leu Val Lys Thr Leu Tyr Met
260 265 270

Leu Gly Ser Leu Leu His Trp Pro Cys Asp Phe Asp Leu Phe Leu Met
275 280 285

Asn Ile Phe Pro Tyr Cys Thr Cys Ile Ser Tyr Val Asn Ser Cys Leu
290 295 300

Asn Pro Phe Leu Tyr Ala Phe Phe Asp Pro Arg Phe Arg Gln Ala Cys
305 310 315 320

Thr Ser Met Leu Cys Cys Gly Gln Ser Arg Cys Ala Gly Thr Ser His
325 330 335

Ser Ser Ser Gly Glu Lys Ser Ala Ser Tyr Ser Ser Gly His Ser Gln

340 345 350

Gly Pro Gly Pro Asn Met Gly Lys Gly Gly Glu Gln Met His Glu Lys

355 360 365

Ser Ile Pro Tyr Ser Gln Glu Thr Leu Val Val Asp

370 375 380

<210> 20
<211> 1140
<212> DNA
<213> Unknown
<220>
<223>
<400> 20

ATGGAGGAAG GTGGTGATTT TGACAACTAC TATGGGGCAG ACAACCAGTC TGAGTGTGAG 60
TACACAGACT GGAAATCCTC GGGGGCCCTC ATCCCTGCCA TCTACATGTT GGTCTTCCTC 120
CTGGGCACCA CGGGAAACGG TCTGGTGCTC TGGACCGTGT TTCGGAGCAG CCGGGAGAAG 180
AGGCGCTCAG CTGATATCTT CATTGCTAGC CTGGCGGTGG CTGACCTGAC CTTCGTGGTG 240
ACGCTGCCCC TGTGGGCTAC CTACACGTAC CGGGACTATG ACTGGCCCTT TGGGACCTTC 300
TTCTGCAAGC TCAGCAGCTA CCTCATCTTC GTCAACATGT ACGCCAGCGT CTTCTGCCTC 360
ACCGGCCTCA GCTTCGACCG CTACCTGGCC ATCGTGAGGC CAGTGGCCAA TGCTCGGCTG 420
AGGCTGCGGG TCAGCGGGGC CGTGGCCACG GCAGTTCTTT GGGTGCTGGC CGCCCTCCTG 480
GCCATGCCTG TCATGGTGTT ACGCACCACC GGGGACTTGG AGAACACCAC TAAGGTGCAG 540
TGCTACATGG ACTACTCCAT GGTGGCCACT GTGAGCTCAG AGTGGGCCTG GGAGGTGGGC 600
CTTGGGGTCT CGTCCACCAC CGTGGGCTTT GTGGTGCCCT TCACCATCAT GCTGACCTGT 660
TACTTCTTCA TCGCCCAAAC CATCGCTGGC CACTTCCGCA AGGAACGCAT CGAGGGCCTG 720
CGGAAGCGGC GCCGGCTGCT CAGCATCATC GTGGTGCTGG TGGTGACCTT TGCCCTGTGC 780
TGGATGCCCT ACCACCTGGT GAAGACGCTG TACATGCTGG GCAGCCTGCT GCACTGGCCC 840
TGTGACTTTG ACCTCTTCCT CATGAACATC TTCCCCTACT GCACCTGCAT CAGCTACGTC 900

AACAGCTGCC TCAACCCCTT CCTCTATGCC TTTTTCGACC CCCGCTTCCG CCAGGCCTGC 960
ACCTCCATGC TCTGCTGTGG CCAGAGCAGG TGCGCAGGCA CCTCCCACAG CAGCAGTGGG 1020
GAGAAGTCAG CCAGCTACTC TTCGGGGCAC AGCCAGGGGC CCGGCCCCAA CATGGGCAAG 1080
GGTGGAGAAC AGATGCACGA GAAATCCATC CCCTACAGCC AGGAGACCCT TGTGGTTGAC 1140

<210> 21
<211> 15
<212> PRT
<213> Artificial Sequence
<223>
<400> 21

Arg Arg Gln Arg Pro Arg Leu Ser Ala Arg Gly Pro Met Pro Phe
1 5 10 15

<210> 22
<211> 13
<212> PRT
<213> Artificial Sequence
<223>
<400> 22

Arg Arg Gln Arg Pro Arg Leu Ser Ala Arg Gly Pro Met
1 5 10 13

<210> 23
<211> 12
<212> PRT
<213> Artificial Sequence
<223>
<400> 23

Arg Arg Gln Arg Pro Arg Leu Ser Ala Arg Gly Pro
1 5 10 12

<210> 24
<211> 13
<212> PRT
<213> Artificial Sequence
<223> Xaa means Met (0)
<400> 24

Arg Arg Gln Arg Pro Arg Leu Ser His Lys Gly Pro Xaa
1 5 10 13

<210> 25
<211> 15
<212> PRT
<213> Artificial Sequence
<223> Xaa means Lys(Arg-Arg)
<400> 25

Arg Arg Xaa Arg Pro Arg Leu Ser His Lys Gly Pro Met Pro Phe
1 5 10 15

<210> 26
<211> 15
<212> PRT
<213> Artificial Sequence
<223>
<400> 26

Arg Arg Arg Arg Pro Arg Leu Ser His Lys Gly Pro Met Pro Phe
1 5 10 15

<210> 27
<211> 15
<212> PRT
<213> Artificial Sequence
<223>
<400> 27

Arg Arg Lys Arg Pro Arg Leu Ser His Lys Gly Pro Met Pro Phe
1 5 10 15

<210> 28
<211> 12
<212> PRT
<213> Artificial Sequence
<223>
<400> 28

Arg Pro Arg Leu Ser Ala Arg Gly Pro Met Pro Phe
1 5 10 12

<210> 29

<211> 15
<212> PRT
<213> Artificial Sequence
<223>
<400> 29

Arg Arg Ala Arg Pro Arg Leu Ser His Lys Gly Pro Met Pro Phe
1 5 10 15

<210> 30
<211> 13
<212> PRT
<213> Artificial Sequence
<223> Xaa means pGlu
<400> 30

Xaa Arg Pro Arg Leu Ser Ala Arg Gly Pro Met Pro Phe
1 5 10 13

<210> 31
<211> 10
<212> PRT
<213> Artificial Sequence
<223>
<400> 31

Arg Pro Arg Leu Ser Ala Arg Gly Pro Met
1 5 10

<210> 32
<211> 11
<212> PRT
<213> Artificial Sequence
<223> Xaa means Phe(Cl)
<400> 32

Arg Pro Arg Leu Ser His Lys Gly Pro Met Xaa
1 5 10 11

<210> 33
<211> 13
<212> PRT
<213> Artificial Sequence
<223> Xaa means pGlu
<400> 33

Xaa Arg Pro Arg Leu Ser Ala Lys Gly Pro Met Pro Phe
1 5 10 13

<210> 34
<211> 13
<212> PRT
<213> Artificial Sequence
<223> Xaa means pGlu
<400> 34

Xaa Arg Pro Arg Leu Ser Arg Lys Gly Pro Met Pro Phe
1 5 10 13

<210> 35
<211> 12
<212> PRT
<213> Artificial Sequence
<223>
<400> 35

Arg Pro Arg Leu Phe Ala Arg Gly Pro Met Pro Phe
1 5 10 12

<210> 36
<211> 12
<212> PRT
<213> Artificial Sequence
<223> Xaa means Nal(2)
<400> 36

Arg Pro Arg Leu Ser His Lys Gly Pro Met Pro Xaa
1 5 10 12

<210> 37
<211> 15
<212> PRT
<213> Artificial Sequence
<223>
<400> 37

Arg Arg Phe Arg Pro Arg Leu Ser His Lys Gly Pro Met Pro Phe
1 5 10 15

<210> 38

<211> 13
<212> PRT
<213> Artificial Sequence
<223> Xaa means pGlu
<400> 38

Xaa Arg Pro Arg Leu Ser His Arg Gly Pro Met Pro Phe
1 5 10 13

<210> 39
<211>12
<212> PRT
<213> Artificial Sequence
<223> Xaa on the 1st position means pGlu, Xaa on the 12th position means Phe(Cl)
<400> 39

Xaa Arg Pro Arg Leu Ser His Lys Gly Pro Met Xaa
1 5 10 12

<210> 40
<211> 11
<212> PRT
<213> Artificial Sequence
<223> Xaa means Cha
<400> 40

Arg Pro Arg Leu Ser His Lys Gly Pro Met Xaa
1 5 10 11

<210> 41
<211> 11
<212> PRT
<213> Artificial Sequence
<223> Xaa on the 10th position means Cha, Xaa on the 11th position means Phe (Cl)
<400> 41

Arg Pro Arg Leu Phe His Lys Gly Pro Xaa Xaa
1 5 10 11

<210> 42
<211> 13
<212> PRT
<213> Artificial Sequence
<223> Xaa means pGlu
<400> 42

Xaa Arg Pro Arg Leu Ser Leu Lys Gly Pro Met Pro Phe

1 5 10 13

<210> 43
<211> 13
<212> PRT
<213> Artificial Sequence
<223> Xaa on the 1st position means pGlu, Xaa on the 13th position means Nal (2)
<400> 43

Xaa Arg Pro Arg Leu Ser His Lys Gly Pro Met Pro Xaa

1 5 10 13

<210> 44
<211> 11
<212> PRT
<213> Artificial Sequence
<223> Xaa means Nal (2)
<400> 44

Arg Pro Arg Leu Ser His Lys Gly Pro Met Xaa

1 5 10 11

<210> 45
<211> 13
<212> PRT
<213> Artificial Sequence
<223> Xaa means pGlu
<400> 45

Xaa Arg Pro Arg Leu Ser Arg Arg Gly Pro Met Pro Phe

1 5 10 13

<210> 46
<211> 13
<212> PRT
<213> Artificial Sequence
<223> Xaa means pGlu
<400> 46

Xaa Arg Pro Arg Leu Phe Arg Arg Gly Pro Met Pro Phe

1 5 10 13

<210> 47

<211> 13
<212> PRT
<213> Artificial Sequence
<223> Xaa means pGlu
<400> 47

Xaa Arg Pro Arg Leu Ser Phe Lys Gly Pro Met Pro Phe
1 5 10 13

<210> 48
<211> 13
<212> PRT
<213> Artificial Sequence
<223> Xaa means pGlu
<400> 48

Xaa Arg Pro Arg Leu Phe His Lys Gly Pro Met Pro Phe
1 5 10 13

<210> 49
<211> 12
<212> PRT
<213> Artificial Sequence
<223> Xaa on the ist position means pGlu, Xaa on the 12th position means Cha
<400> 49

Xaa Arg Pro Arg Leu Ser His Lys Gly Pro Met Xaa
1 5 10 12

<210> 50
<211> 12
<212> PRT
<213> Artificial Sequence
<223> Xaa on the 1st position means pGlu, Xaa on the 12th position means Nal(2)
<400> 50

Xaa Arg Pro Arg Leu Ser His Lys Gly Pro Met Xaa
1 5 10 12

<210> 51
<211> 11
<212> PRT
<213> Artificial Sequence
<223>
<400> 51

Arg Pro Arg Leu Phe Ala Arg Gly Pro Met Phe

1 5 10 11

<210> 52
<211> 13
<212> PRT
<213> Artificial Sequence
<223> Xaa means pGlu
<400> 52

Xaa Arg Pro Arg Leu Ser His Phe Gly Pro Met Pro Phe

1 5 10 13

<210> 53
<211> 12
<212> PRT
<213> Artificial Sequence
<223> Xaa means Cha
<400> 53

Arg Pro Arg Leu Ser His Lys Gly Pro Met Pro Xaa

1 5 10 12

<210> 54
<211> 13
<212> PRT
<213> Artificial Sequence
<223> Xaa means pGlu
<400> 54

Xaa Arg Pro Arg Leu Ser His Leu Gly Pro Met Pro Phe

1 5 10 13

<210> 55
<211> 12
<212> PRT
<213> Artificial Sequence
<223> Xaa means $NMe_2$
<400> 55

Arg Arg Gln Arg Pro Arg Leu Ser Ala Arg Gly Xaa

1 5 10 12

<210> 56
<211> 12

<212> PRT
<213> Artificial Sequence
<223> Xaa means Mor
<400> 56

Arg Arg Gln Arg Pro Arg Leu Ser Ala Arg Gly Xaa
1 5 . 10 12

<210> 57
<211> 12
<212> PRT
<213> Artificial Sequence
<223> Xaa means Phe (Cl)
<400> 57

Arg Pro Arg Leu Ser Ala Arg Gly Pro Ala Pro Xaa
1 5 10 12

<210> 58
<211> 12
<212> PRT
<213> Artificial Sequence
<223> Xaa means Phe(Cl)
<400> 58

Arg Pro Arg Leu Ser Ala Arg Gly Gly Met Pro Xaa
1 5 10 12

<210> 59
<211> 12
<212> PRT
<213> Artificial Sequence
<223> Xaa on the 9 th position means N-MeAla, Xaa on the 12th position means Phe(Cl)
<400> 59

Arg Pro Arg Leu Ser Ala Arg Gly Xaa Met Pro Xaa
1 5 10 12

<210> 60
<211> 12
<212> PRT
<213> Artificial Sequence
<223> Xaa on the 10th position means Cha, Xaa on the 12th position means Phe(Cl)
<400> 60

Arg Pro Arg Leu Ser His Ala Gly Pro Xaa Pro Xaa
1 5 10 12

<210> 61
<211> 12
<212> PRT
<213> Artificial Sequence
<223> Xaa on the 10th position means Cha, Xaa on the 12th position means Phe(Cl)
<400> 61

Arg Pro Arg Ala Ser His Lys Gly Pro Xaa Pro Xaa
1 5 10 12

<210> 62
<211> 12
<212> PRT
<213> Artificial Sequence
<223> Xaa on the 10th position means Cha, Xaa on the 12th position means Phe (Cl)
<400> 62

Arg Pro Ala Leu Ser His Lys Gly Pro Xaa Pro Xaa
1 5 10 12

<210> 63
<211> 11
<212> PRT
<213> Artificial Sequence
<223> Xaa on the 10th position means Cha, Xaa on the 11th position means Pyn
<400> 63

Arg Pro Arg Leu Ser Ala Arg Gly Pro Xaa Xaa
1 5 10 11

<210> 64
<211> 12
<212> PRT
<213> Artificial Sequence
<223> Xaa on the 10th position means Cha, Xaa on the 12th position means Pyn
<400> 64

Arg Pro Arg Leu Ser Ala Arg Gly Pro Xaa Pro Xaa
1 5 10 12

<210> 65

<211> 13
<212> PRT
<213> Artificial Sequence
<223> Xaa on the 13 th position means Cha
<400> 65

Arg Arg Gln Arg Pro Arg Leu Ser Ala Arg Gly Gly Xaa
1 5 10 13

<210> 66
<211> 12
<212> PRT
<213> Artificial Sequence
<223> Xaa on the 3rd position means $Lys(Me)_2$
<400> 66

Arg Pro Xaa Leu Ser Ala Arg Gly Pro Met Pro Phe
1 5 10 12

<210> 67
<211> 12
<212> PRT
<213> Artificial Sequence
<223> Xaa on the 7th position means Lys(Me),
<400> 67

Arg Pro Arg Leu Ser Ala Xaa Gly Pro Met Pro Phe
1 5 10 12

<210> 68
<211> 12
<212> PRT
<213> Artificial Sequence
<223> Xaa on the 6th position means Dap, Xaa on the 10th position means Cha, Xaa on the 12th position means Phe(Cl)
<400> 68

Arg Pro Arg Leu Ser Xaa Arg Gly Pro Xaa Pro Xaa
1 5 10 12

<210> 69
<211> 12
<212> PRT
<213> Artificial Sequence
<223> Xaa on the 6th position means Dap(Ac), Xaa on the 10th position means Cha, Xaa on the 12th position means Phe(Cl)
<400> 69

Arg Pro Arg Leu Ser Xaa Arg Gly Pro Xaa Pro Xaa

1 5 10 12

<210> 70
<211> 12
<212> PRT
<213> Artificial Sequence
<223> Xaa on the 6th pos i t i on means Dap($C_6$), Xaa on the 10th pos i t i on means Cha, Xaa on the 12th position means Phe(Cl)
<400> 70

Arg Pro Arg Leu Ser Xaa Arg Gly Pro Xaa Pro Xaa

1 5 10 12

<210> 71
<211> 12
<212> PRT
<213> Artificial Sequence
<223> Xaa on the 6th position means Dap (Adi), Xaa on the 10th position means Cha, Xaa on the 12th position means Phe(Cl)
<400> 71

Arg Pro Arg Leu Ser Xaa Arg Gly Pro Xaa Pro Xaa

1 5 10 12